# EUROPEAN PATENT SPECIFICATION

(11) **EP 4 504 761 B1**
(45) Date of publication and mention of the grant of the patent: **04.02.2026**
(21) Application number: 23720536.4
(22) Date of filing: 04.04.2023
(51) Int. Cl.: C07K 14/435, A61L 27/22, C07K 14/78, C12N 15/62, C12N 15/82, D01F 4/02, C12N 9/02

(54) **RECOMBINANT SPIDER SILK-REINFORCED COLLAGEN PROTEINS PRODUCED IN PLANTS AND THE USE THEREOF**
REKOMBINANTE, IN PFLANZEN PRODUZIERTE UND MIT SPINNENSEIDENPROTEIN VERSTÄRKTE KOLLAGENPROTEINE UND IHRE VERWENDUNG
PROTÉINES RECOMBINÉES DE COLLAGÈNE RENFORCÉES PAR LA SOIE D'ARAIGNÉE, PRODUITES DANS DES PLANTES ET LEUR UTILISATION

(30) Priority: 04.04.2022 EP 22166454
(43) Date of publication of application: 12.02.2025
(73) Proprietor: SwiftPharma BV, 9000 Gent (BE)
(72) Inventor: HOFENK, Jeroen, 5653 LC Eindhoven (NL)
(74) Representative: Arnold & Siedsma
(86) International application number: PCT/EP2023/058733
(87) International publication number: WO 2023/194333

(56) References cited:
- EP-B1- 2 816 117
- TEULE F ET AL: "Biomimetic manufacturing of fibers", DESIGN AND NATURE: COMPARING DESIGN IN NATURE WITH SCIENCE ANDENGINEERING, WIT, SOUTHAMPTON, GB, 1 January 2002 (2002-01-01), pages 379 - 390, XP008082940
- MERLE C ET AL: "Hydroxylated human homotrimeric collagen I in Agrobacterium tumefaciens-mediated transient expression and in transgenic tobacco plant", FEBS LETTERS, ELSEVIER, AMSTERDAM, NL, vol. 515, 5 March 2002 (2002-03-05), pages 114 - 118, XP071243540, ISSN: 0014-5793, DOI: 10.1016/S0014-5793(02)02452-3
- PENG CONGYUE ANNIE ET AL: "Advances in Plant-Derived Scaffold Proteins", FRONTIERS IN PLANT SCIENCE, vol. 11, 25 February 2020 (2020-02-25), XP093062129, DOI: 10.3389/fpls.2020.00122
- XING XU ET AL: "Hydroxylation of recombinant human collagen type I alpha 1 in transgenic maize co-expressed with a recombinant human prolyl 4-hydroxylase", BMC BIOTECHNOLOGY, BIOMED CENTRAL LTD, vol. 11, no. 1, 24 June 2011 (2011-06-24), pages 69, XP021105282, ISSN: 1472-6750, DOI: 10.1186/1472-6750-11-69
- NUGENT J M ET AL: "PRODUCING HUMAN THERAPEUTIC PROTEINS IN PLASTIDS", CURRENT PHARMACEUTICAL DESIGN, BENTHAM SCIENCE PUBLISHERS, NL, vol. 11, no. 19, 1 January 2005 (2005-01-01), pages 2459 - 2470, XP009059415, ISSN: 1381-6128, DOI: 10.2174/1381612054367562
- LEE JAE HOON ET AL: "Golden Gate Cloning-Compatible DNA Replicon/2A-Mediated Polycistronic Vectors for Plants", FRONTIERS IN PLANT SCIENCE, vol. 11, 21 October 2020 (2020-10-21), XP093062135, DOI: 10.3389/fpls.2020.559365

## Description

### ABSTRACT AND FIELD OF INVENTION

The invention described herein relates to a novel non-naturally occurring, elastomeric animalfree recombinant fusion biopolymers produced in plants through transient expression as set out in the appended set of claims. More specifically, the present invention describes polynucleotides encoding fusion proteins of a Spidroin like protein with a human collagen. Beingr, either a Spidroin-I/Collagen Type-I fusion protein, capable of forming hydroxylated triple helix fibers, or a Fibroin-III/Collagen Type-I fusion protein, capable of forming hydroxylated triple helix fibers. The present invention has improved properties (e.g., thermostability, young's modulus, cell adhesion, degradability, and the like) versus that of native Collagen Type-I. Also described are methods for use thereof, such as the use of electrospun scaffolds which are particularly well suited for biomedical or cosmetic applications as defined in the claims.

### BACKGROUND OF THE PRIOR ART

The extracellular matrix (ECM) is the non-cellular component present within all tissues and organs, and provides not only essential physical scaffolding for the cellular constituents but also initiates crucial biochemical and biomechanical cues that are required for tissue morphogenesis, differentiation and homeostasis **[1].**

Moreover, the ECM is a highly dynamic structure that is constantly being remodeled, either enzymatically or non-enzymatically, and its molecular components are subjected to a myriad of post-translational modifications. Through these physical and biochemical characteristics the ECM generates the biochemical and mechanical properties of each organ, such as its tensile and compressive strength and elasticity, and also mediates protection by a buffering action that maintains extracellular homeostasis and water retention. In addition, the ECM directs essential morphological organization and physiological function by binding growth factors (GFs) and interacting with cell-surface receptors to elicit signal transduction and regulate gene transcription. The biochemical and biomechanical, protective and organizational properties of the ECM in a given tissue can vary tremendously from one tissue to another (e.g. lungs versus skin versus bone) and even within one tissue (e.g. renal cortex versus renal medulla), as well as from one physiological state to another (normal versus cancerous).

The ECM is composed of two main classes of macromolecules: proteoglycans (PGs) and fibrous proteins **[2, 3].** The main fibrous ECM proteins are collagens, elastins, and fibronectins **[4].** PGs fill the majority of the extracellular interstitial space within the tissue in the form of a hydrated gel **[2].** PGs have a wide variety of functions that reflect their unique buffering, hydration, binding and force-resistance properties. For example, in the kidney glomerular basement membrane, perlecan has a role in glomerular filtration **[5, 6].** By contrast, in ductal epithelial tissues, decorin, biglycan and lumican associate with collagen fibers to generate a molecular structure within the ECM that is essential for mechanical buffering and hydration and that, by binding growth factors (GFs), provides an easy, enzymatically accessible repository for these factors.

Collagen is the most abundant fibrous protein within the interstitial ECM and constitutes up to 30% of the total protein mass of a multicellular animal. Collagens, which constitute the main structural element of the ECM, provide tensile strength, regulate cell adhesion, support chemotaxis and migration, and direct tissue development **[7].** The bulk of interstitial collagen is transcribed and secreted by fibroblasts that either reside in the stroma or are recruited to it from neighboring tissues **[8].**

By exerting tension on the matrix, fibroblasts are able to organize collagen fibrils into sheets and cables and, thus, can dramatically influence the alignment of collagen fibers. Although within a given tissue, collagen fibers are generally a heterogeneous mix of different types, one type of collagen usually predominates.

The structure of collagen is a triple helix in which three polypeptide strands together form a helical coil. The individual polypeptide strands are composed of repeating triplet amino acid sequences designated as GLY-X-Y. X and Y can be any amino acid and the third amino acid is glycine. The amino acids proline and hydroxyproline are found in high concentrations in collagen. The most common triplet is proline-hydroxyproline-glycine (Gly-Pro-Hyp) accounting for approximately 10.5% of the triplets in collagen.

Collagen associates with elastin, another major ECM fiber. Elastin fibers provide recoil to tissues that undergo repeated stretch. Importantly, elastin stretch is crucially limited by tight association with collagen fibrils **[9].** Secreted tropoelastin (the precursor of elastin) molecules assemble into fibers and become highly crosslinked to one another via their lysine residues by members of the lysyl oxidase (LOX) enzyme family, which include LOX and LOXL **[10].** Elastin fibers are covered by glycoprotein microfibrils, mainly fibrillins, which are also essential for the integrity of the elastin fiber **[9].**

A third fibrous protein, fibronectin (FN) is intimately involved in directing the organization of the interstitial ECM and, additionally, has a crucial role in mediating cell attachment and function. FN can be stretched several times over its resting length by cellular traction forces **[11].** Such force-dependent unfolding of FN exposes cryptic integrin-binding sites within the molecule that result in pleiotropic changes in cellular behavior and implicate FN as an extracellular mechanoregulator.

Like FN, other ECM proteins such as tenascin exert pleiotropic effects on cellular behavior, including the promotion of fibroblast migration during wound healing **[12, 13].** Indeed, levels of tenascins C and W are elevated in the stroma of some transformed tissues where they can inhibit the interaction between syndecan4 and FN to promote tumor growth and metastasis **[13].**

Both collagen, elastin, and fibronectin are considered being biodegradable biopolymers, which are naturally occurring biomolecules with the highest degree of biocompatibility. In other words they are polymeric biomolecules. These also include other animal protein-based biopolymers such as wool, silk, gelatin, and polysaccharides such as cellulose, starch, carbohydrate polymers produced by bacteria and fungi. Biopolymers, especially the carbohydrate origin, have been found very promising for biomedical application in various forms because of their low toxicity, low antigenicity, high bio-activity, biodegradability, stability, processability to complicated shapes with appropriate porosity to support cell growth, mechanical properties, and renewable nature.

Biopolymers are generated from renewable sources and are easily biodegradable because of the oxygen and nitrogen atoms found in their structural backbone. Biodegradation converts them to CO², water, biomass, humid matter, and other natural substances. Biopolymers are thus naturally recycled by biological processes.

The bioactive properties such as antimicrobial, immune-modulatory, cell proliferative and angiogenic nature of the polymers create a microenvironment favorable for the healing process and provide a plethora of applications in pharmaceutical, medical, and cosmetic applications. One of the major advantageous properties of biopolymers though, is their ability to absorb large volumes of water when in the dry state and donate water when hydrated.

Both collagen and elastin are typically isolated from natural sources, such as bovine, porcine, or poultry hide, and for collagen also from cartilage, or bones. Bones are usually dried, defatted, crushed, and demineralized to extract collagen, while hide and cartilage are usually minced and digested with proteolytic enzymes. As collagen and elastin are resistant to most proteolytic enzymes, this procedure conveniently serves to remove most of the contaminating protein found with collagen or elastin.

Daniels et al U.S. Pat. No. 3,949,073, disclosed the preparation of soluble collagen by dissolving tissue in aqueous acid, followed by enzymatic digestion. The resulting atelopeptide collagen is soluble, and substantially less immunogenic than unmodified collagen. It may be injected into suitable locations of a subject with a fibril-formation promoter (described as a polymerization promoter in the patent) to form fibrous collagen implants in situ, for augmenting hard or soft tissue. This material is now commercially available from Collagen Corporation (Palo Alto, Calif.) under the trademark Zyderm^{®} collagen implant.

Luck et al U.S. Pat. No. 4,488,911, disclosed a method for preparing collagen in solution (CIS), wherein native collagen is extracted from animal tissue in dilute aqueous acid, followed by digestion with an enzyme such as pepsin, trypsin, or Pronase^{®}. The enzyme digestion removes the telopeptide portions of the collagen molecules, providing "atelopeptide" collagen in solution. The atelopeptide CIS so produced is substantially non-immunogenic, and is also substantially non-cross-linked due to loss of the primary crosslinking regions. The CIS may then be precipitated by dialysis in a moderate shear environment to produce collagen fibers which resemble native collagen fibers. The precipitated, reconstituted fibers may additionally be crosslinked using a chemical agent (for example aldehydes such as formaldehyde and glutaraldehyde), or using heat or radiation. The resulting products are suitable for use in medical implants due to their biocompatibility and reduced immunogenicity.

Wallace et al U.S. Pat. No. 4,424,208, disclosed an improved collagen formulation suitable for use in soft tissue augmentation. Wallace's formulation comprises reconstituted fibrillar atelopeptide collagen (for example, Zyderm^{®} collagen) in combination with particulate, crosslinked atelopeptide collagen dispersed in an aqueous medium. The addition of particulate crosslinked collagen improves the implant's persistence, or ability to resist shrinkage following implantation.

Smestad et al U.S. Pat. No. 4,582,640, disclosed a glutaraldehyde crosslinked atelopeptide CIS preparation (GAX) suitable for use in medical implants. The collagen is crosslinked under conditions favoring intrafiber bonding rather than interfiber bonding, and provides a product with higher persistence than noncross-linked atelopeptide collagen, and is commercially available from Collagen Corporation under the trademark Zyplast^{®} Implant.

Nguyen et al U.S. Pat. No. 4,642,117, disclosed a method for reducing the viscosity of atelopeptide CIS by mechanical shearing. Reconstituted collagen fibers are passed through a fine-mesh screen until viscosity is reduced to a practical level for injection.

Nathan et al U.S. Pat. No. 4,563,350, disclosed osteoinductive bone repair compositions comprising an osteoinductive factor, at least 5% nonreconstituted (afibrillar) collagen, and the remainder reconstituted collagen and/or mineral powder (e.g., hydroxyapatite). CIS may be used for the nonreconstituted collagen, and Zyderm^{®} collagen implant (ZCI) is preferred for the reconstituted collagen component. The material is implanted in bone defects or fractures to speed ingrowth of osteoclasts and promote new bone growth.

Chu U.S. Pat. No. 4,557,764, disclosed a "second nucleation" collagen precipitate which exhibits a desirable malleability and putty-like consistency. Collagen is provided in solution (e.g., at 2-4 mg/mL), and a "first nucleation product" is precipitated by rapid titration and centrifugation. The remaining supernatant (containing the bulk of the original collagen) is then decanted and allowed to stand overnight. The precipitated second nucleation product is collected by centrifugation.

Chu U.S. Pat. No. 4,689,399, disclosed a collagen membrane preparation, which is prepared by compressing and drying a collagen gel. The resulting product has high tensile strength.

J. A. M. Ramshaw et al, Anal Biochem (1984) 141:361-65, and PCT application WO87/04078 disclosed the precipitation of bovine collagen (types I, II, and III) from aqueous PEG solutions, where there is no binding between collagen and PEG.

Werner U.S. Pat. No. 4,357,274, disclosed a method for improving the durability of sclero protein (e.g., brain meninges) by soaking the degreased tissue in H2 O2 or PEG for several hours prior to lyophilizing. The resulting modified whole tissue exhibits increased persistence.

Hiroyoshi U.S. Pat. No. 4,678,468, disclosed the preparation of polysiloxane polymers having an interpenetrating network of water-soluble polymer dispersed within. The water-soluble polymer may be a collagen derivative, and the polymer may additionally include heparin. The polymers are shaped into artificial blood vessel grafts, and are designed to prevent clotting.

Other patents disclose the use of collagen preparations with bone fragments or minerals. For example, Miyata et al U.S. Pat. No. 4,314,380 disclosed a bone implant prepared by baking animal bone segments, and soaking the baked segments in a solution of atelopeptide collagen.

Deibig et al U.S. Pat. No. 4,192,021 disclosed an implant material which comprises powdered calcium phosphate in a pasty formulation with a biodegradable polymer (which may be collagen).

There are several references in the art to proteins modified by covalent conjugation to polymers, to alter the solubility, antigenicity and biological clearance of the protein. For example, U.S. Pat. No. 4,261,973 disclosed the conjugation of several allergens to PEG or PPG (polypropylene glycol) to reduce the proteins' immunogenicity. U.S. Pat. No. 4,301,144 disclosed the conjugation of hemoglobin with PEG and other polymers to increase the protein's oxygen carrying capability.

EPO 98,110 disclosed coupling an enzyme or interferon to a polyoxyethylene-polyoxypropylene (POE-POP) block polymer increases the protein's half-life in serum. U.S. Pat. No. 4,179,337 disclosed conjugating hydrophilic enzymes and insulin to PEG or PPG reduce immunogenicity.

Davis et al, Lancet (1981) 2:281-83 disclosed the enzyme uricase modified by conjugation with PEG to provide uric acid metabolism in serum having a long half-life and low immunogenicity.

Nishida et al, J Pharm Pharmacol (1984) 36.:354-55 disclosed PEG-uricase conjugates administered orally to chickens, demonstrating decreased serum levels of uric acid. Inada et al, Biochem & Biophys Res Comm (1984) 122:845-50 disclosed lipoprotein lipase conjugation with PEG to render it soluble in organic solvents.

M. Chvapil et al, J Biomed Mater Res (1969) 3:315-32 disclosed a composition prepared from collagen sponge and a crosslinked ethylene glycol monomethacrylate-ethylene glycol dimethacrylate hydrogel. The collagen sponge was prepared by lyophilizing an aqueous mixture of bovine hide collagen and methylglyoxal (a tanning agent). The sponge-hydrogel composition was prepared by polymerizing ethylene glycol monomethacrylate and ethylene glycol dimethacrylate in the sponge.

Kenton W. Gregory, U.S. Pat. No. US6667051B1 disclosed a method for joining at least one layer of pressed biomaterial to a tissue substrate, comprising: I) providing the pressed biomaterial consisting essentially of elastin or tropoelastin materials; II) applying a biodegradable cyanoacrylate adhesive to either one of the material and the tissue; and III) adhering the pressed biomaterial to the tissue and forming a substantially water-tight engagement therebetween.

Kenton W. Gregory et al, Pat. No. AU712868B2 disclosed elastin and elastin-based biomaterials and to methods of using same in tissue repair and replacement. The invention further relates to methods of securing the elastin and elastin-based biomaterials to existing tissue.

Nikolay Ouzounov et al, U.S. Pat. No. US11041015B2 disclosed non-naturally occurring full-length and truncated collagen molecules and full-length and truncated elastin molecules and their production, being recombinantly expressed in bacterial cells.

Véronique-Résidence Sainte Madeleine Gruber et al, Pat. No. EP0951537B1 disclosed the production of recombinant collagens by plants, in particular by stable expression, and in particular homocatalar type I collagen [α1 (I) 3 ], as well as their uses.

Klaus During, Pat. No. CA2336064A1 disclosed a method for the production of a fibrous protein, comprising the following steps: (a) expression of a precursor fibrous protein in a plant cell and (b) incubation of the precursor fibrous protein with a protein processing the latter. The invention also relates to the plant cells used for this purpose and to the fibrous proteins produced according to the inventive method.

Oded Shoseyov et al, Pat. No. US9783816B2 disclosed a method of producing at least one type of a collagen alpha chain in a manner enabling accumulation of the collagen alpha chain in a subcellular compartment devoid of endogenous P4H activity, thereby producing the collagen in the plant.

Kirivikko et al (WO 93/07889) described the expression of procollagens humans (types I and II). An example reports the expression of the chain alpha-1 or alpha-2 procollagen I or II in mammalian cells HT1080. A Another example describes the expression of the alpha and beta chains of prolyl-4-hydroxylase in Sf9 insect cells. Suggested examples for the expression of collagen and recombinant P4H are given for yeasts S. cerevisiae and P. pastoris, without demonstration of result.

While the mechanical properties and very useful biological agents of collagen, elastin, and fibronectin are recognized without ambiguity, the use of this protein is put back into question because of possible risks of contamination by unconventional infectious agents. Indeed, if the risks posed by bacterial contaminations or viruses can be perfectly controlled, it is not the same for those associated with prion-type agents.

These infectious agents that seem to be protein-like involved in the development of encephalopathies animal degenerative (scrapie of sheep, bovine spongiform encephalopathy) and human of Creutzfeld-Jacob, Gertstmann-Straussler syndrome, kuru). The period of their possible expression is such formal controls are difficult to achieve. These risks have already virtually blocked any marketing of human collagen and the provisions regulations made with regard to collagens concerned animals complicate purification processes and increase their cost.

In view of these difficulties and in the face of a radical deterioration in the image of mammalian collagen, one solution is the production of recombinant collagen which can be easily purified in a system which is not likely to cause pathogenic risks for humans and whose industrial cost is not prohibitive. The inventors have therefore developed the present invention e.g. a recombinant spidroin/collagen fusion protein transiently produced in plants.

Animal cells are, a priori, more adapted to the expression of mammalian genes. The enzymatic equipment that carries out the post-translational maturation however is different from a tissue, from one organ or species to another. For example, it has been reported that the post-translational maturation of a plasma protein may be different when it is obtained from human blood or when it is produced by a recombinant cell like cells of Chinese hamster ovaries or in the milk of an animal transgenic. In addition, low levels of expression obtained with mammalian cells involve in vitro cultures in very large volumes at high costs. The production of recombinant proteins in the milk of transgenic animals (mice, sheep, and cows) reduces the costs of production and overcome problems of expression level. However, there are still problems of ethics and contaminations viral and subviral (prions).

For these reasons, there has been an urgent need in developing novel biodegradable biopolymers that have a wider pleiotropic anti-inflammatory action, and improved toxicity, antigenicity, bio-activity, biodegradability, stability, processability, mechanical properties and renewable nature. Therefore, there is still the need to produce such biodegradable biopolymers with the aforementioned capabilities, obtained from a low-cost and highly productive process, in a highly purified state which served as the inspiration to develop present invention.

Consequential to its essential role as a mechanical support and affinity regulator in extracellular matrices, both collagen and silk constitutes a highly sought after scaffolding material for regeneration and healing applications. However, substantiated concerns have been raised with regard to quality and safety of animal tissue-extracted collagen or spider- or silkworm-derived silk, particularly in relation to its immunogenicity, risk of disease transmission and overall quality and consistency. In parallel, contamination with undesirable cellular factors can significantly impair its bioactivity, vis-a-vis its impact on cell recruitment, proliferation and differentiation.

Overall, the shortcomings of animal- and cadaver-derived collagens arising from their source diversity and recycled nature are fully overcome by the present invention.

### DESCRIPTION OF THE INVENTION

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Notwithstanding the exemplary embodiments described hereinbelow, is the present invention only limited by the attached claims. The attached claims are hereby explicitly incorporated in this detailed description, in which each claim, and each combination of claims as allowed for by the dependency structure defined by the claims, forms a separate embodiment of the present invention.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof.

In the present described invention, various specific details are presented. Embodiments of the present invention can be carried out without these specific details. Furthermore, well-known features, elements and/or steps are not necessarily described in detail for the sake of clarity and conciseness of the present disclosure.

Reference throughout this specification to "one embodiment" or "an embodiment" or "embodiments" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

The non-essential improvement and adjustment made by the method disclosed by the invention should still be within the protection scope of the invention. Meanwhile, raw materials being used are not always described in detail. The raw materials may be commercially available products. The process steps or preparation methods that are not always described in detail are process steps or preparation methods which are known by those skilled in the art.

The raw materials used to obtain the present invention are commercially available products; the process steps or the preparation method which are also not always described in detail, are process steps or preparation methods which are all known by those skilled in the art.

As used herein the term "about" refers to ± 10 %.

The term "consisting of means "including and limited to".

The term "consisting essentially of" means that the composition, process or structure may contain additional components, steps and / or parts, but only if the additional components, steps and / or parts meet the basic and novel properties of the claimed composition and therefor do not materially alter the basic and novel characteristics of the claimed composition and/or method or structure.

As used herein, the singular forms "a", "an" and "the" include plural references unless the context clearly indicates otherwise. For example, the term "a compound" or "at least one compound" can encompass a variety of compounds, including mixtures thereof which can be used in foods, cosmetics, pharmaceuticals, industrial products, medical products, laboratory culture growth media, and many other applications.

Throughout this application, various embodiments of this disclosure may be presented in a range format. It should be understood that the description in range format is merely for convenience and brevity and should not be construed as an inflexible limitation on the scope of the disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals there between.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

In the description provided herein, numerous specific details are set forth. However, it is understood that embodiments of the invention may be practiced without these specific details. In other instances, well-known methods, structures and techniques have not been shown in detail in order not to obscure an understanding of this description.

It is an object of the present invention to provide a novel fusion biopolymers consisting of 2 moieties being: I) a recombinant non-human scleroprotein, and a II) human recombinant Collagen Type I. The non-human scleroprotein, being selected from a fibrin or spidroin, also commonly referred to as spidroin-like proteins, as set out in the appended set of claims
In one embodiment the present invention provides a novel fusion biopolymers consisting of 2 moieties being: I) recombinant a Fibroin-III, and a II) human recombinant Collagen Type I, as set out in the appended set of claims.

In one embodiment the present invention provides a novel fusion biopolymers consisting of 2 moieties being: I) recombinant spidroin-I, and a II) human recombinant Collagen Type I, as set out in the appended set of claims.

It is an object of the present invention to provide the novel fusion biopolymers as herein disclosed in large scale in which proteins can readily be manipulated to polymerize into fibers at wish.

The term "collagen" or "collagen-like" as used herein refers to a monomeric polypeptide that can form a quaternary structure with one or more collagen or collagen-like polypeptides. The quaternary structure of natural collagen is a triple helix typically composed of three polypeptides. Of the three polypeptides that form natural collagen, two are usually identical and are designated as the alpha chain. The third polypeptide is designated as the beta chain. Thus a typical natural collagen can be designated as AAB, wherein the collagen is composed of two Colα1(I) chains and one Colα2(I) chain. The term "procollagen" as used herein refers to polypeptides produced by cells that can be processed to naturally occurring collagenThe collagen chain expressed in this present invention, is an alpha 1 and 2 chain of type I as set out in the appended set of claims.

The term "Spidroins" as used herein refers to the main proteins in spider silk. Different types of spider silk contain different spidroins, all of which are members of a single protein family. The two most ubiquitous types of spidroins are the major ampullate silk proteins (MaSp) used in the construction of dragline silk. Dragline silk fiber is made up of two types of spidroins being: the Major Ampulate Spidroin-1 (MaSp1) and Spidroin-2 (MaSp2) proteins.

The term "fibroin" as used herein refers to insoluble scleroproteins comprising the filaments of raw silk fiber obtained from spiders or silkworms. Preferably, fibroin is obtained from spider species by means of recombinant technology. Alternatively fibroin may be even well obtained from silkworm species, for example but not limited to Bombyx mori and other moth genera such as Antheraea, Cricula, Samia and Gonometa, by means of obtaining fibroin from a solution containing dissolved silkworm silk, or by recombinant technology. Spidroin and Fibroin are typically obtained from spider species including, but not limited to: Arachnura higginsi, Araneus circulissparsus, Araneus diadematus, Argiope picta, Banded Garden Spider (Argiope trifasciata), Batik Golden Web Spider (Nephila antipodiana), Beccari's Tent Spider (Cyrtophora beccarii), Bird-dropping Spider (Celaenia excavata), Black-and-White Spiny Spider (Gasteracantha kuhlii), Black-and-yellow Garden Spider (Argiope aurantia), Bolas Spider (Ordgarius furcatus), Bolas Spiders - Magnificent Spider (Ordgarius magnificus), Brown Sailor Spider (Neoscona nautica), Brown-Legged Spider (Neoscona rufofemorata), Capped Black-Headed Spider (Zygiella calyptrata), Common Garden Spider (Parawixia dehaani), Common Orb Weaver (Neoscona oxancensis), Crab-like Spiny Orb Weaver (Gasteracantha cancriformis (elipsoides)), Curved Spiny Spider (Gasteracantha arcuata), Cyrtophora moluccensis, Cyrtophora parnasia, Dolophones conifera, Dolophones turrigera, Doria's Spiny Spider (Gasteracantha doriae), Double-Spotted Spiny Spider (Gasteracantha mammosa), Double-Tailed Tent Spider (Cyrtophora exanthematica), Aculeperia ceropegia, Eriophora pustulosa, Flat Anepsion (Anepsion depressium), Four-spined Jewel Spider (Gasteracantha quadrispinosa), Garden Orb Web Spider (Eriophora transmarina), Giant Lichen Orbweaver (Araneus bicentenarius), Golden Web Spider (Nephila maculata), Hasselt's Spiny Spider (Gasteracantha hasseltii), Tegenaria atrica, Heurodes turrita, Island Cyclosa Spider (Cyclosa insulana), Jewel or Spiny Spider (Astracantha minax), Kidney Garden Spider (Araneus mitificus), Laglaise's Garden Spider (Eriovixia laglaisei), Long-Bellied Cyclosa Spider (Cyclosa bifida), Malabar Spider (Nephilengys malabarensis), MultiColoured St Andrew's Cross Spider (Argiope versicolor), Ornamental Tree-Trunk Spider (Herennia ornatissima), Oval St. Andrew's Cross Spider (Argiope aemula), Red Tent Spider (Cyrtophora unicolor), Russian Tent Spider (Cyrtophora hirta), Saint Andrew's Cross Spider (Argiope keyserlingi), Scarlet Acusilas (Acusilas coccineus), Silver Argiope (Argiope argentata), Spinybacked Orbweaver (Gasteracantha cancriformis), Spotted Orbweaver (Neoscona domiciliorum), St. Andrews Cross (Argiope aetheria), St. Andrew's Cross Spider (Argiope Keyserlingi), Tree-Stump Spider (Poltys illepidus), Triangular Spider (Arkys clavatus), Triangular Spider (Arkys lancearius), Two-spined Spider (Poecilopachys australasia), Nephila species, e.g. Nephila clavipes, Nephila senegalensis, and Nephila madagascariensis. In the present invention the Spidroin proteins are derived from Nephila clavipes, and the Fibroin proteins from Araneus diadematus, as set out in the appended set of claims.

According to one aspect of the present invention, a method of producing a Collagen protein in a plant or an isolated plant cell comprising expressing in the plant or the isolated plant cell at least one type of a Collagen Alpha Chain and exogenous Proline 4-hydroxylase (P4H) in a manner enabling accumulation of the at least one type of the Collagen Alpha Chain and the exogenous P4H in a subcellular compartment devoid of endogenous P4H activity, thereby producing a collagen protein in the plant is defined in the claims. In prior described art, an attempt to produce human collagens that rely on the hydroxylation machinery naturally present in plants resulted in collagen that is poor in proline hydroxylation which has been described by Merle et al., 2002 **[14].** Such collagen melts or loses its triple helical structure at temperatures below 30 °C. Co-expression of collagen and prolyl-hydroxylase results with stable hydroxylated collagen that is biologically relevant for applications at body temperatures **[14].** As is used herein, the phrase "subcellular compartment devoid of endogenous P4H activity" refers to any compartmentalized region of the cell which does not include plant P4H or an enzyme having plant-like P4H activity. Examples of such subcellular compartments include the vacuole, apoplast and cytoplasm as well as organelles such as the chloroplast, mitochondria and the like. Accumulation of the expressed collagen chain in a subcellular compartment devoid of endogenous P4H activity can be effected via any one of several approaches. For example, the expressed collagen chain can include a signal sequence for targeting the expressed protein to a subcellular compartment such as the apoplast or more preferably, the chloroplast or other organelles such as the mitochondria. Examples of suitable signal sequences include the chloroplast transit peptide (included in Uniprot entry G5DBJ0, amino acids 1- 40) and the Mitochondrion transit peptide (included in Uniprot entry Q9ZP06, amino acids 1- 22). The Examples section which follows provides additional examples of suitable signal sequences as well as guidelines for employing such signal sequences in expression of collagen chains in plant cells. Alternatively, the sequence of the collagen chain can be modified in a way which alters the cellular localization of collagen when expressed in plants. As is mentioned hereinabove, the endoplasmic reticulum (ER) of plants includes a P4H which is incapable of correctly hydroxylating collagen chains. Collagen alpha chains natively include an ER targeting sequence which directs expressed collagen into the ER where it is post-translationally modified (including incorrect hydroxylation). Thus, removal of the ER targeting sequence will lead to cytoplasmic accumulation of collagen chains which are devoid of post translational modification including any hydroxylations.

As is also mentioned hereinabove, hydroxylation of alpha chains is required for assembly of a stable type I collagen. Full collagen proline hydroxylation also significantly raises the melting temperature by stabilizing the collagen triple helix, a process that is well understood by those known in the art. Since alpha chains expressed by transient expression in the wildtype plant of the present invention accumulate in a compartment devoid of endogenous P4H activity, such chains must normally be isolated from the plant, plant tissue or cell and correctly hydroxylated using an in-vitro technique which can be achieved by the method of Torre-Blanco A, Alvizouri A. [15]. However, such method is cumbersome and costly to achieve the desired effect. To overcome this limitation, the method of the present invention also transiently co-expresses P4H which is capable of correctly hydroxylating the collagen alpha chain(s) (i.e., hydroxylating only the proline (Y) position of the Gly-X-Y triplets). P4H is an enzyme composed of two subunits, alpha and beta, and both are needed to form an active catalytic enzyme **[16].** Mammalian prolyl 4-hydroxylase is an alpha-2/beta-2 tetramer **[17].** The 59-kDa alpha subunit contains the substrate-binding domain and the enzymic active site **[18].** Humans and most other vertebrates have three isoforms of the alpha subunit, isoform alpha-1 is the most prevalent. The pair of alpha subunits can be any of the three isoforms **[19].** The 55-kDa beta subunit is protein disulphide isomerase (PDI), which has additional functions in collagen formation. As part of P4H it retains the tetramer in the ER lumen and maintains the otherwise insoluble alpha subunit in an active form. In prior art, the inventors of patent no° EP2816117B1, Collagen producing plants and methods of generating and using same, used an exogenous human P4H to generate a stable transformant (e.g., transgenic) plant, capable of producing human P4H with the objective to correctly hydroxylate only the proline (Y) position of the Gly-X-Y triplets. However, tetrameric human P4H is inhibited by poly(L-proline) by extensine molecules that are substrates of plant prolyl-4-hydroxylase which are rich in Ser-(Pro)₄-Ser-Pro-Ser-(Pro)₄ sequences and thus could inhibit P4H of mammalian origin. To overcome this limitation, the inventor of the present invention, uses an alternative approach and generated a chimeric alpha/beta dimer with the same specific activity as native human P4H but without the inhibition potential by poly-(L-proline), as set out in the appended set of claims.

According to one aspect of the present invention, a method of producing a fibrillar Spidroin-I/Collagen Type-I fusion protein is provided comprising transiently co-expressing two vectors in which vector 1 expresses A) a spidroin-I chain, B) a Collagen Type-I alpha-I chain, and C) a Collagen Type-I Alpha-II chain, wherein transient expression is configured such that the Spidroin-I chain and the Collagen Alpha-I and Alpha-II chains are each capable of accumulating in a subcellular compartment devoid of both endogenous P4H and LH activity. Such compartment is preferably the chloroplast and is functionalized by using a transit signal leading to the chloroplast. Vector 2 expresses A) the aforementioned chimeric P4H and B) LH3, both of which are capable of accumulating in the subcellular compartment devoid of both endogenous P4H and LH activity. Both vectors are preferably targeted to a the chloroplast by introducing a chloroplast transit peptide at the N-terminal of the respective gene constructs. Such transit peptide is preferably, but not limited to, the chloroplastic Protein Chaperone-Like Protein of POR1. The respective genes assembled in both expression vectors are separated by introducing so-called "2A self-cleaving peptides" which can induce ribosomal skipping during translation of a protein in a cell, thus making it possible to generate multiple separated sequences expressed within a single transcript. Such fusion protein is termed "SPID1COL1" from here on in this present invention. **The amino acid sequence encoding for the SPID1COL1 fusion protein in vector 1 is set forth by SEQ NO: 15. Its respective nucleotide sequence is set forth by SEQ NO: 16. Preferably, the nucleotide sequence has been optimized for chloroplast expression in N. benthamiana. The amino acid sequences encoding for the P4H/LH3 proteins in vector 2 are set forth by SEQ NO: 17. Their respective nucleotide sequence is set forth by SEQ NO: 18. Preferably, the nucleotide sequence has been optimized for chloroplast expression in N. benthamiana.**

According to one aspect of the present invention, a method of producing a fibrillar Fibroin-III/Collagen Type-I fusion protein is provided comprising transiently co-expressing two vectors in which vector 1 expresses A) a Fibroin-III chain, B) a Collagen Type-I alpha-I chain, and C) a Collagen Type-I Alpha-II chain, wherein transient expression is configured such that the Fibroin-III chain and the Collagen Alpha-I and Alpha-II chains are each capable of accumulating in a subcellular compartment devoid of both endogenous P4H and LH activity. Such compartment is preferably the chloroplast and is functionalized by using a transit signal leading to the chloroplast. Vector 2 expresses A) the aforementioned chimeric P4H and B) LH3, both of which are capable of accumulating in the subcellular compartment devoid of both endogenous P4H and LH activity. Both vectors are preferably targeted to a the chloroplast by introducing a chloroplast transit peptide at the N-terminal of the respective gene constructs. Such transit peptide is preferably, but not limited to, the chloroplastic Protein Chaperone-Like Protein of POR1. The respective genes assembled in both expression vectors are separated by introducing so-called "2A self-cleaving peptides" which can induce ribosomal skipping during translation of a protein in a cell, thus making it possible to generate multiple separated sequences expressed within a single transcript. Such fusion protein is termed "FIB3COL1" from here on in this present invention. **The amino acid sequence encoding for the FIB3COL1 fusion protein in vector 1 is set forth by SEQ NO: 19. Its respective nucleotide sequence is set forth by SEQ NO: 20. Preferably, the nucleotide sequence has been optimized for chloroplast expression in N. benthamiana. The amino acid sequences encoding for the P4H/LH3 proteins in vector 2 are set forth by SEQ NO: 17. Their respective nucleotide sequence is set forth by SEQ NO: 18. Preferably, the nucleotide sequence has been optimized for chloroplast expression in N. benthamiana.**

According to still further features in the described preferred embodiments the plant is selected from the group consisting of Tobacco, Maize, Alfalfa, Rice, Potato, Soybean, Tomato, Wheat, Barley, Canola, beets, sunflower, and Cotton, more preferably Nicotiana benthamiana in which the portion of the is leaves, seeds, roots, tubers or stems, more preferably the leaves.

Plant: is generally understood as meaning any single- or multi-celled organism or a cell, tissue, organ, part or propagation material (such as seeds or fruit) of same which is capable of photosynthesis. Included for the purpose of the invention are all genera and species of higher and lower plants of the Plant Kingdom. Annual, perennial, monocotyledonous and dicotyledonous plants are preferred. The term includes the mature plants, seed, shoots and seedlings and their derived parts, propagation material (such as seeds or microspores), plant organs, tissue, protoplasts, callus and other cultures, for example cell cultures, and any other type of plant cell grouping to give functional or structural units. Mature plants refer to plants at any desired developmental stage beyond that of the seedling. Seedling refers to a young immature plant at an early developmental stage. Annual, biennial, monocotyledonous and dicotyledonous plants are preferred host organisms for the generation of transgenic plants. The expression of genes is furthermore advantageous in all ornamental plants, useful or ornamental trees, flowers, cut flowers, shrubs or lawns. Plants which may be mentioned by way of example but not by limitation are angiosperms, bryophytes such as, for example, Hepaticae (liverworts) and Musci (mosses); Pteridophytes such as ferns, horsetail and club mosses; gymnosperms such as conifers, cycads, ginkgo and Gnetatae; algae such as Chlorophyceae, Phaeophpyceae, Rhodophyceae, Myxophyceae, Xanthophyceae, Bacillariophyceae (diatoms), and Euglenophyceae. Most preferred are plants which are not used for food or feed purpose such as Arabidopsis thaliana, or preferably Nicotiana tabacum, or most preferably Nicotiana benthamiana. Alternatively, plants which are used for food or feed purposes can be used as well, such as the families of the Leguminosae such as pea, alfalfa and soya; Gramineae such as rice, maize, wheat, barley, sorghum, millet, rye, triticale, or oats; the family of the Umbelliferae, especially the genus Daucus, very especially the species carota (carrot) and Apium, very especially the species Graveolens dulce (celery) and many others; the family of the Solanaceae, especially the genus Lycopersicon, very especially the species esculentum (tomato) and the genus Solanum, very especially the species tuberosum (potato) and melongena (eggplant), and the genus Capsicum, very especially the species annuum (peppers) and many others; the family of the Leguminosae, especially the genus Glycine, very especially the species max (soybean), alfalfa, pea, lucerne, beans or peanut and many others; and the family of the Cruciferae (Brassicacae), especially the genus Brassica, very especially the species napus (oil seed rape), campestris (beet), oleracea cv Tastie (cabbage), oleracea cv Snowball Y (cauliflower) and oleracea cv Emperor (broccoli); and of the genus Arabidopsis, very especially the species thaliana and many others; the family of the Compositae, especially the genus Lactuca, very especially the species sativa (lettuce) and many others; the family of the Asteraceae such as sunflower, Tagetes, lettuce or Calendula and many other; the family of the Cucurbitaceae such as melon, pumpkin/squash or zucchini, and linseed. Further preferred are cotton, sugar cane, hemp, flax, chillies, and the various tree, nut and wine species.

According to still further features in the described preferred embodiments the plant is subjected to a stress condition. Such stress conditions are selected from the group consisting of drought, salinity, injury, cold and spraying with stress inducing compounds and/or compounds known in the art to increase endogenous ascorbate (Vitamin C) levels. As both P4H and LH3 enzymes are long known to suffer oxidative inactivation during catalysis, and the cofactor ascorbate (vitamin C) is required to reactivate the enzyme by reducing its iron center from Fe(III) to Fe(II), it may be beneficial to administer Vitamin C by means of biofortification **[21].**

According to another aspect of the present invention there is provided a method of transiently expressing or isolated plant cell capable of accumulating a collagen alpha chain having a hydroxylation pattern identical to that produced when the collagen alpha chain is expressed in human cells.

The term "transient expression" or "transient gene expression" as used herein refers to the temporary expression of genes that are expressed for a short time after a nucleic acid, most frequently plasmid DNA encoding an expression cassette, has been introduced into eukaryotic cells. Transient expression should result in a time-limited use of transferred nucleic acids, since any long-term expression would be called "stable expression". The use of transient expression in a plant cell or a plant according to the invention makes it possible to produce high yields compatible with a commercial exploitation. In the case of transient expression, harvesting of the plant biomass takes place during peak expression of the recombinant protein, e.g., typically 5 to 9 days after transfection. Transient expression of the aforementioned recombinant fusion proteins in Nicotiana Benthamiana plants may advantageously enable a high throughput platform to produce the compound at industrial scale and/or at a low cost. However, embodiments of the present invention are not necessarily limited thereto, e.g. any other suitable expression host may equally be used, including but not limited to, bacteria, yeast, insect, mammalian, or other plant expression systems.

The term "fluorescent protein" is a protein that is commonly used in genetic engineering technologies used as a reporter of expression of an exogenous polynucleotide. The protein when exposed to ultraviolet or blue light fluoresces and emits a bright visible light. Proteins that emit green light is green fluorescent protein (GFP) and proteins that emit red light is red fluorescent protein (RFP).

The term "gene" as used herein refers to a polynucleotide that encodes a specific protein, and which may refer to the coding region alone or may include regulatory sequences preceding (5' non-coding sequences) and following (3' non-coding sequences) the coding sequence.

The term "host cell" is a cell that is programmed to express an introduced exogenous polynucleotide, preferably this host cell is the chloroplast subcellular compartment of Nicotiana benthamiana (N. benthamiana).

The term "non-naturally occurring" as used herein refers to collagen, Spidroin, or Fibroin, that is not normally found in nature. The non-naturally occurring collagen, Spidroin and Fibroin moieties are recombinantly prepared. The non-naturally occurring Collagen, Spidroin, or Fibroin protein is a recombinant Collagen, recombinant Spidroin, or recombinant Fibroin.

The term "signal peptide" refers to an amino acid sequence that recruits the host cell's cellular machinery to transport an expressed protein to a particular location or cellular organelle of the host cell. Preferably the target peptide sequence is located on the C-terminal end of the amino acid structure of the protein. Preferably the signal peptide is a transit peptide, functionalizing targeting to the chloroplast.

According to another aspect of the present invention, the resulting Spidroin/Collagen or Fibroin/Collagen fusion proteins are purified after extraction from the plant or plant cells that express it. In order to facilitate their purification, the fusion proteins may be expressed in fusion with tags (His6, GST, MBP, FLAG etc.) which will preferably be located in the N- or C-terminal position of the mature protein.

The general methods of growing plants, as well as methods for introducing expression vectors into plant tissue, are available to those skilled in the art. They are varied and depend on the selected plant. In general, this method comprises a first step of cultivating the plant, aeroponic or hydroponic, preferably free float culture, and under LED lighting. After this first step, in particular five weeks of hydroponic culture on free floats, agroinfiltration plants is carried out under vacuum, by agrobacteria comprising a DNA fragment coding for the aforementioned Spidroin/Collagen or Fibroin/Collagen fusion proteins according to the invention. This step of agroinfiltration can be implemented by any means to evacuate. Preferably, in the method used according to the invention, it is carried out under vacuum by the Venturi effect. Agrobacterium refers to a soil-borne, Gram-negative, rod-shaped phytopathogenic bacterium which causes crown gall. The term "Agrobacterium" includes, but is not limited to, the strains Agrobacterium tumefaciens (which typically causes crown gall in infected plants), and Agrobacterium rhizogenes (which causes hairy root disease in infected host plants). Infection of a plant cell with Agrobacterium generally results in the production of opines (e.g., nopaline, agropine, octopine, etc.) by the infected cell. Thus, Agrobacterium strains which cause production of nopaline (e.g., strain LBA4301, C58, A208) are referred to as "nopaline-type" Agrobacteria; Agrobacterium strains which cause production of octopine (e.g., strain LBA4404, Ach5, B6) are referred to as "octopine-type" Agrobacteria; and Agrobacterium strains which cause production of agropine (e.g., strain EHA105, EHA101, A281) are referred to as "agropine-type" Agrobacteria.

After agroinfiltration, the plants are typically further cultured for 5 to 9 days. Finally, the protein is extracted and purified using industry-standard methods known in the art.

The term "expression vector" or "vector" as used herein refers to a nucleic acid assembly which is capable of directing the transient expression of the exogenous gene. The expression vector may include a promoter which is operably linked to the exogenous gene, restriction endonuclease sites, nucleic acids that encode one or more selection markers, and other nucleic acids useful in the practice of recombinant technologies. Preferably, the expression vector used in step a) comprises: prokaryotic DNA elements encoding an origin of bacterial replication and an antibiotic resistance gene; at least one heterologous nucleotide sequence coding for the aforementioned fusion proteins according to the invention operatively linked to a strong promoter, preferably a 35S promoter; an expression cassette for the expression of a silencing inhibitor, preferably p19; and DNA elements that control the processing of transcripts, such as termination / polyadenylation sequences, preferably the Tnos sequence. Numerous plant functional expression promoters and enhancers which can be either tissue specific, developmentally specific, constitutive or inducible can be utilized in conjunction with the constructs of the present invention. As used herein in the specification and in the claims section that follows the phrase "plant promoter" or "promoter" includes a promoter which can direct gene expression in plant cells (including DNA containing organelles, more specifically the protoplast). Such a promoter can be derived from a plant, bacterial, viral, fungal or animal origin. Such a promoter can be constitutive, i.e., capable of directing high level of gene expression in a plurality of plant tissues, tissue specific, i.e., capable of directing gene expression in a particular plant tissue or tissues, inducible, i.e., capable of directing gene expression under a stimulus, or chimeric, i.e., formed of portions of at least two different promoters. The plant promoter employed can be a constitutive promoter, a tissue specific promoter, an inducible promoter or a chimeric promoter. Examples of constitutive plant promoters include, without being limited to, CaMV35S and CaMV19S promoters, FMV34S promoter, sugarcane bacilliform badnavirus promoter, CsVMV promoter, Arabidopsis ACT2/ACT8 actin promoter, Arabidopsis ubiquitin UBQ1 promoter, barley leaf thionin BTH6 promoter, and rice actin promoter. Examples of tissue specific promoters include, without being limited to, bean phaseolin storage protein promoter, DLEC promoter, PHS promoter, zein storage protein promoter, conglutin gamma promoter from soybean, AT2S1 gene promoter, ACT11 actin promoter from Arabidopsis, napA promoter from Brassica napus and potato patatin gene promoter.

Collagen and silk are extensively used in the biomedical, regenerative medicine, food and cosmetics industry. Thus, although for both collagen and silk fiber components and modifying enzymes expressed by plants find utility in industrial synthesis of collagen and silk, complete collagen and/or silk production in plants is preferred for its simplicity and cost effectiveness.

The present invention successfully addresses the shortcomings of the presently known collagen configurations by providing a plant capable of expressing correctly hydroxylated Spidroin/Collagen or Fibroin/Collagen fusion proteins with improved properties (e.g., thermostability, young's modulus, cell adhesion, and the like) versus that of native human collagen. The resulting Spidroin/Collagen or Fibroin/Collagen fusion proteins thus obtained can be used in biomedical applications, cosmetics, esthetics, but not limited thereof.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting. Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

### EXAMPLES

A large quantity of biochemically modified, active recombinant photolyase fusion protein, highly purified in N. Benthamiana plants, can be obtained. To facilitate rapid, simple purification of the recombinant photolyase fusion protein, a 6xHis tag, also known as polyhistidine tag, His6 tag and/or hexa histidine tag, may be attached to the N-terminus of the protein. We have demonstrated that the recombinant fusion proteins are biologically active and show improved functionality when compared to native heterotrimeric Collagen Type I.

N. benthamiana is a particularly suitable bioreactor for the transient expression of recombinant protein in a manufacturing setting. The small ornamental plant has a high leaf to stem ratio and is very prolific in hydroponic culture. N. benthamiana tolerates the transfection vectors and delivers maximum synthesis of heterologous proteins in 5-7 days after transfection. Scale-up of this bioreactor is a matter of growing more plants, not re-engineering processes.

Plants have all the eukaryotic cell machinery to accurately produce human and animal proteins. Thus, the bioreactor is the individual plant. Plants are well suited to express complex proteins such as monoclonal antibodies, and minimize risk by not supporting growth of human or animal pathogens.

For all agroinfiltration experiments, discussed hereinbelow, 5-week-old N. benthamiana plants were used.

N. Benthamiana seeds were grown in a greenhouse. Seedling and germination of N. benthamiana plants were carried out under LED illumination in a 16/8 h light/dark cycle, 7 days/week. Red and blue diodes were selected that match the action spectrum of photosynthesis (25% blue and 75% red). Other wavelengths were not productive. The LED's were focused on the plants. Plants grown to usable maturity 20% faster in this system as compared to other commercial solutions. All seeds were germinated using rockwool growing medium at 26.6°C, using an ebb and flow hydroponic, well known in the art.

### EXAMPLE ONE: Spidroin-I/Collagen Type-I fusion protein

As aforementioned, two separate vectors are constructed. One of which carries the Spidroin-1/Collagen Type-I construct, the other one carrying the P4H/LH3 construct. Both vectors are designed to target a subcellular compartment known to be devoid of endogenous P4H or LH activity.

The inventor constructed a fundamental set of Golden Gate cloning-compatible modular vectors which comprise the expression cassette and acceptor backbones named GGC-TC1 and GGC-TC2. The acceptor backbone is a binary T-DNA vector suitable for transient expression in plants and is designed to possess the geminiviral replicon system, capable of producing circular DNA replicons for high-level multiple protein expression.

### 1) Spidroin-I/Collagen Type I Tricistronic construct:

For the biosynthesis of the genes of interest; Spidroin-1 (Uniprot: P19837, entry version N° 69, 2017-03-15), Collagen Type-I Alpha-I without the signal peptide (1-22) and without the N-terminal propeptide (23-161) and C-terminal propeptide (1219-1464) (Uniprot: P02452, entry version N° 22, 2017-05-10), Collagen Type-I Alpha-II without the signal peptide (1-22) and without the N-terminal propeptide (23-79) and C-terminal propeptide (1120-1366) (Uniprot: P08123, entry version N° 202, 2017-05-10), an A2 sequence GSGEGRGSLLTCEDVEENPGP placed between the Spidroin-I, Collagen Type-I Alpha-I, and Collagen Type-I Alpha-II chain, a chloroplast transit peptide (included in Uniprot entry G5DBJ0, amino acids 1-40, entry version N° 10, 2017-11-22) placed at the N-terminus, and a 6x HIS Tag were used as a template.

### 2) P4H alpha-beta chimeric/LH3 Tricistronic vector construct:

For the biosynthesis of the genes of interest; a chimeric P4H enzyme comprising: an alpha subunit (Uniprot: Q86KR9, entry version N° 81, 2017-06-07) and a beta subunit (Uniprot: P05307, entry version N° 141, 2017-05-10) sequence without its native signal peptide (1-20), an A2 sequence GSG EGRGSLLTCEDVEENPGP placed between the alpha subunit and beta subunit, a LH3 sequence without its native signal peptide (1-24) (Uniprot: O60568, entry version N° 165, 2017-09-27), an A2 sequence GSGEGRGSLLTCEDVEENPGP placed between LH3 and the PH4 beta subunit sequence, and a chloroplast transit peptide (included in Uniprot entry G5DBJ0, amino acids 1-40, entry version N° 10, 2017-11-22) placed at the N-terminus, were used as a template.

Using the Golden Gate cloning approach, a T2A-linked tricistronic vector, whereby three transgenes encoding I) Spidroin-I and II) Collagen Type-I proteins (termed SPID1COL1) were combinatorially placed along the expression cassette in the binary vector. The P4H alpha-beta chimeric/LH3 constructs also comprises 3 genes (e.g., P4H alpha subunit, P4H beta subunit, and LH3, termed P4HLH3) and were combinatorially placed along the expression cassette.

Using the assembly protocol described in the Golden Gate modular cloning system (Weber et al., 2011) [23], so-called "level-0" modular vectors containing parts of the expression cassette, such as promoter (Pro), T2A signals (T2A), coding sequences (CDS), and terminator (Ter), were constructed. All level-0 modules were flanked by inward-facing Bsal restriction enzyme sites and fusion sites (5 bp-overhangs) to allow directional linear assembly in a Pro-CDS1-T2A-CDS2-T2A-CDS3-Ter (SPID1COL1) and Pro-CDS1-T2A-CDS2-T2A-CDS3-Ter (P4HLH3) orientation, resulting in a T2A-linked tricistronic constructs. In order to construct Pro, T2A, and Ter modules, sequences of CmYLCV promoter, and AtHSP 3' UTR were retrieved from publications of the prior art being: Stavolone et al. (2003) **[24],** Nagaya et al. (2010) **[25],** and Liu et al. (2017) **[26],** respectively.

As the aforementioned genes described above, employ tandem rare codons and could reduce the efficiency of translation or even disengage the translational machinery, the codon usage bias in N. benthamiana was used by upgrading the codon adoption index (CAI) from 0.70 to 0.91. The GC content and unfavorable peaks have been optimized to prolong the half-life of the mRNA. The Stem-Loop structures, which impact ribosomal binding and stability of mRNA, were broken. In addition, negative cis-acting sites were screened and successfully modified. Pro, T2A, CDS and Ter modules were harbored into a pLUG-Prime vector (iNtRON Biotechnology). Codon-optimized CDS modules, were prepared by PCR using primers carrying inward-facing Bsal sites and fusion sites.

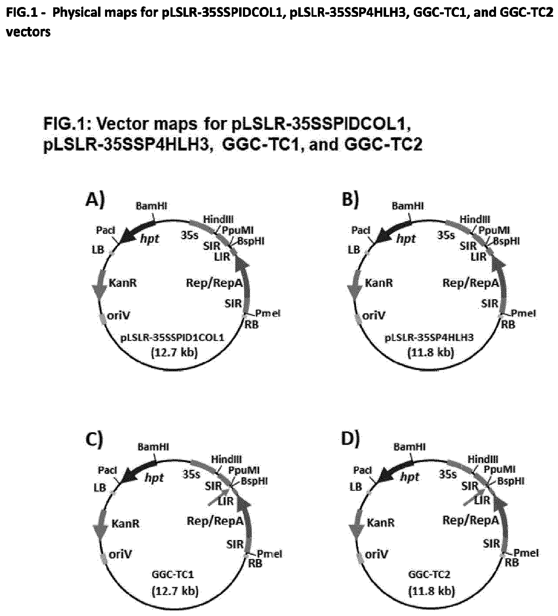

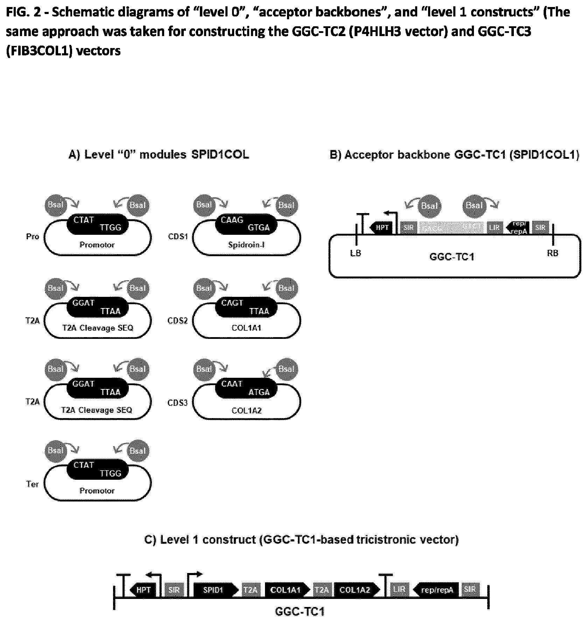

"Level 1" acceptor backbones were constructed based on a modified pLSLR vector (Baltes et al., 2014) **[27]** (Addgene plasmid #51493). Firstly, CaMV 35S promoter flanked by UBl1 intron was inserted into BamHI and Hindlll-digested pLSLR with a pCAMBIA1300 backbone, and the existing bi-directional cis-acting replication elements "LIR-SIR-LIR" (LIR = Long Intergenic Region, SIR = Short Intergenic Region) were placed in a "SIR-LIR-SIR" architecture. A benefit of this architecture and delivery mechanism is that the population of replicating viral genomes is both homogenous and predictable, consisting of the sequence between the origins within the duplicated SIRs. The resulting vectors were named pLSLR-35SSPID1COL1 and pLSLR-35SP4HLH3, respectively. A fragment flanked by two Bsal sites (5'-CTATGGAGACCGAGGTCTCGTAAG-3') for Golden Gate cloning was then inserted into pLSLR-35SSPID1COL1 and pLSLR-35SP4HLH3. Cloning into PpuMI- and BspHI-digested pLSLR-35SSPID1COL1 and pLSLR-35SP4HLH3 formed GGC-TC1 and GGC-TC2, respectively. **(See** **FIG. 1** **for physical maps of resulting vectors)**
To construct T2A-linked tricistronic (GGC-BC1 and GGC-TC1) vectors in a Pro-CDS1-T2A-CDS2-T2A-CDS3-Ter orientation, level 0 modules were directionally assembled into the level 1 acceptor backbone using a single digestion-ligation procedure. An equal molar ratio of level 0 modules and level 1 acceptor was mixed with Bsal (Bsal-HFv2, New England Biolabs) and T4 ligase (Thermo Fisher). The reaction was carried out for 10 cycles of 5 min at 37°C and 10 min at 16°C, followed by 5 min at 50°C and 5 min at 80°C. Assembled level 1 constructs were amplified in Escherichia coli DH5α, and the subsequent plasmid recovery, restriction digestion, and sequencing procedures confirmed correct vector assembly. The resulting tricistronic vectors were transformed into agropine-type Agrobacterium tumefaciens EHA 105 and octopine-type Agrobacterium tumefaciens LBA4404, respectively by electroporation to carry out agroinfiltration experiments. The transformed cells were plated on LB agar medium containing 50 mg/ml Ampicillin (Sigma Aldrich). **(See** **FIG. 2** **for schematic diagrams of "level 0", "acceptor backbones", and "level 1 constructs")**

Agroinfiltration was used for transient expression in N. Benthamiana with A. tumefaciens. strains as previously described. l00ml of transformed Agrobacterium frozen cells stock was inoculated in 5 ml LB broth (Thermo Fisher) and supplemented with 50 µg/ml rifampicin and 50 µg/ml kanamycin. Overnight, the culture was incubated at 28°C, shaking at 220 rpm. 500ml was used to inoculate 50 ml of LB medium. The cultural cells were incubated at 28°C shaking at 220 rpm until the culture had reached an O.D.600 = 0.6. The cells were harvested by centrifugation at 6000 rpm and resuspended in 50 ml MES buffer (10 mM MES; pH 5.5, lOmM MgC ). This mixture was incubated for 2.5 hours at room temperature with 120 mM acetosyringone and was added to the Agrobacterium suspension in infiltration buffer (lx MS, 10 mM MES, 2.5% glucose). For the effect of monosaccharide on induction of virulence genes, different 2% was added to the Agrobacterium suspension in the infiltration buffer (lx MS, 10 mM MES, 200 mM acetosyringone). 5-weeks old N. benthamiana plants were infiltrated in a vacuum chamber by submerging N. Benthamiana plant aerial tissues in Agrobacterium suspension and applying a 50-400 mbar vacuum for 45 seconds. Once the vacuum was broken, infiltrated N. Benthamiana plants were removed from the vacuum chamber, thoroughly rinsed in water, and grown for 5-7 days under the same growth conditions used for pre-infiltration growth. To avoid any variability, the leaves and location on the leaf, comparably-sized leaves for each plant of similar age were agroinfiltrated for each experiment.

### EXAMPLE TWO: Fibroin-III/Collagen Type-I fusion protein

As aforementioned, two separate vectors are constructed. One of which carries the Fibroin-III/Collagen Type-I construct, the other one carrying the P4H/LH3 construct. Both vectors are designed to target a subcellular compartment known to be devoid of endogenous P4H or LH activity.

The inventor constructed a fundamental set of Golden Gate cloning-compatible modular vectors which comprise the expression cassette and acceptor backbones named GGC-TC3 and GGC-TC2. The acceptor backbone is a binary T-DNA vector suitable for transient expression in plants and is designed to possess the geminiviral replicon system, capable of producing circular DNA replicons for high-level multiple protein expression.

### 1) Fibroin-III/Collagen Type I Tricistronic construct:

For the biosynthesis of the genes of interest; Fibroin-III (Uniprot: Q16987, entry version N° 44, 2017-08-30), Collagen Type-I Alpha-I without the signal peptide (1-22) and without the N-terminal propeptide (23-161) and C-terminal propeptide (1219-1464) (Uniprot: P02452, entry version N° 22, 2017-05-10), Collagen Type-I Alpha-II without the signal peptide (1-22) and without the N-terminal propeptide (23-79) and C-terminal propeptide (1120-1366) (Uniprot: P08123, entry version N° 202, 2017-05-10), an A2 sequence GSGEGRGSLLTCEDVEENPGP placed between the Fibroin-Ill, Collagen Type-I Alpha-I, and Collagen Type-I Alpha-II chain, a chloroplast transit peptide (included in Uniprot entry G5DBJ0, amino acids 1-40, entry version N° 10, 2017-11-22) placed at the N-terminus, and a 6x HIS Tag were used as a template.

### 2) P4H alpha-beta chimeric/LH3 Tricistronic vector construct:

For the biosynthesis of the genes of interest; a chimeric P4H enzyme comprising: an alpha subunit (Uniprot: Q86KR9, entry version N° 81, 2017-06-07) and a beta subunit (Uniprot: P05307, entry version N° 141, 2017-05-10) sequence without its native signal peptide (1-20), an A2 sequence GSG EGRGSLLTCEDVEENPGP placed between the alpha subunit and beta subunit, a LH3 sequence without its native signal peptide (1-24) (Uniprot: O60568, entry version N° 165, 2017-09-27), an A2 sequence GSGEGRGSLLTCEDVEENPGP places between LH3 and the PH4 beta subunit sequence, and a chloroplast transit peptide (included in Uniprot entry G5DBJ0, amino acids 1-40, entry version N° 10, 2017-11-22) placed at the N-terminus, were used as a template.

Using the Golden Gate cloning approach, a T2A-linked tricistronic vector, whereby three transgenes encoding I) Fibroin-III and II) Collagen Type-I proteins (termed FIB3COL1) were combinatorially placed along the expression cassette in the binary vector. The P4H alpha-beta chimeric/LH3 constructs also comprises 3 genes (e.g., P4H alpha subunit, P4H beta subunit, and LH3, termed P4HLH3) and were combinatorially placed along the expression cassette.

Using the assembly protocol described in the Golden Gate modular cloning system (Weber et al., 2011) [23], so-called "level-0" modular vectors containing parts of the expression cassette, such as promoter (Pro), T2A signals (T2A), coding sequences (CDS), and terminator (Ter), were constructed.

All level-0 modules were flanked by inward-facing Bsal restriction enzyme sites and fusion sites (5 bp-overhangs) to allow directional linear assembly in a Pro-CDS1-T2A-CDS2-T2A-CDS3-Ter (FIB3COL1) and Pro-CDS1-T2A-CDS2-T2A-CDS3-Ter (P4HLH3) orientation, resulting in T2A-linked tricistronic constructs. In order to construct Pro, T2A, and Ter modules, sequences of CmYLCV promoter, and AtHSP 3' UTR were retrieved from publications of the prior art being: Stavolone et al. (2003) **[24],** Nagaya et al. (2010) **[25],** and Liu et al. (2017) **[26],** respectively.

As the aforementioned genes described above, employ tandem rare codons and could reduce the efficiency of translation or even disengage the translational machinery, the codon usage bias in N. benthamiana was used by upgrading the codon adoption index (CAI) from 0.70 to 0.91. The GC content and unfavorable peaks have been optimized to prolong the half-life of the mRNA. The Stem-Loop structures, which impact ribosomal binding and stability of mRNA, were broken. In addition, negative cis-acting sites were screened and successfully modified.

Pro, T2A, CDS and Ter modules were harbored into a pLUG-Prime vector (iNtRON Biotechnology). Codon-optimized CDS modules, were prepared by PCR using primers carrying inward-facing Bsal sites and fusion sites.

"Level 1" acceptor backbones were constructed based on a modified pLSLR vector (Baltes et al., 2014) [27] (Addgene plasmid #51493). Firstly, CaMV 35S promoter flanked by UBI1 intron was inserted into BamHI and Hindlll-digested pLSLR with a pCAMBIA1300 backbone, and the existing bi-directional cis-acting replication elements "LIR-SIR-LIR" (LIR = Long Intergenic Region, SIR = Short Intergenic Region) were placed in a "SIR-LIR-SIR" architecture. A benefit of this architecture and delivery mechanism is that the population of replicating viral genomes is both homogenous and predictable, consisting of the sequence between the origins within the duplicated SIRs. The resulting vectors were named pLSLR-35SSPID1COL1 and pLSLR-35SP4HLH3, respectively. A fragment flanked by two Bsal sites (5'-CTATGGAGACCGAGGTCTCGTAAG-3') for Golden Gate cloning was then inserted into pLSLR-35SFIB3COL1 and pLSLR-35SP4HLH3. Cloning into PpuMI- and BspHI-digested pLSLR-35SFIB3COL1 and pLSLR-35SP4HLH3 formed GGC-TC3 and GGC-TC2, respectively.

To construct T2A-linked tricistronic (GGC-TC3 and GGC-TC2) vectors in a Pro-CDS1-T2A-CDS2-T2A-CDS3-Ter orientation, level 0 modules were directionally assembled into the level 1 acceptor backbone using a single digestion-ligation procedure. An equal molar ratio of level 0 modules and level 1 acceptor was mixed with Bsal (Bsal-HFv2, New England Biolabs) and T4 ligase (Thermo Fisher). The reaction was carried out for 10 cycles of 5 min at 37°C and 10 min at 16°C, followed by 5 min at 50°C and 5 min at 80°C. Assembled level 1 constructs were amplified in Escherichia coli DH5α, and the subsequent plasmid recovery, restriction digestion, and sequencing procedures confirmed correct vector assembly. The resulting tricistronic vectors were transformed into agropine-type Agrobacterium tumefaciens EHA 105 and octopine-type Agrobacterium tumefaciens LBA4404, respectively by electroporation to carry out agroinfiltration experiments. The transformed cells were plated on LB agar medium containing 50 mg/ml Ampicillin (Sigma Aldrich).

Agroinfiltration was used for transient expression in N. Benthamiana with A. tumefaciens. strains as previously described. l00ml of transformed Agrobacterium frozen cells stock was inoculated in 5 ml LB broth (Thermo Fisher Scientific) and supplemented with 50 µg/ml rifampicin and 50 µg/ml kanamycin. Overnight, the culture was incubated at 28°C, shaking at 220 rpm. 500ml was used to inoculate 50 ml of LB medium. The cultural cells were incubated at 28°C shaking at 220 rpm until the culture had reached an O.D.600 = 0.6. The cells were harvested by centrifugation at 6000 rpm and resuspended in 50 ml MES buffer (10 mM MES; pH 5.5, IOmM MgC ). This mixture was incubated for 2.5 hours at room temperature with 120 mM acetosyringone and was added to the Agrobacterium suspension in infiltration buffer (lx MS, 10 mM MES, 2.5% glucose). For the effect of monosaccharide on induction of virulence genes, different 2% was added to the Agrobacterium suspension in the infiltration buffer (lx MS, 10 mM MES, 200 mM acetosyringone). 5-weeks old N. benthamiana plants were infiltrated in a vacuum chamber by submerging N. Benthamiana plant aerial tissues in Agrobacterium suspension and applying a 50-400 mbar vacuum for 45 seconds.

Once the vacuum was broken, infiltrated N. Benthamiana plants were removed from the vacuum chamber, thoroughly rinsed in water, and grown for 5-7 days under the same growth conditions used for pre-infiltration growth. To avoid any variability, the leaves and location on the leaf, comparably-sized leaves for each plant of similar age were agroinfiltrated for each experiment.

### RESULTS

### Extraction and purification of SPID1COL1 and FIB3COL1 heterotrimeric fusion proteins

For the extraction of both SPID1COL1 and FIB3COL1 proteins, infiltrated N. benthamiana leaves (300 g for each protein) were harvested and grinded, and blended (in 3 intervals of 1 minute each) with 2.5 g of activated carbon and cold (4°C) extraction buffer (100 mM Tris-HCl pH 8.0, 4 mM EDTA, 600 mM NaCl, 25 mM DL-Dithiothreitol (DTT), 0.5% NP40, 2% Poly-(Vinyl-Poly-Pyrolidone) (PVPP), 10% glycerol and 2 x Roche EDTA-free Complete protease inhibitor cocktail (Roche Diagnostics, Germany) at a ratio of 2 ml per gram of leaves (fresh weight). During this protocol, the temperatures were kept below 12°C. The resulting crude extracts were then filtered using Whatman No. 1 filter paper, followed by centrifugation of the filtered extract (15000 g for 30 min at 5°C). The resulting supernatants were then collected, and together with 1g/L activated carbon, CaCl 2 was added at a final concentration of 10 mM. Nonsoluble contaminants were then further removed by centrifugation (20000 g for 30 min at 15 °C).

Both SPID1COL1 and FIB3COL1 in the recovered supernatants were precipitated by gradually adding crystalline NaCl to a final concentration of 2.85 M (20 min, at room temperature with constant stirring). The solutions were incubated in a cold room for 6h without stirring. Collection of the SPID1COL1-containing and FIB3COL1-containing pellets were performed following centrifugation (22000 g for 2 h at 5 °C). The pellets were then resuspended in a 200 mL solution of 250 mM acetic acid + 2 M NaCl for 5 min, using a magnetic stirrer, and then centrifuged (22000 g for 30 min at 5 °C). Supernatants were then discarded, and the pellets were resuspended in 200 mL of 0.5 M acetic acid (for 1 h at room temperature). Elimination of insoluble matter was performed by centrifugation (15000 g for 30 min at 15 °C). The resulting supernatants were passed through 3 layers Whatman No. 1 filter paper.

The resulting SPID1COL1 and FIB3COL1 proteins were then precipitated by slowly adding NaCl to a final concentration of 3 M along with constant stirring for 25 min at room temperature. The solution was incubated in a cold room for 8 h at 4 °C and the SPID1COL1 and FIB3COL1 proteins were collected following centrifugation (22000 g for 2.5 h at 5 °C). All remaining supernatant traces were removed. Pellet redissolving and SPID1COL1 and FIB3COL1 precipitation steps were repeated as above in acetic acid and NaCl solutions, respectively. Following the incubation and collection of SPID1COL1-containing and FIB3COL1-containing pellets, the samples were redissolved in 50 mL of 10 mM HCl by vigorously pipettation and vortexing for 5 min at room temperature. The solutions were transferred to dialysis bags (Thermo Fisher, MWCO 25000 Da) and dialyzed against 5 L of 10 mM HCl (for 3 h at 4 °C). An additional dialysis was performed. Both SPID1COL1 and FIB3COL1 proteins were sterilized by filtering through a 0.2 µ filter using 30 mL syringes. The SPID1COL1 and FIB3COL1 proteins were then concentrated using Vivaspin PES 6 mL filtration tubes (Sartorius, MWCO 300000) before loading into Nickel-nitrilotriacetic (Ni-NTA) affinity resin (Amintra). Briefly, the column was washed with 10 column volumes of wash buffer (5 mM and 20 mM Imidazole, 20 mM Tris-HCl, 50 mM NaCl, pH 7.4, respectively) and eluted the recombinant protein with elution buffer (250 mM Imidazole, 20 mM Tris-HCl, 50 mM NaCl, pH 7.4). The purified SPID1COL1 and FIB3COL1 protein samples were analyzed by SDS-PAGE, Southern blot analysis, Western blot analysis, and quantified by ELISA. The total soluble protein (TSP) in the plant crude extracts was estimated by using Bradford assay (Bio-Rad) by following manufacturer's instruction.

### Southern blot analysis

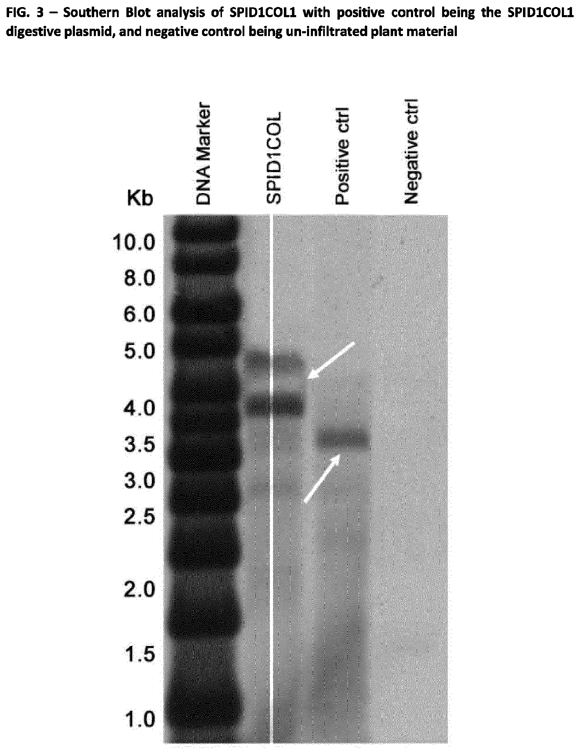

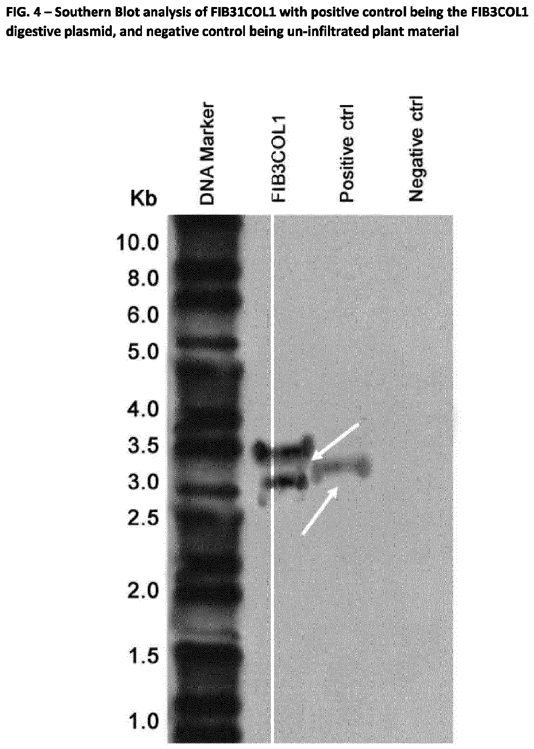

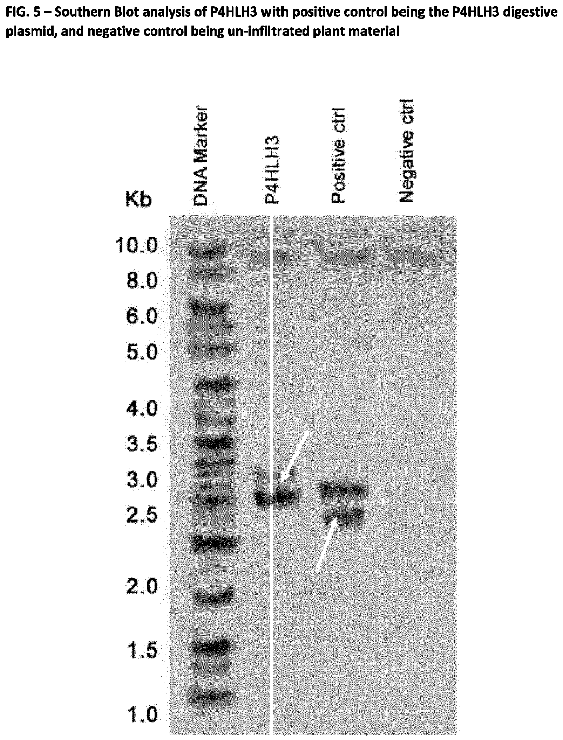

Genomic DNA from the agroinfiltrated leaves expressing SPID1COL1, FIB3COL1 and P4HLH3, respectively, were extracted by DNeasy Plant DNA mini kit (Qiagen) and digested with both EcoRl/Bglll and subjected to Southern blot analysis. Results showed that both the synthetic SPID1COL1, FIB3COL1, and the P4HLH3 open reading frames (ORF) were successfully transformed into N. benthamiana leaves after agroinfiltration. Labeling and detection were carried out using Biotin Deca Label DNA Labeling Kit, ThermoScientific and Biotin chromogenic Detection kit, ThermoScientific, respectively. The presence of amplified fragments with the expected sizes indicates that the genes were successfully transformed into N. benthamiana leaves via agroinfiltration. Higher molecular weight fragments were visualized due to partial digestion of some DNA of the samples. The digested recombinant GGC-TC1, GGC-TC2, and GGC-TC3 vectors were used as positive control and resulted in the same size band while the un-infiltrated leaves were used as negative control. **(See** **FIG. 3****,** **4** **and** **5** **for Southern Blot results using SPID1COL1 probe, FIB3COL1 probe and P4HLH3 probe for the total DNA of infiltrated tobacco leaves after digestion with EcoRI and BgIII)**

### Detection of chimeric genes by RT-PCR

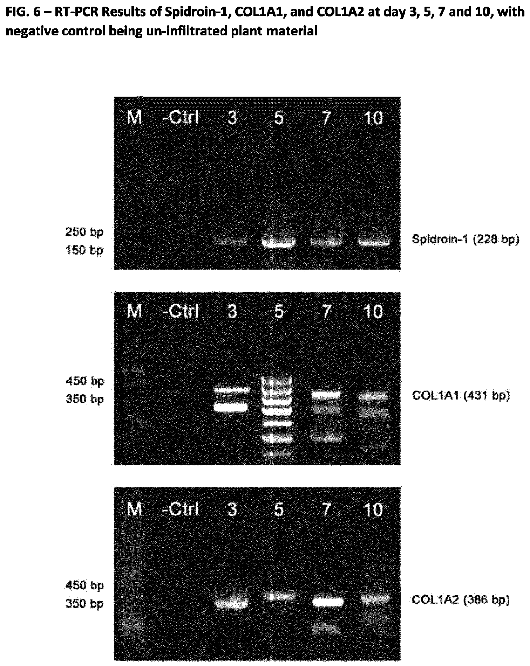

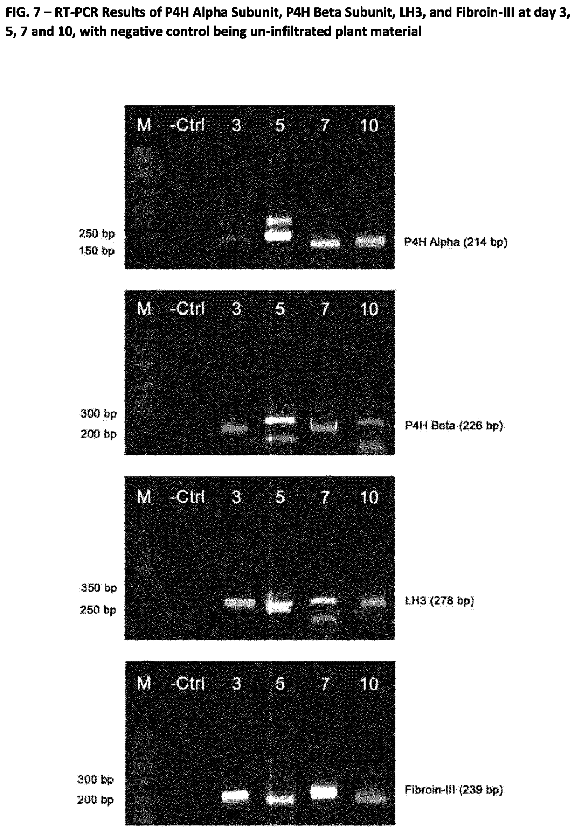

Transcription for both the respective Spidroin-I, Collagen Type I Alpha I, Collagen Type I Alpha II, Fibroin-3, P4H Alpha Subunit, P4H Beta Subunit, and LH3 genes was confirmed using Reverse-Transcription Polymerase Chain Reaction (RT-PCR). The extracted RNA samples from infiltrated N. benthamiana leaves were subjected to RT-PCR analysis using specific primers for each gene to amplify the core region of each gene. Total RNA was extracted using Illustra RNAspin mini kit (GE healthcare). Oligonucleotide pairs at the core region were designed to detect the presence of the respective genes at the core region; for Spidroin-I; TE-F: 5'- GGAGGACAAGGAGCTGGAG-3', and TE-R: 5'-CTAGAAGCAGCAGCAGAAGC-3', for Collagen Type-I Alpha-I; TE-F: 5'-ACCTATGGGACCTCCTGGAT-3', and TE-R: 5'-GCAGGTCCAGTTTCTCCTCT-3', for Collagen Type-I Alpha-II; TE-F: 5'-AGAACCTGGATCTGCTGGAC-3', and TE-R: 5'-CCAGGAGGTCCCATTACTCC-3', for P4H alpha subunit; TE-F: 5'-GCTGGAATGAATAAAGGAACTGA-3', and TE-R: 5'-ATCTTCCTCCATTTAAATATACAGCTA-3', for P4H beta subunit; TE-F: 5'-TCCTGCTTCTGCTGATAGAACT-3', and TE-R: 5'-TCAGGTTCTTCAGCTTCTTCT-3', for LH3; TE-F: 5'-TGTAGTACATGGAAATGGACCT-3', and TE-R: 5'-GGAGGAGGTTGTCCTCCAG-3', for Fibroin-III; TE-F: 5'-CTGCTGCTGGAGGATATGGA-3', and TE-R: 5'-TCCTCCAGGTCCTTGTTGTC-3'. One step RT-PCR was carried out according to manufacturer instructions using SuperScript^{®}III with Platinum^{®} Taq DNA Polymerase. The reactions resulted in the expected bp-fragments of the core region of the genes being; Spidroin-I (228 bp-fragment), Collagen Alpha-I (431 bp-fragment), Collagen Alpha-II (386 bp-fragment) P4H alpha subunit (214 bp-fragment), P4H beta subunit (226 bp-fragment), LH3 (278 bp-fragment), and Fibroin-III (239 bp-fragment). The RT-PCR amplified fragments of indicated that all the infiltrated leaves at day 3, 5, 7, and 10 clearly exhibited the transcription of the respective genes as shown in **FIG. 6** **and** **7****,**while un-infiltrated leaves showed negative results.

### Western blot analysis

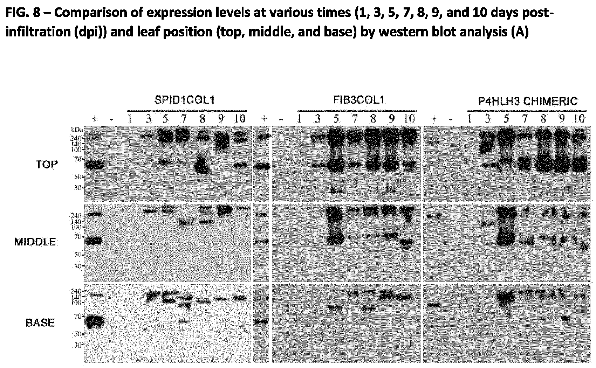

Western blot was performed to confirm the production of both the chimeric SPID1COL1 and FIB3COL1 proteins within plant's tissue. Total soluble proteins were extracted from infiltrated plants by grinding 500 mg of leaves in 0.5 mL 50mM Tris-HCl (pH 7.5) enriched with 1x Roche EDTA-free Complete protease inhibitor cocktail (Roche Diagnostics, Germany). The crude extract was boiled for 5 minutes in 300 µL of 4X SDS Sample Loading Buffer (Sigma Aldrich: Tris-HCl: 0.2 M, DTT: 0.4 M, SDS: 277 mM, 8.0% (w/v), Bromophenol blue: 6 mM, Glycerol: 4.3 M) and centrifuged (12000 rpm for 7 min, at room temperature). Supernatant samples (25 µL) were separated on a 10% polyacrylamide gel (NuPAGE BIS-TRIS gel, Thermo Fisher) and proteins of interest were immunodetected using standard Western blot procedures. Detection of Spidroin-1, Collagen Type-I Alpha-I chain, Collagen Type-I Alpha-II chain, P4H alpha subunit, P4H beta subunit, LH3, and Fibroin-III was effected using a custom designed anti-Dictyostelium discoideum (Slime mold) P4H alpha subunit antibody, a custom designed anti-Bovine P4H beta subunit antibody, an anti-rabbit-LH antibody (LSBio), anti-rabbit polyclonal antibody to MASP (MASP1) (LSBio), anti-collagen type I antibody (OriGene Technologies) antibody, and a custom designed anti-rabbit polyclonal antibody to Fibroin-III. Broad range prestained protein marker were purchased from Thermo Fisher (PageRuler Prestained Protein Ladder, 30 to 240 kDa). As anticipated it showed no reactivity with un-infiltrated plants which were used as negative control. **(See** **FIG. 8** **for western blot results comparative over days 1, 3, 5, 7, 9, and 10 post-infiltration and leaf position top, middle, and base)**

### Thermal Stability

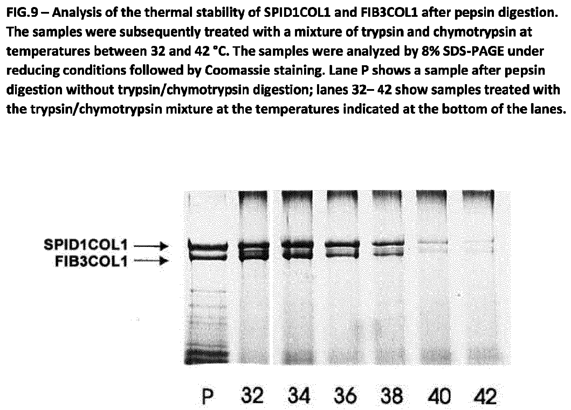

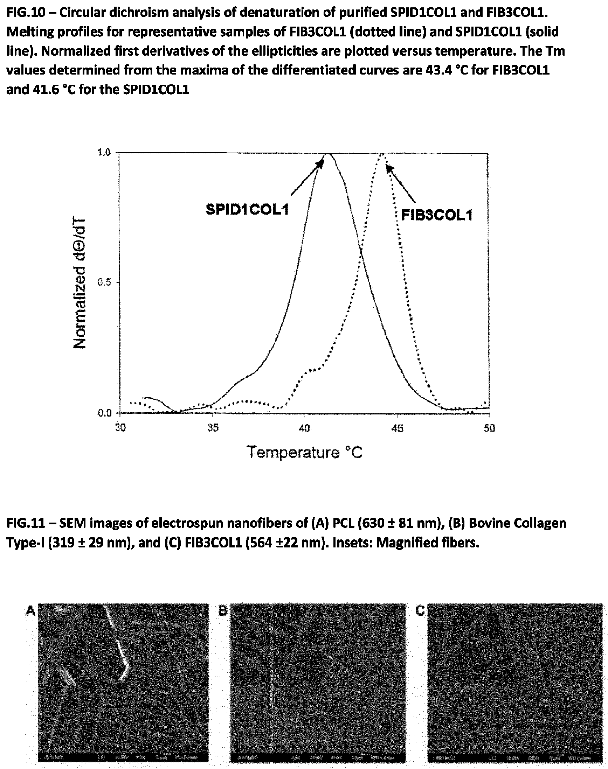

To assess the thermal stability of the SPID1COL1 and FIB3COL1 proteins, their sensitivity to either pepsin or a trypsin/chymotrypsin mixture was determined according to the method of P. Bruckner (1981) **[28].** Using a temperature range between 32°C and 42°C the study showed that both purified SPID1COL1 and FIB3COL1 were resistant to pepsin up to 39.4 and 39.8°C, respectively (50% degradation point as measured by scanning of SPID1COL1 and FIB3COL1 bands after PAGE) **(****FIG. 9****).** To obtain more accurate data on the thermal stability of the resulting SPID1COL1 and FIB3COL1 proteins, circular dichroism (CD) spectra were performed. CD measurements of 45µg/mL SPID1COL1 or 45µg/mL FIB3COL1, prepared in 10 mM HCl were performed using a Jasco J-810 Circular Dichroism Spectropolarimeter (Jasco) in a UV Fused Quartz Cuvette with 10 mm Path Length (CV10Q7A, Thorlabs). The cuvette was filled with 1 mL of sample for each measurement. CD spectra were obtained at room temperature by continuous wavelength scans ranging from 200 to 270nm at a scanning speed of 50 nm per minute. Averages of three scans per sample were calculated. The spectra were typical for a triple helical conformation which is in line with earlier work established by F. Ruggiero (2000) **[29]** (data not shown). The thermal transition curve for both SPID1COL1 and FIB3COL1 proteins measured by circular dichroism at 225 nm indicated a Tₘ value of 41.6°C and 43.4°C, respectively at which 50% of the SPID1COL1 and FIB3COL1 molecules remain in a fully folded conformation as compared to 40°C for bovine heterotrimeric Type I Collagen shown in prior art **(****FIG. 10****).** The gradual decrease in the quantity of the detected SPID1COL1 and FIB3COL1 is due to the fact that the extent of hydroxylation can vary from one SPID1COL1 or FIB3COL1 molecule to the other, resulting in a population of triple helices with different melting point temperatures. These results show that co-expression of the chimeric P4H and LH3 enzymes with the both the SPID1COL1 an FIB3COL1 proteins proved to be essential for conformation and stability.

### Structural analysis

To visualize the fibril lattice network of SPID1COL1 and FIB3COL1, the resulting fusion proteins were allowed to assemble to fibrils, collected, and analyzed by scanning electron microscopy (SEM) **(See** **FIG. 11****).** For the preparation of samples for SEM, fibril formation of the SPID1COL1 and FIB3COL1 proteins was induced by mixing with 5µL of fibrillogenesis buffer (60 mM NaH2PO4, 1.4% NaCl (w/v), pH 9.5) and incubating for 1 h at 37°C. The SPID1COL1 and FIB3COL1 samples were then immersed in 0.1 M phosphate buffer (pH7.3) and 2.5% glutaraldehyde (4 °C), followed by rinsing the samples three times in phosphate buffer and gradually dehydrating them by adding increasing concentrations of ethanol (25-100%). The samples were again rinsed for another 25 min, and finally dried in a Critical Point Dryer (Leica EM CPD300). The resulting samples were gold coated (coating thickness 25 nm) using an EM ACE600 Sputter coater (Leica) and SEM images were obtained using a Camscan MX 2600 FEGSEM using an accelerating voltage of 10 kV and magnifications of 500×. Long homogeneous fibrils and lattice structures characteristic to both Spidroin-I, Fibroin-Ill and Collagen Type-I were observed, indicating proper structures of the SPID1COL1 and FIB3COL1 proteins.

### Biofunctionality

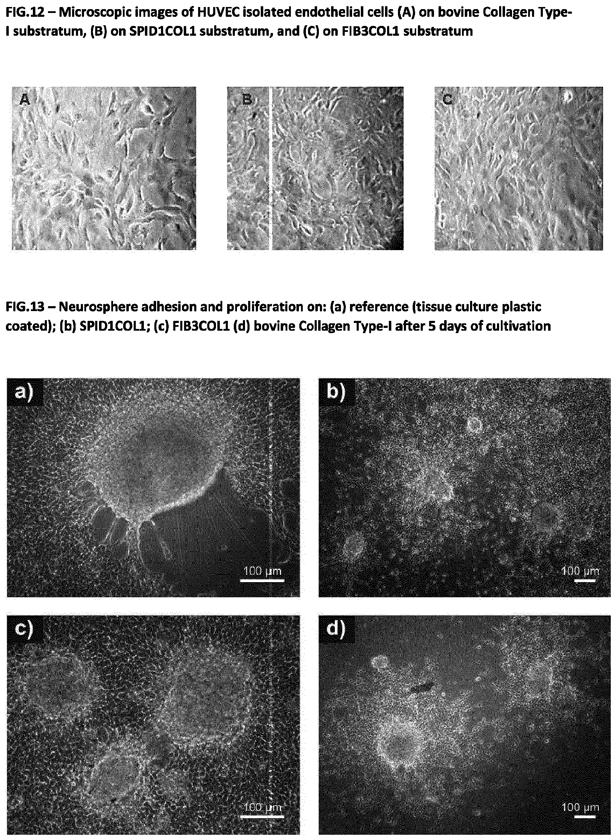

In culture collagenous extracellular maxtrix proteins can bind to biological substrata and simultaneously to cell surfaces, thereby promoting attachment, spreading and growth of these cells (Klebe, 1974; Pearlstein, 1976). To determine the biofunctionality of the resulting SPID1COL1 and FIB3COL1 proteins, isolated endothelial cells derived from adult human umbilical veins (HUVEC) were seeded on antimicrobial plastic matrices precoated with either SPID1COL1, FIB3COL1, or native human skin type I Collagen (GenoSkin). Human endothelial cells were isolated from normal, term umbilical veins as described by Gimbrone et al. (1974) **[30].** Endothelial progenitor cell (EPC) yields obtained from SPID1COL1 and FIB3COL1 were 2- and 2.5-fold higher than those obtained with native human tissue-derived collagen type I and were several fold higher than those obtained from uncoated matrices. Furthermore, the SPID1COL1 and FIB3COL1 proteins were more effective in the isolation of cells from HUVEC isolated endothelial cell samples containing either very low or high endogenous levels of EPC. The majority of cells isolated and grown on both SPID1COL1 and FIB3COL1 proteins appeared as typical spheroid-shaped cells supported by strong interactions with the SPID1COL1 or FIB3COL1 protein matrix, while cells grown on either native human skin type I Collagen or uncoated matrices were mostly round. These results display that the biological activity of both SPID1COL1 and FIB3COL1 are proven to be superior over naïve human tissue-derived collagen through its capacity to support attachment and proliferation of isolated endothelial cells derived from adult human umbilical veins. **(See** **FIG. 12****)**

### Amino acid composition analysis

To further verify the identity of the expressed SPID1COL1 and FIB3COL1 proteins at amino acid composition level, samples were digested with a sulfhydryl-specific protease (ficin) and further purified which mimicked the migration of pure human skin type I collagen samples. Following electrophoretic separation of the purified SPID1COL1 protein to Spidroin-I and Collagen Type-I, and FIB3COL1 protein to Fibroin-III and Collagen Type-I, respectively, protein sequence analysis was performed on the respective bands using an LCMS-8050 triple quadrupole LC-MS/MS (Shimadzu), which were thought to correspond to both Spidroin-I and Fibroin-III and Collagen Type-I (data analyzed by Traverse MS data analysis software). The bands indicated in were identified as alpha I type I collagen (Homo sapiens; *p* = 1.08 × 10⁻³⁰ ), alpha II Type I Collagen (Homo sapiens; *p* = 1.24 x 10⁻¹⁴ and Spidroin-I (Nephila clavipes; *p* = 1.48 × 10⁻¹² ). All identified peptides (80% sequence coverage) displayed 100% identity to human collagen, Spidroin-I, and Fibroin-III protein sequences, respectively. Amino acid analysis of the resulting SPID1COL1 and FIB3COL1 proteins showed significant identity to the human-extracted Collagen Type-I heterotrimer level **[31, 32, 33]** and Spidroin-I and Fibroin-III level. Additionally, the hydroxylysine content was 36-fold and 39-fold higher for SPID1COL1 and FIB3COL1, respectively, than the levels detected in LH3-free N. benthamiana plants **[14]** thereby establishing heterologous activity of the chimeric P4H and LH3 proteins. Measured percentages of hydroxyproline content (8.24% for SPID1COL1 and 8.32% for FIB3COL1) were quite similar to those reported for recombinant transgenic plant-derived collagen (8.41%) performed by Merle et al. (2002) **[14]** and (7.55%) performed by Hanan Stein et al. (2009) **[34]** and hydroxylysine content (0.86%) to those of human collagen (1%), which is also in line with the 0.74% performance in the study by Hanan Stein et al. (2009) **[34].** (see Table 1 for amino acid analysis of SPID1COL1 and FIB3COL1 vs. Human-derived Collagen heterotrimers).

| **Amino Acid** | **SPID1COL (%)** | **FIB3COL1 (%)** | **Human Col^{l}agen Type-I (%)** |
|---|---|---|---|
| **Asp** + **Asn** | 4.11 | 4.19 | 4.3 |
| **Hydroxyproline** | 8.24 | 8.32 | 10.3 |
| **Threonine** | 1.43 | 1.47 | 1.7 |
| **Serine** | 3.81 | 3.63 | 3.3 |
| **Glu** + **Gln** | 7.47 | 7.51 | 7.1 |
| **Proline** | 15.71 | 15.94 | 12.0 |
| **Glycine** | 35.64 | 34.42 | 33.5 |
| **Alanine** | 14.82 | 14.21 | 11.1 |
| **Valine** | 2.71 | 2.57 | 2.6 |
| **Isoleucine** | 1.24 | 1.08 | 0.9 |
| **Leucine** | 3.37 | 2.87 | 2.3 |
| **Tyrosine** | 0.78 | 0.69 | 0.2 |
| **Phenylalanine** | 0.96 | 1.14 | 1.2 |
| **Hydroxylysine** | 0.89 | 0.86 | 1.0 |
| **Lysine** | 2.44 | 2.56 | 2.3 |
| **Histidine** | 0.51 | 0.49 | 0.6 |
| **Arginine** | 5.61 | 5.42 | 5.0 |
| **Cysteine** | ND | ND | ND |
| **Methionine** | 0.41 | 0.38 | 0.6 |
| **Tryptophan** | ND | ND | ND |

### EXAMPLE THREE: SPID1COL1 and FIB3COL1 electrospun scaffolds

One of the main objectives in tissue engineering is the fabrication of cyto-compatible scaffolds and the selection of (bio)materials that can perform cell interactions to ensure the physiological activity of the construction. There is a spectrum of requirements for these materials, such as non-toxicity, low immunogenicity, a well-defined biodegradation rate, and the like. The structure of the scaffold should imitate the native extracellular matrix structure as closely as possible and perform its functions to recreate the native conditions for cells. The inventor of the present invention investigated three different scaffold constructions fabricated either with SPID1COL1, FIB3COL1, or native Human Type I Collagen proteins. Both spider-based Spidroins and fibroins are characterized by their unique combination of physico-chemical and biological properties, and can be used in different fields of tissue engineering, both in a solo-state and in composites (e.g., SPID1COL1 and FIB3COL1). The main advantage of spidroin or fibroin proteins when compared with other cyto-compatible materials such as collagen, are their mechanical properties **[35],** which ensure the Spidroin or fibroin application as a frame-reinforcing component in various constructions **[36, 37]** and as a composite additive to polymers with insufficient mechanical strength **[38-40]** or weak mechanical properties under wet conditions such as Collagens **[41].**

Both Spidroin-I, derived from Nephila clavipes and Fibroin-III (an analogue of Spidroin-2 **[42]),** derived from Araneus diadematus are characterized by the presence of a huge number of repetitive sequences in the central part (the so-called primary repeats of 25-40 amino acid residues in size) and unique sequences of 100-300 amino acid residues at the N- and C-domains. All repeats contain poly-Ala (Alaline) blocks in 4-8 amino acid residues, which alternate with Gly (Glycine) repeat regions with the GGX motif for Spidroin-I and the GPGXX motif for Fibroin-III (as well as Spidroin-II) **[43].** Such an alteration of the hydrophobic and hydrophilic regions of molecules ensures amphiphile properties for interaction with tissues. The presence of up to 15% of proline residues in the amino acid sequence of Fibroin-III, which are absent in spidroin-I **[44],** has a significant effect on the further formation of higher-level structures and determines the various properties of these proteins. Furthermore, both Spidroin-I and Fibroin-III are characterized by the ability to phase transition during dehydration. This property makes it possible to ensure the structural stability of the protein in constructions that are based on them.

The electrospinning method is one of the most promising methods for fabricating scaffolds with a defined structure. Electrospun scaffolds have a multilayer fibrous structure with a high porosity and a high surface area-to-volume ratio (SA:V). Many different types of constructions based on silk proteins have been fabricated using the electrospinning method **[45, 46].** It is well known in the art that electrospun collagen nanofibers are mechanically weak in nature and readily soluble in water **[47, 48, 49].** Rapid degradation is not ideal for tissue engineering application as the scaffold will disappear before the cells layout their own ECM. Thus, collagen fibers have to be cross-linked to reduce the water solubility, to improve the resistant to enzymatic degradation and to enhance the mechanical strength.

The present invention makes it possible to create cyto-compatible scaffolds using either SPID1COL1 or FIB3COL1 proteins that both combine mechanical properties and high cytocompatibility with modification potential versus conventional animal-derived Collagen Type I. These properties allow the requirements of tissue engineering to be satisfied. Furthermore, both SPID1COL1 and FIB3COL1 are characterized by high strength and an elasticity modulus compared to conventional animal-derived Collagen Type I, which are necessary to accelerate regenerative potential and to reduce surgical trauma. Thus, in the course of this study, a comparative analysis of the structure, biological properties and regenerative potential of SPID1COL1 and FIB3COL1 electrospun scaffolds vs. commercial animal-derived Collagen Type I was performed and novel data on their structure and biological properties was obtained, highlighting the obvious performance superiority of SPID1COL1 and FIB3COL1.

### Fabrication of SPID1COL1- and FIB3COL1-based scaffolds

Aqueous solutions (30% concentrations) of SPID1COL1, FIB3COL1, and Bovine Collagen Type I proteins were dried in Petri dishes in a Critical Point Dryer (Leica EM CPD300). The dried proteins were dissolved in a phosphate buffered saline (PBS)/1,1,1,3,3,3 hexafluoro-2-propanol (HFIP) /acetic acid ternary mixture as solvent at a ratio of 1:1:1 and a rate of 50 mg/mL. HFIP, a volatile solvent (boiling point of 61 °C), evaporates under normal atmospheric conditions generating polymer fibers in a dry state **[50].** This approach has been used successfully to develop various scaffolds that were assessed in both in vitro and in vivo studies **[51-55].** The resulting solutions were centrifuged for 12 min at 11,500× g and then each protein was mixed separately in a volume ratio of 7:3, respectively, to a total protein concentration of 50 mg/mL. Microfibrous scaffolds were fabricated using the electrospinning method using an E-Fiber EF100 electrospinning device (SKE Research Equipment). The solutions that were loaded into CadenceScience Tuberculin glass syringens (Fisher Scientific) were deposited to the fixed collector surface (steel plate) under an electric field with a voltage of 6.7-7 kV through a standard 18 G blunt tip needle. The solution feed rate was 0.125 mL/h, and the needle-collector distance was 10 cm. The scaffolds were dried in a Critical Point Dryer (Leica EM CPD300) and were then separated. To create scaffolds for cell adhesion and proliferation research, the solutions were deposited with similar parameters on cover glasses that were attached to the collector.

### Morphology and characterization of electrospun nanofibers

The structure of the SPID1COL1, FIB3COL1, and Bovine Collagen Type I (Thermo Fisher) scaffolds were analyzed using Scanning Electron Microscopy (SEM). The SEM method enabled to confirm the porous fibrous structure of the resulting scaffolds, as well as to estimate the average thickness of their fiber composition. In brief, Nanofibers were fixed in a mixture of 1.5% glutaraldehyde / 3% paraformaldehyde in 100 mM sodium cacodylate buffer (pH 7.4) with 2.5% sucrose for 40 minutes at room temperature, followed by a 1% osmium tetroxide in 100 mM sodium cacodylate buffer (pH 7.4) fixation for 20 minutes at room temperature. The respective samples were dehydrated with a graded ethanol series (50/75/85/95/100 % in water) followed by critical drying using a a Critical Point Dryer (Leica EM CPD300). Subsequently, the resulting samples were gold coated (coating thickness 10 nm) using an EM ACE600 Sputter coater (Leica) and SEM images were obtained using a Camscan MX 2600 FEGSEM. The average diameter of the electrospun fibers was analyzed from at least five different sections of the SEM images using Image J software, which were 630 ± 81 nm for SPID1COL1, 564 ± 22 nm for FIB3COL1, and 319 ± 29 nm for Bovine Collagen Type I in mean diameter, respectively **(See** **FIG. 11****).** It was confirmed that both fiber diameter and alignment of SPID1COL1, FIB3COL1 and Bovine Collagen Type I influenced NSC adhesion, proliferation, and differentiation thereby obviating the superiority of both SPID1COL1 and FIB3COL1 compared to native Bovine Collagen Type I **(Table 2).** These tests were performed on primary Mouse Neural Stem Cells. BALB/cA mouse embryos at embryonic day 13.5-14.5 (E13.5 -E14.5) were isolated after sacrifice of gravid females and placed into icecold Hank's balanced salt solution (HBSS, Thermo Fisher) for extraction of neural stem cells. Retrieval of the spinal cords was collected from 1 to 2 litters of embryos at a time, and rinsed in HBSS. Following rinsing, the tissue was placed in NeuroCult-XF proliferation medium (StemCell Technologies) and mechanically dissociated by repeated gentle trituration through wide bore tips (Thermo Fisher). The suspension was placed in a T75 TC treated falcon cell culture flask (Fudau) containing NeuroCult-XF proliferation medium (StemCell Technologies) and associated NeuroCult^{™} Proliferation Supplement (STEMCELL Technologies), as well as penicillin (100 U)/streptomycin (125 µg/mL; Thermo Fisher). Cells were grown as free-floating clustered neurospheres at 37 °C with 92% air and 8% CO₂, passaged by mechanical dissociation every 5 days, preventing from attachment (gently knocking flasks) every other day. Proliferation kinetics of the cultures were studied by microscopic examination (e.g., collecting neurospheres every 2 days, and assessing the total number of viable cells at each passage by Trypan Blue exclusion). For the microscopic examination, dark, dense spheres were considered to be unhealthy and composed of more dead cells than lighter colored spheres, as viable neurospheres are generally semitransparent. Initially, single cells proliferated to form small clusters of cells that lightly adhered to the SPID1COL1, FIB3COL1, or Bovine Collagen Type I scaffolds; however some of these clusters lifted off as the density of the sphere increases. Cells used for transplantation or in vitro differentiation had been passaged 5 times.

**Table 2. The influence of fiber diameter and alignment on adhesion, proliferation, and differentiation. Cell counts were performed three days after seeding cells on the scaffolds.**

| **Scaffold** | **Fiber diameter** | **Adhesion (%)** | **(%) Proliferation** | **Differentiation (% Neurons)** |
|---|---|---|---|---|
| **SPID1COL1** | 630 ± 81 nm | 91 | 90 | 80 |
| **FIB3COL1** | 564 ± 22 nm | 87 | 92 | 82 |
| **Native Bovine Collagen Type I** | 319 ± 29 nm | 68 | 78 | 38 |

### In vitro differentiation and immunocytochemisty

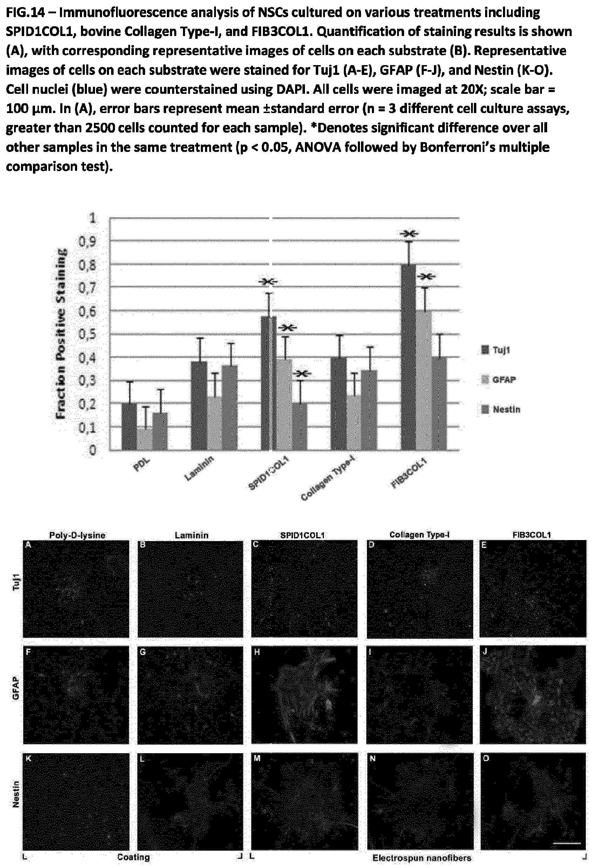

In vivo extracellular matrices, such as collagen and laminin, exhibit micro- to nano- scale fibrous topography, which explains why electrospun matrices significantly influence the adhesion, survival, proliferation, and differentiation of stem cells. In order to gain insight on how either SPID1COL1, FIB3COL1, or Bovine Collagen Type I influence neural development, the aforementioned neurospheres were were seeded on either electrospun SPID1COL1, FIB3COL1 or native Bovine Collagen Type I scaffolds to study their effect on adhesion and proliferation. Therefore, the aforementioned neurospheres were plated as small spheres onto poly-D-lysine (PDL, Sigma Aldrich), laminin coated coverslips (Thermo Fisher), or electrospun meshes three days after the last passage, in NeuroCult NS-A Differentiation medium (StemCell Technologies), as well as penicillin (100 U)/streptomycin - 125 µg/mL; Thermo Fisher). The cells were differentiated for seven days and then fixed for 15 minutes in 4% paraformaldehyde (PFA) at room temperature. Following rinses in Phosphate buffered saline (PBS, pH 7.2) and block in a blocking solution of 5% normal goat serum and 0.25% Triton X-100 in 0.02 M PBS (PBS+), the cultures underwent immunocytochemistry with reaction to primary antibodies overnight at 5°C. After 5 rinses in PBS+, the cultures were further incubated in the absence of light (dark room) with Alexa Fluor 488- and 594-conjugated secondary antibodies at a 1:100 ratio (Invitrogen) in PBS+ for 2.5 hours at room temperature. After 5 rinses in PBS, 4',6-diamidino-2-phenylindole (DAPI, Thermo Fisher) was added for 5 minutes before gently rinsing in PBS and placing the coverslip on a microscope slide. Negative controls with omission of primary antibodies were performed in parallel, and no positive signals were detected. Cells were also evaluated after 1, 5, 10, 21, and 28 days. Cell viability, estimated by trypan blue exclusion, was around 90% and 92% for the SPID1COL1, and FIB3COL1 scaffolds at Day 3, while it was only approximately 78% for the native Bovine Collagen Type I scaffold. The small clusters observed on the native Bovine Collagen Type I scaffold were dark and dense, indicating unhealthy or dead cells. By Day 5, the neurospheres on the SPID1COL1 and FIB3COL1 scaffolds were still mainly semi-transparent and cell viability was around 86% and 88%, respectively. Some spheres adhered to the scaffold, as the single cells were proliferating and forming small clusters of cells. The neurospheres on the native Bovine Collagen Type I scaffold did not readily adhere at the ratio SPID1COL1 or FIB3COL1 scaffolds; as the dark, dense spheres of unhealthy or dead cells lifted off and the density of the sphere increased. Cell viability on the native Bovine Collagen Type I scaffold was only around 63% by Day 5 **(****FIG. 13****).** As both the Spidroin-I or Fibroin-III moiety of the SPID1COL1 or FIB3COL1 proteins promoted a more significant profileration rate compared to native Bovine Collagen Type I, the inventor also investigated whether it also had an effect on differentiation. To test this, the aforementioned neurospheres were plated as small spheres onto either poly-D-lysine (PDL), laminin coated coverslips, or electrospun meshes three days after the sixth passage, in NeuroCult NS-A Differentiation medium (StemCell Technologies). After seven days, the neurospheres readily adhered, flattened, and spread to yield large numbers of migrating cells. Cells were stained for neuron specific beta-Tubulin (Tuj1) to demonstrate neurons, glial fibrillary acidic protein (GFAP) for astrocytes, O4 for oligodendrocytes, and Nestin to show intermediate filament proteins to identify neuroepithelial stem cells **(****FIG. 14****).** Neurons, astrocytes, and nestin-expressing cells were observed for all treatments, but oligodendrocytes were not detected. The electrospun SPID1COL1 and FIB3COL1 treatments displayed the highest proportion of neurons (80% for SPID1COL1 and 82% for FIB3COL1), astrocytes (61% for SPID1COL1 and 62% for FIB3COL1), and Nestin positive (40% for SPID1COL1 and 41% for FIB3COL1) cells. In contrast, treatment with native Bovine Collagen Type 1 displayed much lower proportion of neurons (38%), astrocytes (14%), and Nestin positive (35%) cells. As both the electrospun SPID1COL1 and FIB3COL1 scaffolds generated the highest proportion of neurons, astrocytes, and Nestin positive cells compared to native Bovine Collagen Type I, it has the potential to be an excellent scaffold for neural tissue engineering. In this cell culture model, the biocomposite scaffolds SPID1COL1 and FIB3COL1 increased the proportion of cells that differentiated into neurons, astrocytes, and Nestin positive cells more substantially compared to laminin, and native Bovine Collagen Type I. The behavior of the SPID1COL1 and FIB3COL1 scaffolds superiorly mimicked the native ECM compared to the native Bovine Collagen Type I scaffolds, and are therefore encouraging for use as a component of a therapeutic strategy to repair the injured spinal cord. **(****FIG. 14****)**

### Tensile strength

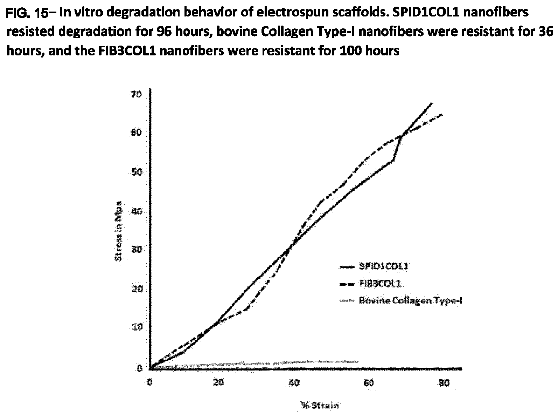

Sufficient tensile strength is essential for a peripheral nerve substitute, as it must withstand manipulation during surgery. In addition, subsequent tissue movements associated with the cardiorespiratory cycle and patient movement must be tolerated, especially when tissue begins to infiltrate the scaffolds and axonal growth increases [56]. Tensile properties of the electrospun SPID1COL1, FIB3COL1, and Bovine Collagen Type I nanofiber scaffolds were determined using a tabletop Sauter SD 500N100 tensile tester (Imlab) at a load cell capacity of 10 N. Dogbone shaped test specimens consisting of dimensions 10 mm breadth × 15 mm length, with a thickness of 500 µm were tested at a crosshead speed of 10 mm/min and gauge length of 20 mm, at room temperature **[57, 58].** A minimum of 20 specimens of individual scaffolds were tested until a break was endured; the results obtained were then plotted for the determination of the stress-strain curve of the scaffolds. **FIG. 15** shows the maximum stress-strain comparison for the electrospun SPID1COL1, FIB3COL1, and native Collagen Type I nanofibers. The maximum tensile strength of the SPID1COL1 and FIB3COL1 scaffolds were approximately 45-fold higher than the native Bovine Collagen Type I scaffold, performing at 122.51 MPa, with an average of 89.77 ± 2.18 MPa and an ultimate strain of 84% for SPID1COL1, and at 126.23 MPa, with an average of 91.49 ± 3.04 MPa and an ultimate strain of 82% for FIB3COL1, compared to a 1.32 MPa, with an average of 0.54 ± 0.68 MPa, and an elongation at break of 58% for the native Bovine Collagen Type I scaffold. The native Bovine Collagen Type I scaffolds therefore, had insufficient tensile strength to be used as a nerve graft alone, considering the well-known fact that the tensile strength of a fresh human sciatic nerve is 11.63 ± 1.80 MPa [59]. Both the SPID1COL1 and FIB3COL1 scaffolds show substantially improved tensile properties making them suitable for neural tissue engineering.

### Degradation

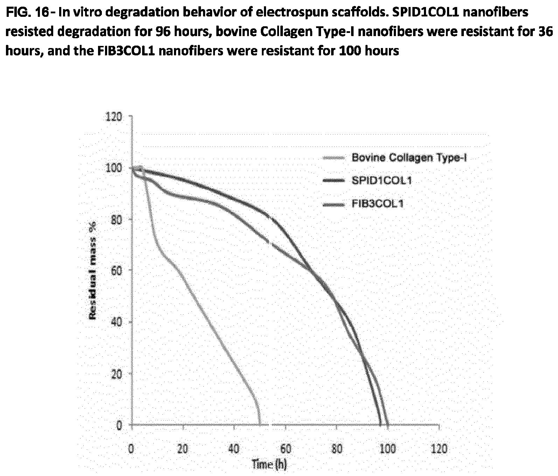

To determine the degradation rate of the SPID1COL1, FIB3COL1 scaffolds, a combination of lipase (7 mg/mL) and collagenase (1 mg/mL) was dissolved in PBS (pH 7.4). For the native Collagen Type I scaffolds a single concentration of 1mg/mL collagenase was used. The samples were weighed prior placement in a tube of the respective enzymatic solutions kept at 37°C. The samples were removed, blot-dried with paper cloth until the mass remained constant, and weighed after 2, 4, 8, 24 hours, and then every 24 hours, until the mass of the samples remained constant. The net weight of the scaffolds were calculated by subtracting the wet chamber weights from the scaffold-containing wet chamber weights Once the initial wet well weight was reached, a value of 0 was assigned. As shown in **FIG. 16****,** the Bovine Collagen Type I scaffolds degraded faster than both the SPID1COL1 and FIB3COL1 scaffolds. When incubated in collagenase solution at 37 °C for 64 hours, the native Bovine Collagen Type I nanofibers were resistant for up to 36 hours and were complete degraded at 64 hours. However, both the SPID1COL1 and FIB3COL1 scaffolds were substantially more stable and resisted both lipase and collagenase degradation as it took 100 hours for the complete degradation of the nanofibers. Both SPID1COL1 and FIB3COL1 scaffolds showed resistance up to 96 and 100 hours, respectively in the lipase/collagenase solution, showing their superiority towards degradation compared to native Bovine Collagen Type I.

### Conclusions

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub combination.

Although the invention has been described in conjunction with specific embodiments thereof, it is evident that many alternatives, modifications and variations will be apparent to those skilled in the art. In addition, citation or identification of any reference in this application shall not be construed as an admission that such reference is available as prior art to the present invention.

This work provides evidence that a combination of biochemical and topographical cues can influence the direction of cellular differentiation, and raises important questions regarding fatespecification mechanisms enhanced by substrate topography. Electrospun nanofibrous scaffolds provide mechanical stability, structural guidance, and a matrix for cell integration with surrounding tissue. Collagen physically supports cells by providing specific ligands for cell adhesion, thereby acting as an ECM-mimicking nano-scaffold. The SPID1COL1 and FIB3COL1 proteins show improved cell differentiation in vitro compared to native Collagen Type I and similar to how the native ECM does in vivo. We found increased fiber diameters, along with improved mechanical properties for both SPID1COL1 and FIB3COL1 nanofibers compared to native Collagen Type I nanofibers, thereby allowing a proportion of desired cell types to be controlled for possible therapeutic purposes.

### REFERENCES:

**1.** Frantz C, Stewart KM, Weaver VM. The extracellular matrix at a glance. J Cell Sci. 2010;123(Pt 24):4195-4200. doi:10.1242/jcs.023820
**2.** Järveläinen H, Sainio A, Koulu M, Wight TN, Penttinen R. Extracellular matrix molecules: potential targets in pharmacotherapy. Pharmacol Rev. 2009 Jun;61(2):198-223. doi: 10.1124/pr.109.001289. PMID: 19549927; PMCID: PMC2830117.
**3.** Schaefer L, Schaefer RM. Proteoglycans: from structural compounds to signaling molecules. Cell Tissue Res. 2010 Jan;339(1):237-46. doi: 10.1007/s00441-009-0821-y. Epub 2009 Jun 10. PMID: 19513755.
**4.** Alberts B., Johnson A., Lewis J., Raff M., Roberts K., Walter P. (2007). Molecular Biology of the Cell. London: Garland Science;
**5.** Harvey SJ, Miner JH. Revisiting the glomerular charge barrier in the molecular era. Curr Opin Nephrol Hypertens. 2008 Jul;17(4):393-8. doi: 10.1097/MNH.0b013e32830464de. PMID: 18660676.
**6.** Morita H, Yoshimura A, Inui K, Ideura T, Watanabe H, Wang L, Soininen R, Tryggvason K. Heparan sulfate of perlecan is involved in glomerular filtration. J Am Soc Nephrol. 2005 Jun;16(6):1703-10. doi: 10.1681/ASN.2004050387. Epub 2005 May 4. PMID: 15872080.
**7.** Rozario T, DeSimone DW. The extracellular matrix in development and morphogenesis: a dynamic view. Dev Biol. 2010 May 1;341(1):126-40. doi: 10.1016/j.ydbio.2009.10.026. Epub 2009 Oct 23. PMID: 19854168; PMCID: PMC2854274.
**8.** De Wever O, Demetter P, Mareel M, Bracke M. Stromal myofibroblasts are drivers of invasive cancer growth. Int J Cancer. 2008 Nov 15;123(10):2229-38. doi: 10.1002/ijc.23925. PMID: 18777559.
**9.** Wise SG, Weiss AS. Tropoelastin. Int J Biochem Cell Biol. 2009 Mar;41(3):494-7. doi: 10.1016/j.biocel.2008.03.017. Epub 2008 Apr 1. PMID: 18468477.
**10.** Lucero HA, Kagan HM. Lysyl oxidase: an oxidative enzyme and effector of cell function. Cell Mol Life Sci. 2006 Oct;63(19-20):2304-16. doi: 10.1007/s00018-006-6149-9. PMID: 16909208.
**11.** Smith ML, Gourdon D, Little WC, Kubow KE, Eguiluz RA, Luna-Morris S, Vogel V. Forceinduced unfolding of fibronectin in the extracellular matrix of living cells. PLoS Biol. 2007 Oct 2;5(10):e268. doi: 10.1371/journal.pbio.0050268. PMID: 17914904; PMCID: PMC1994993.
**12.** Trebaul A, Chan EK, Midwood KS. Regulation of fibroblast migration by tenascin-C. Biochem Soc Trans. 2007 Aug;35(Pt 4):695-7. doi: 10.1042/BST0350695. PMID: 17635125.
**13.** Tucker RP, Chiquet-Ehrismann R. The regulation of tenascin expression by tissue microenvironments. Biochim Biophys Acta. 2009 May;1793(5):888-92. doi: 10.1016/j.bbamcr.2008.12.012. Epub 2008 Dec 31. PMID: 19162090.
**14.** Merle C, Perret S, Lacour T, Jonval V, Hudaverdian S, Garrone R, Ruggiero F, Theisen M. Hydroxylated human homotrimeric collagen I in Agrobacterium tumefaciens-mediated transient expression and in transgenic tobacco plant. FEBS Lett. 2002 Mar 27;515(1-3):114-8. doi: 10.1016/s0014-5793(02)02452-3.
**15.** Torre-Blanco A, Alvizouri AM. In vitro hydroxylation of proline in the collagen of the cysticercus of Taenia solium. Comp Biochem Physiol B. 1987;88(4):1213-7. doi: 10.1016/0305-0491(87)90026-5.
**16.** Myllyharju J. Prolyl 4-hydroxylases, the key enzymes of collagen biosynthesis. Matrix Biol. 2003 Mar;22(1):15-24. doi: 10.1016/s0945-053x(03)00006-4.
**17.** Berg RA, Prockop DJ. The thermal transition of a non-hydroxylated form of collagen. Evidence for a role for hydroxyproline in stabilizing the triple-helix of collagen. Biochem Biophys Res Commun. 1973 May 1;52(1):115-20. doi: 10.1016/0006-291x(73)90961-3.
**18.** Annunen P, Helaakoski T, Myllyharju J, Veijola J, Pihlajaniemi T and Kivirikko KI (1997) Cloning of the human prolyl 4-hydroxylase α subunit isoform α(II) and characterization of the type II enzyme tetramer. The α(I) and α(II) subunits do not form a mixed α(I) α(II) β2 tetramer. J Biol Chem, 272, 17342-17348.
**19.** Gorres KL, Raines RT. Prolyl 4-hydroxylase. Crit Rev Biochem Mol Biol. 2010 Apr;45(2):106-24. doi: 10.3109/10409231003627991.
**20.** Myllyharju, J. & Kivirikko, K. I. Collagens, modifying enzymes and their mutations in humans, flies and worms. Trends Genet. 20, 33-43 (2004).
**21.** Guengerich FP. Introduction: Metals in Biology: α-Ketoglutarate/Iron-Dependent Dioxygenases. J Biol Chem. 2015;290(34):20700-20701. doi:10.1074/jbc.R115.675652
**22.** Ibrahimi A, Vande Velde G, Reumers V, Toelen J, Thiry I, Vandeputte C, Vets S, Deroose C, Bormans G, Baekelandt V, Debyser Z, Gijsbers R. Highly efficient multicistronic lentiviral vectors with peptide 2A sequences. Hum Gene Ther. 2009 Aug;20(8):845-60. doi: 10.1089/hum.2008.188.
**23.** Weber, E., Engler, C., Gruetzner, R., Werner, S., and Marillonnet, S. (2011). A modular cloning system for standardized assembly of multigene constructs. PLoS One 6:e16765. doi: 10.1371/journal.pone.0016765
**24.** Stavolone L, Kononova M, Pauli S, Ragozzino A, de Haan P, Milligan S, Lawton K, Hohn T. Cestrum yellow leaf curling virus (CmYLCV) promoter: a new strong constitutive promoter for heterologous gene expression in a wide variety of crops. Plant Mol Biol. 2003 Nov;53(5):663-73. doi: 10.1023/B:PLAN.0000019110.95420.bb.
**25.** Nagaya S, Kawamura K, Shinmyo A, Kato K. The HSP terminator of Arabidopsis thaliana increases gene expression in plant cells. Plant Cell Physiol. 2010 Feb;51(2):328-32. doi: 10.1093/pcp/pcp188.
**26.** Liu Z, Chen O, Wall JBJ, Zheng M, Zhou Y, Wang L, Vaseghi HR, Qian L, Liu J. Systematic comparison of 2A peptides for cloning multi-genes in a polycistronic vector. Sci Rep. 2017 May 19;7(1):2193. doi: 10.1038/s41598-017-02460-2.
**27.** Baltes NJ, Gil-Humanes J, Cermak T, Atkins PA, Voytas DF. DNA replicons for plant genome engineering. Plant Cell. 2014 Jan;26(1):151-63. doi: 10.1105/tpc.113.119792. Epub 2014 Jan 17. PMID: 24443519; PMCID: PMC3963565.
**28.** P. Bruckner, D.J. Prockop Anal. Biochem., 110 (1981), pp. 360-368
**29.** F. Ruggiero, J.-Y. Exposito, P. Bournat, V. Gruber, S. Perret, J. Comte, B. Olagnier, R. Garrone, M. Theisen FEBS Lett., 469 (2000), pp. 132-136
**30.** Michael A. Gimbrone, Jr.,Ramzi S. Cotran, Judah Folkman J Cell Biol (1974) 60 (3): 673-684. https://doi.org/10.1083/jcb.60.3.673
**31.** Nokelainen M, Tu H, Vuorela A, Notbohm H, Kivirikko KI, Myllyharju J. High-level production of human type I collagen in the yeast Pichia pastoris. Yeast. 2001 Jun 30;18(9):797-806. doi: 10.1002/yea.730.
**32.** Myllyharju J, Nokelainen M, Vuorela A, Kivirikko KI. Expression of recombinant human type I-III collagens in the yeast pichia pastoris. Biochem Soc Trans. 2000;28(4):353-7. PMID: 10961918.
**33.** Vuorela A, Myllyharju J, Nissi R, Pihlajaniemi T, Kivirikko KI. Assembly of human prolyl 4-hydroxylase and type III collagen in the yeast pichia pastoris: formation of a stable enzyme tetramer requires coexpression with collagen and assembly of a stable collagen requires coexpression with prolyl 4-hydroxylase. EMBO J. 1997 Nov 17;16(22):6702-12. doi: 10.1093/emboj/16.22.6702.
**34.** Stein H, Wilensky M, Tsafrir Y, Rosenthal M, Amir R, Avraham T, Ofir K, Dgany O, Yayon A, Shoseyov O. Production of bioactive, post-translationally modified, heterotrimeric, human recombinant type-I collagen in transgenic tobacco. Biomacromolecules. 2009 Sep 14;10(9):2640-5. doi: 10.1021/bm900571b.
**35.** Stoppato, M.; Stevens, H.Y.; Carletti, E.; Migliaresi, C.; Motta, A.; Guldberg, R.E. Effects of silk fibroin fiber incorporation on mechanical properties, endothelial cell colonization and vascularization of PDLLA scaffolds. Biomaterials 2013, 34, 4573-4581
**36.** Mobini, S.; Hoyer, B.; Solati-Hashjin, M.; Lode, A.; Nosoudi, N.; Samadikuchaksaraei, A.; Gelinsky, M. Fabrication and characterization of regenerated silk scaffolds reinforced with natural silk fibers. J. Biomed. Mater. Res. A 2013, 101, 2392-2404.
**37.** Park, S.; Edwards, S.; Hou, S.; Boudreau, R.; Yee, R.; Jeong, K.J. Multi-interpenetrating network (ipn) hydrogel by gelatin and silk fibroin. Biomater. Sci. 2019, 7, 1276-1280.
**38.** Panas-Perez, E.; Gatt, C.J.; Dunn, M.G. Development of a silk and collagen fiber scaffold for anterior cruciate ligament reconstruction. J. Mater. Sci. Mater. Med. 2013, 24, 257-265
**39.** Ghezzi, C.E.; Marelli, B.; Muja, N.; Hirota, N.; Martin, J.G.; Barralet, J.E.; Alessandrino, A.; Freddi, G.; Nazhat, S.N. Mesenchymal stem cell-seeded multilayered dense collagensilk fibroin hybrid for tissue engineering applications. Biotechnol. J. 2011, 6, 1198-1207
**40.** Vasconcelos, A.; Gomes, A.C.; Cavaco-Paulo, A. Novel silk fibroin/elastin wound dressing. Acta Biomater. 2012, 8, 3049-3060
**41.** Shunji Yunoki, Toshiyuki Ikoma, Junzo Tanaka. Development of collagen condensation method to improve mechanical strength of tissue engineering scaffolds, Material Characterization, Volume 61, Issue 9, 2010, Pages 907-911, ISSN 1044-5803, https://doi.org/10.1016/j.matchar.2010.05.010.
**42.** Gatesy, J.; Hayashi, C.; Motriuk, D.; Woods, J.; Lewis, R. Extreme diversity, conservation, and
   convergence of spider silk fibroin sequences. Science 2001, 291, 2603-2605
**43.** Römer L, Scheibel T. The elaborate structure of spider silk: structure and function of a natural high performance fiber. Prion. 2008;2(4):154-161. doi:10.4161/pri.2.4.7490
**44.** Hayashi, C.Y.; Shipley, N.H.; Lewis, R.V. Hypotheses that correlate the sequence, structure, and mechanical properties of spider silk proteins. Int. J. Biol. Macromol. 1999, 24, 271-275
**45.** Zhao, L.; Chen, D.; Yao, Q.; Li, M. Studies on the use of recombinant spider silk protein/polyvinyl alcohol electrospinning membrane as wound dressing. Int. J. Nanomed. 2017, 12, 8103-8114
**46.** Meng, Z.X.; Wang, Y.S.; Ma, C.; Zheng, W.; Li, L.; Zheng, Y.F. Electrospinning of PLGA/gelatin randomly oriented and aligned nanofibers as potential scaffold in tissue engineering. Mater. Sci. Eng. 2010, 30, 1204-1210
**47.** Telemeco T, Ayres C, Bowlin G, Wnek G, Boland E, Cohen N, et al. Regulation of cellular infiltration into tissue engineering scaffolds composed of submicron diameter fibrils produced by electrospinning. Acta Biomater. 2005;1:377-385. doi: 10.1016/j.actbio.2005.04.006
**48.** Rho KS, Jeong L, Lee G, Seo BM, Park YJ, Hong SD, et al. Electrospinning of collagen nanofibers: effects on the behavior of normal human keratinocytes and early-stage wound healing. Biomaterials. 2006;27:1452-1461. doi:
   10.1016/j.biomaterials.2005.08.004.
**49.** Buttafoco L, Kolkman N, Engbers-Buijtenhuijs P, Poot A, Dijkstra P, Vermes I, et al. Electrospinning of collagen and elastin for tissue engineering applications. Biomaterials. 2006;27:724-734. doi: 10.1016/j.biomaterials.2005.06.024.
**50.** Matthews JA, Wnek GE, Simpson DG, Bowlin GL. Electrospinning of collagen nanofibers. Biomacromolecules 2002;3:232-238.
**51.** Zhang X, Reagan MR, Kaplan DL. Electrospun silk biomaterial scaffolds for regenerative medicine. Adv Drug Deliv Rev 2009;61:988-1006.
**52.** Boland ED, Matthews JA, Pawlowski KJ, Simpson DG, Wnek GE, Bowlin GL. Electrospinning collagen and elastin: preliminary vascular tissue engineering. Front Biosci 2004;9:1422-1432.
**53.** Rho KS, Jeong L, Lee G, Seo B, Park YJ, Hong S, Roh S, Cho JJ, Park WH, Min B. Electrospinning of collagen nanofibers: Effects on the behavior of normal human keratinocytes and early-stage wound healing. Biomaterials 2006;27:1452-1461.
**54.** Zhang X, Baughman CB, Kaplan DL. In vitro evaluation of electrospun silk fibroin scaffolds
   for vascular cell growth. Biomaterials 2008;29:2217-2227.
**55.** Noh HK, Lee SW, Kim J, Oh J, Kim K, Chung C, Choi S, Park WH, Min B. Electrospinning of chitin nanofibers: Degradation behavior and cellular response to normal human keratinocytes and fibroblasts. Biomaterials 2006;27:3934-3944.
**56.** Ma, M.; Wei, P.; Wei, T.; Ransohoff, R.M.; Jakeman, L.B. Enhanced axonal growth into a spinal cord contusion injury site in a strain of mouse (129X1/SvJ) with a diminished inflammatory response. J. Comp. Neurol. 2004, 474, 469-486.
**57.** Prabhakaran, M.P.; Venugopal, J.; Chan, C.K.; Ramakrishna, S. Surface modified electrospun nanofibrous scaffolds for nerve tissue engineering. Nanotechnology 2008, 19, 455102-455109.
**58.** Mobarakeh, L.G.; Prabhakaran, M.P.; Morshed, M.; Esfahani, M.H.N.; Ramakrishna, S. Electropsun PCL/gelatin nanofibrous scaffolds for nerve tissue engineering. Biomaterials 2008, 29, 4532-4539.
**59.** Borschel GH, Kia KF, Kuzon WM Jr, Dennis RG. Mechanical properties of acellular peripheral nerve. J Surg Res. 2003 Oct;114(2):133-9. doi: 10.1016/s0022-4804(03)00255-5.
**60.** Perona R. Cell signalling: growth factors and tyrosine kinase receptors. Clin Transl Oncol. 2006;8(2):77-82. doi:10.1007/s12094-006-0162-1
**61.** Richardson, S.M. Tissue engineering today, not tomorrow. Regen. Med. 2007, 2, 91-94
**62.** Sahoo, S.; Ang, L.T.; Goh, J.C.H.; Toh, S.L. Growth factor delivery through electrospun nanofibers in scaffolds for tissue engineering applications. J. Biomed. Mater. Res. Part A 2009, 4, 1539-1550.

### SEQUENCE LISTING

**SEQ NO 1: Amino Acid Sequence of Collagen Type I Alpha I** 
10 20 30 40 50 60
QLSYGYDEKS TGGISVPGPM GPSGPRGLPG PPGAPGPQGF QGPPGEPGEP GASGPMGPRG

70 80 90 100 110 120
PPGPPGKNGD DGEAGKPGRP GERGPPGPQG ARGLPGTAGL PGMKGHRGFS GLDGAKGDAG

130 140 150 160 170 180
PAGPKGEPGS PGENGAPGQM GPRGLPGERG RPGAPGPAGA RGNDGATGAA GPPGPTGPAG

190 200 210 220 230 240
PPGFPGAVGA KGEAGPQGPR GSEGPQGVRG EPGPPGPAGA AGPAGNPGAD GQPGAKGANG

250 260 270 280 290 300
APGIAGAPGF PGARGPSGPQ GPGGPPGPKG NSGEPGAPGS KGDTGAKGEP GPVGVQGPPG

310 320 330 340 350 360
PAGEEGKRGA RGEPGPTGLP GPPGERGGPG SRGFPGADGV AGPKGPAGER GSPGPAGPKG

370 380 390 400 410 420
SPGEAGRPGE AGLPGAKGLT GSPGSPGPDG KTGPPGPAGQ DGRPGPPGPP GARGQAGVMG

430 440 450 460 470 480
FPGPKGAAGE PGKAGERGVP GPPGAVGPAG KDGEAGAQGP PGPAGPAGER GEQGPAGSPG

490 500 510 520 530 540
FQGLPGPAGP PGEAGKPGEQ GVPGDLGAPG PSGARGERGF PGERGVQGPP GPAGPRGANG

550 560 570 580 590 600
APGNDGAKGD AGAPGAPGSQ GAPGLQGMPG ERGAAGLPGP KGDRGDAGPK GADGSPGKDG

610 620 630 640 650 660
VRGLTGPIGP PGPAGAPGDK GESGPSGPAG PTGARGAPGD RGEPGPPGPA GFAGPPGADG

670 680 690 700 710 720
QPGAKGEPGD AGAKGDAGPP GPAGPAGPPG PIGNVGAPGA KGARGSAGPP GATGFPGAAG

730 740 750 760 770 780
RVGPPGPSGN AGPPGPPGPA GKEGGKGPRG ETGPAGRPGE VGPPGPPGPA GEKGSPGADG

790 800 810 820 830 840
PAGAPGTPGP QGIAGQRGVV GLPGQRGERG FPGLPGPSGE PGKQGPSGAS GERGPPGPMG

850 860 870 880 890 900
PPGLAGPPGE SGREGAPGAE GSPGRDGSPG AKGDRGETGP AGPPGAPGAP GAPGPVGPAG

910 920 930 940 950 960
KSGDRGETGP AGPAGPVGPV GARGPAGPQG PRGDKGETGE QGDRGIKGHR GFSGLQGPPG

970 980 990 1000 1010 1020
PPGSPGEQGP SGASGPAGPR GPPGSAGAPG KDGLNGLPGP IGPPGPRGRT GDAGPVGPPG

1030 1040 1050
PPGPPGPPGP PSAGFDFSFL PQPPQEKAHD GGRYYRA
**SEQ NO 2: Amino Acid Sequence of Collagen Type I Alpha II** 
10 20 30 40 50 60
QYDGKGVGLG PGPMGLMGPR GPPGAAGAPG PQGFQGPAGE PGEPGQTGPA GARGPAGPPG

70 80 90 100 110 120
KAGEDGHPGK PGRPGERGVV GPQGARGFPG TPGLPGFKGI RGHNGLDGLK GQPGAPGVKG

130 140 150 160 170 180
EPGAPGENGT PGQTGARGLP GERGRVGAPG PAGARGSDGS VGPVGPAGPI GSAGPPGFPG

190 200 210 220 230 240
APGPKGEIGA VGNAGPAGPA GPRGEVGLPG LSGPVGPPGN PGANGLTGAK GAAGLPGVAG

250 260 270 280 290 300
APGLPGPRGI PGPVGAAGAT GARGLVGEPG PAGSKGESGN KGEPGSAGPQ GPPGPSGEEG

310 320 330 340 350 360
KRGPNGEAGS AGPPGPPGLR GSPGSRGLPG ADGRAGVMGP PGSRGASGPA GVRGPNGDAG

370 380 390 400 410 420
RPGEPGLMGP RGLPGSPGNI GPAGKEGPVG LPGIDGRPGP IGPAGARGEP GNIGFPGPKG

430 440 450 460 470 480
PTGDPGKNGD KGHAGLAGAR GAPGPDGNNG AQGPPGPQGV QGGKGEQGPP GPPGFQGLPG

490 500 510 520 530 540
PSGPAGEVGK PGERGLHGEF GLPGPAGPRG ERGPPGESGA AGPTGPIGSR GPSGPPGPDG

550 560 570 580 590 600
NKGEPGVVGA VGTAGPSGPS GLPGERGAAG IPGGKGEKGE PGLRGEIGNP GRDGARGAPG

610 620 630 640 650 660
AVGAPGPAGA TGDRGEAGAA GPAGPAGPRG SPGERGEVGP AGPNGFAGPA GAAGQPGAKG

670 680 690 700 710 720
ERGAKGPKGE NGVVGPTGPV GAAGPAGPNG PPGPAGSRGD GGPPGMTGFP GAAGRTGPPG

730 740 750 760 770 780
PSGISGPPGP PGPAGKEGLR GPRGDQGPVG RTGEVGAVGP PGFAGEKGPS GEAGTAGPPG

790 800 810 820 830 840
TPGPQGLLGA PGILGLPGSR GERGLPGVAG AVGEPGPLGI AGPPGARGPP GAVGSPGVNG

850 860 870 880 890 900
APGEAGRDGN PGNDGPPGRD GQPGHKGERG YPGNIGPVGA AGAPGPHGPV GPAGKHGNRG

910 920 930 940 950 960
ETGPSGPVGP AGAVGPRGPS GPQGIRGDKG EPGEKGPRGL PGLKGHNGLQ GLPGIAGHHG

970 980 990 1000 1010 1020
DQGAPGSVGP AGPRGPAGPS GPAGKDGRTG HPGTVGPAGI RGPQGHQGPA GPPGPPGPPG

1030 1040
PPGVSGGGYD FGYDGDFYRA
**SEQ NO 3: Nucleotide Sequence of Collagen Type I Alpha I, Codon Optimized for Nicotiana Benthamiana Chloroplast Expression** 
1 CAGTTGTCTT ATGGTTATGA TGAAAAATCA ACTGGAGGAA TTAGTGTTCC
AGGTCCAATG
61 GGACCATCTG GACCAAGAGG TCTTCCTGGA CCTCCAGGTG CTCCTGGTCC
ACAGGGTTTT
121 CAGGGACCAC CAGGAGAACC AGGAGAGCCA GGAGCTTCAG GTCCTATGGG
TCCAAGAGGT
181 CCACCTGGCC CTCCAGGAAA GAATGGTGAT GATGGAGAAG CAGGAAAGCC
TGGTCGTCCA
241 GGCGAAAGAG GTCCTCCTGG ACCACAAGGG GCTAGAGGAC TGCCTGGTAC
TGCTGGACTT
301 CCAGGAATGA AAGGTCATAG AGGTTTTTCT GGACTTGACG GTGCTAAGGG
AGATGCAGGA
361 CCAGCTGGAC CTAAGGGTGA GCCAGGATCT CCAGGCGAGA ACGGAGCCCC
TGGTCAGATG
421 GGACCAAGAG GATTGCCAGG TGAAAGAGGA AGGCCTGGAG CTCCTGGTCC
AGCTGGAGCT
481 AGGGGTAATG ATGGAGCTAC TGGAGCTGCA GGACCTCCTG GTCCAACTGG
TCCTGCTGGA
541 CCACCAGGTT TTCCTGGAGC TGTGGGAGCT AAAGGTGAGG CTGGTCCACA
AGGTCCTAGA
601 GGATCAGAAG GACCCCAAGG AGTTAGAGGA GAACCAGGTC CACCTGGACC
AGCCGGTGCA
661 GCTGGTCCTG CTGGTAATCC TGGTGCTGAC GGACAACCTG GCGCTAAAGG
TGCAAACGGA
721 GCTCCTGGAA TCGCAGGTGC TCCAGGTTTT CCAGGTGCAA GAGGTCCTAG
TGGTCCACAG
781 GGTCCAGGAG GTCCACCAGG ACCAAAGGGT AATAGTGGTG AGCCTGGAGC
TCCAGGAAGC
841 AAAGGAGATA CTGGTGCTAA GGGCGAACCA GGACCAGTTG GAGTGCAAGG
ACCTCCAGGA
901 CCAGCAGGAG AAGAAGGTAA GAGAGGAGCT AGGGGAGAAC CAGGACCTAC
TGGTTTGCCA
961 GGACCACCAG GTGAACGTGG AGGACCTGGA TCAAGGGGTT TTCCAGGAGC
TGATGGGGTT
1021 GCTGGTCCTA AGGGACCAGC AGGAGAAAGA GGATCTCCAG GTCCTGCTGG
ACCAAAAGGA
1081 AGTCCTGGAG AAGCTGGCAG ACCTGGAGAA GCAGGTCTTC CAGGTGCTAA
GGGTCTTACT
1141 GGATCTCCAG GATCTCCTGG TCCTGATGGA AAAACTGGAC CACCAGGTCC
TGCTGGACAA
1201 GACGGTAGAC CTGGACCTCC TGGTCCACCT GGAGCTAGAG GTCAAGCTGG
TGTTATGGGA
1261 TTTCCTGGAC CAAAGGGTGC TGCTGGTGAA CCAGGAAAGG CTGGTGAAAG
AGGAGTGCCT
1321 GGTCCACCTG GAGCTGTTGG CCCTGCTGGA AAGGATGGTG AAGCTGGTGC
TCAAGGACCA
1381 CCAGGACCTG CTGGTCCTGC TGGAGAAAGA GGTGAGCAGG GACCAGCTGG
AAGTCCTGGT
1441 TTTCAAGGAT TGCCAGGACC AGCTGGTCCT CCAGGGGAAG CAGGTAAGCC
AGGCGAACAA
1501 GGAGTCCCAG GAGATTTGGG AGCTCCTGGA CCATCTGGAG CAAGAGGTGA
AAGAGGATTT
 
1561 CCAGGAGAAA GAGGAGTTCA GGGTCCGCCA GGACCTGCTG GACCAAGAGG
AGCAAACGGA
1621 GCACCAGGAA ATGATGGAGC TAAGGGGGAT GCTGGTGCTC CAGGTGCACC
TGGATCTCAA
1681 GGAGCTCCAG GACTCCAAGG AATGCCTGGT GAAAGAGGTG CTGCTGGTCT
TCCAGGACCT
1741 AAGGGAGATA GAGGAGATGC AGGACCAAAG GGAGCTGATG GAAGCCCTGG
TAAGGATGGT
1801 GTTAGAGGAC TTACTGGACC AATAGGTCCT CCTGGTCCAG CTGGAGCACC
TGGGGATAAG
1861 GGTGAGAGTG GTCCTTCTGG TCCTGCAGGC CCAACAGGAG CAAGAGGTGC
TCCTGGTGAT
1921 AGAGGTGAAC CTGGACCTCC AGGTCCTGCT GGATTTGCTG GTCCACCTGG
TGCTGATGGA
1981 CAACCTGGAG CAAAGGGAGA GCCTGGAGAT GCAGGAGCAA AAGGAGATGC
TGGTCCACCT
2041 GGACCAGCTG GTCCTGCTGG TCCTCCTGGA CCAATCGGTA ATGTTGGAGC
TCCTGGTGCT
2101 AAAGGTGCTA GGGGTTCAGC TGGACCTCCT GGAGCTACTG GTTTTCCTGG
TGCTGCTGGC
2161 AGGGTTGGAC CACCTGGTCC AAGTGGAAAT GCCGGACCAC CTGGCCCACC
AGGACCAGCT
2221 GGAAAAGAAG GTGGAAAAGG ACCAAGAGGA GAAACTGGTC CAGCAGGTCG
TCCAGGTGAA
2281 GTGGGCCCTC CAGGCCCACC AGGACCTGCT GGAGAAAAGG GAAGTCCAGG
TGCAGATGGA
2341 CCAGCTGGCG CTCCTGGTAC TCCAGGACCT CAGGGTATCG CTGGACAAAG
AGGTGTTGTT
2401 GGTTTGCCAG GTCAGAGAGG AGAGAGAGGT TTTCCAGGAT TGCCAGGTCC
TTCTGGTGAG
2461 CCTGGTAAAC AGGGTCCTTC TGGAGCTTCT GGTGAAAGAG GACCTCCTGG
TCCTATGGGT
2521 CCACCAGGAT TGGCAGGACC ACCAGGTGAA TCTGGAAGAG AAGGTGCACC
AGGAGCAGAA
2581 GGATCTCCAG GTAGGGATGG AAGCCCTGGG GCTAAAGGAG ATAGGGGAGA
AACTGGACCA
2641 GCAGGACCAC CAGGTGCTCC TGGTGCCCCA GGTGCTCCTG GACCAGTTGG
TCCTGCTGGT
2701 AAGTCTGGTG ACAGAGGTGA AACTGGGCCA GCTGGACCAG CTGGACCTGT
TGGTCCTGTT
2761 GGTGCTAGAG GTCCAGCTGG ACCTCAAGGT CCTAGAGGAG ATAAAGGAGA
AACTGGTGAA
2821 CAAGGTGATA GGGGTATTAA GGGTCATAGG GGATTTTCTG GTTTGCAAGG
ACCACCTGGA
2881 CCACCAGGTT CACCAGGAGA GCAAGGTCCA AGTGGAGCAT CTGGACCAGC
TGGTCCAAGG
2941 GGACCTCCTG GATCTGCTGG AGCTCCAGGT AAAGATGGAC TTAATGGTCT
TCCAGGTCCA
3001 ATTGGACCTC CTGGACCAAG AGGAAGAACT GGAGATGCAG GACCAGTTGG
ACCACCAGGT
3061 CCACCAGGAC CTCCTGGTCC TCCAGGACCT CCAAGTGCAG GTTTTGATTT
TTCATTTCTT
3121 CCTCAACCAC CACAAGAGAA GGCTCACGAT GGAGGAAGGT ATTATAGAGC TTAA
**SEQ NO 4: Nucleotide Sequence of Collagen Type I Alpha II, Codon Optimized for Nicotiana Benthamiana Chloroplast Expression** 
1 CAATACGATG GAAAAGGAGT TGGTCTCGGA CCAGGTCCAA TGGGTTTGAT
GGGACCAAGA
61 GGTCCTCCAG GAGCTGCTGG TGCTCCTGGT CCTCAAGGAT TTCAAGGACC
AGCTGGAGAG
121 CCAGGTGAGC CTGGACAGAC TGGTCCAGCT GGTGCAAGAG GACCTGCAGG
ACCTCCCGGT
181 AAAGCTGGTG AAGATGGACA TCCAGGAAAA CCAGGAAGAC CAGGAGAGAG
GGGTGTCGTT
241 GGACCACAAG GTGCAAGAGG TTTTCCAGGA ACACCAGGTC TTCCTGGTTT
TAAAGGTATT
301 AGAGGGCACA ATGGTTTGGA TGGTTTGAAG GGTCAACCAG GTGCTCCAGG
AGTTAAAGGA
361 GAACCTGGTG CTCCAGGTGA AAATGGTACT CCGGGACAAA CTGGAGCAAG
AGGATTGCCT
421 GGAGAAAGGG GTCGTGTTGG TGCACCAGGT CCTGCAGGAG CAAGAGGTTC
AGATGGATCT
481 GTGGGTCCAG TTGGTCCTGC TGGACCAATT GGTTCTGCTG GTCCTCCTGG
ATTTCCAGGA
541 GCTCCTGGAC CAAAGGGAGA AATTGGAGCA GTTGGAAATG CAGGACCTGC
TGGTCCTGCT
601 GGACCTAGAG GTGAAGTTGG ATTGCCTGGT TTGTCGGGCC CAGTAGGTCC
TCCAGGAAAT
661 CCAGGAGCTA ATGGATTGAC TGGTGCTAAA GGAGCTGCTG GATTGCCTGG
TGTGGCAGGT
721 GCTCCTGGTC TTCCAGGTCC TAGAGGCATT CCTGGTCCAG TAGGAGCTGC
AGGAGCTACT
781 GGTGCAAGAG GTCTTGTTGG AGAACCAGGA CCCGCAGGTT CAAAAGGAGA
ATCTGGAAAT
841 AAAGGTGAAC CAGGATCTGC TGGACCTCAG GGTCCACCTG GTCCTAGTGG
TGAAGAAGGA
901 AAGAGAGGAC CTAATGGTGA GGCCGGAAGC GCTGGTCCTC CTGGACCACC
AGGTCTTAGA
961 GGAAGTCCTG GTAGTAGAGG ATTGCCAGGA GCAGATGGAA GAGCTGGTGT
TATGGGACCA
1021 CCAGGTTCTA GAGGAGCTAG CGGACCAGCT GGAGTGAGGG GTCCAAATGG
AGATGCTGGA
1081 AGGCCTGGAG AACCAGGATT GATGGGTCCT AGGGGTTTAC CAGGAAGTCC
AGGAAATATT
1141 GGACCAGCAG GTAAAGAAGG ACCTGTGGGT TTGCCAGGAA TTGATGGAAG
GCCAGGACCA
1201 ATTGGACCAG CTGGTGCTAG AGGAGAGCCT GGTAATATTG GTTTTCCAGG
TCCAAAGGGT
1261 CCAACTGGAG ACCCTGGAAA GAACGGTGAT AAAGGACATG CAGGACTTGC
TGGAGCAAGA
1321 GGAGCTCCTG GCCCTGATGG TAATAATGGT GCTCAAGGTC CTCCAGGACC
ACAAGGTGTT
1381 CAAGGAGGAA AAGGTGAGCA AGGACCACCT GGACCACCAG GTTTTCAAGG
ACTTCCTGGC
1441 CCATCTGGTC CAGCTGGTGA AGTTGGAAAA CCAGGAGAGA GAGGTCTTCA
TGGAGAATTT
1501 GGACTTCCAG GACCAGCTGG CCCTAGAGGA GAAAGAGGAC CTCCAGGTGA
ATCTGGTGCT
1561 GCAGGTCCAA CTGGACCAAT TGGTTCCAGA GGACCATCCG GACCTCCTGG
ACCAGATGGA
 
1621 AATAAAGGTG AACCAGGAGT TGTGGGTGCT GTTGGTACAG CAGGTCCATC
AGGTCCATCT
1681 GGTCTTCCAG GAGAGAGGGG CGCTGCTGGT ATTCCTGGTG GAAAGGGAGA
GAAGGGCGAA
1741 CCAGGACTCA GAGGTGAAAT TGGAAATCCC GGAAGAGATG GAGCAAGAGG
AGCTCCTGGA
1801 GCTGTTGGTG CTCCAGGACC AGCTGGTGCA ACAGGTGATA GGGGTGAAGC
TGGGGCTGCT
1861 GGACCTGCTG GACCAGCTGG TCCTAGGGGT TCTCCTGGAG AAAGAGGTGA
GGTAGGTCCT
1921 GCTGGACCTA ATGGTTTTGC TGGGCCAGCC GGTGCTGCTG GACAACCAGG
AGCCAAGGGA
1981 GAGAGAGGAG CTAAAGGACC AAAAGGAGAG AATGGAGTCG TTGGTCCTAC
TGGACCAGTT
2041 GGAGCTGCTG GACCAGCTGG ACCAAATGGA CCACCAGGAC CAGCTGGATC
TAGAGGAGAT
2101 GGTGGACCAC CAGGTATGAC AGGTTTCCCA GGTGCAGCTG GAAGGACTGG
ACCTCCAGGG
2161 CCATCAGGTA TTTCTGGACC TCCAGGACCA CCAGGTCCAG CTGGAAAAGA
GGGTCTCAGA
2221 GGACCAAGAG GAGATCAAGG ACCAGTGGGA AGAACAGGTG AAGTGGGTGC
TGTGGGTCCA
2281 CCTGGTTTTG CTGGGGAGAA AGGTCCTTCC GGAGAAGCTG GAACTGCAGG
TCCACCAGGA
2341 ACTCCAGGTC CACAGGGTTT GCTTGGAGCT CCTGGAATTC TTGGTTTACC
TGGTTCAAGA
2401 GGAGAAAGAG GTCTTCCTGG TGTTGCTGGA GCCGTTGGAG AGCCAGGACC
ATTGGGAATT
2461 GCTGGACCTC CAGGTGCTAG GGGACCACCT GGCGCTGTGG GATCTCCAGG
CGTGAATGGT
2521 GCACCAGGAG AGGCAGGAAG GGATGGTAAT CCGGGTAACG ATGGACCTCC
AGGTAGAGAT
2581 GGGCAACCAG GACACAAGGG GGAAAGGGGT TATCCAGGAA ATATTGGACC
TGTTGGAGCT
2641 GCAGGTGCTC CAGGTCCTCA TGGACCAGTT GGACCTGCAG GAAAACATGG
AAATAGAGGA
2701 GAGACTGGTC CTTCAGGTCC TGTGGGACCA GCAGGTGCTG TTGGTCCTAG
AGGTCCATCA
2761 GGACCACAAG GTATTAGAGG AGATAAGGGA GAGCCAGGAG AAAAGGGACC
AAGAGGTTTA
2821 CCTGGTTTGA AAGGACATAA TGGATTGCAG GGGCTTCCAG GTATTGCTGG
CCATCACGGT
2881 GATCAGGGAG CTCCAGGTTC TGTAGGTCCA GCAGGTCCAA GGGGACCAGC
TGGTCCTTCT
2941 GGACCTGCTG GTAAGGATGG TAGGACTGGT CATCCAGGAA CTGTGGGACC
AGCAGGAATT
3001 AGGGGTCCTC AAGGGCATCA AGGTCCTGCT GGTCCACCTG GACCTCCAGG
TCCTCCAGGA
3061 CCTCCTGGTG TTTCTGGTGG TGGGTATGAT TTTGGTTACG ATGGAGATTT
TTATAGGGCA
3121 TGA
**SEQ NO 5: Amino Acid Sequence of Spidroin-I** 
10         20         30         40         50         60
QGAGAAAAAA GGAGQGGYGG LGGQGAGQGG YGGLGGQGAG QGAGAAAAAA AGGAGQGGYG

70         80         90        100        110        120
GLGSQGAGRG GQGAGAAAAA AGGAGQGGYG GLGSQGAGRG GLGGQGAGAA AAAAAGGAGQ

130        140        150        160        170        180
GGYGGLGNQG AGRGGQGAAA AAAGGAGQGG YGGLGSQGAG RGGLGGQGAG AAAAAAGGAG

190        200        210        220        230        240
QGGYGGLGGQ GAGQGGYGGL GSQGAGRGGL GGQGAGAAAA AAAGGAGQGG LGGQGAGQGA

250        260        270        280        290        300
GASAAAAGGA GQGGYGGLGS QGAGRGGEGA GAAAAAAGGA GQGGYGGLGG QGAGQGGYGG

310        320        330        340        350        360
LGSQGAGRGG LGGQGAGAAA AGGAGQGGLG GQGAGQGAGA AAAAAGGAGQ GGYGGLGSQG

370        380        390        400        410        420
AGRGGLGGQG AGAVAAAAAG GAGQGGYGGL GSQGAGRGGQ GAGAAAAAAG GAGQRGYGGL

430        440        450        460        470        480
GNQGAGRGGL GGQGAGAAAA AAAGGAGQGG YGGLGNQGAG RGGQGAAAAA GGAGQGGYGG

490        500        510        520        530        540
LGSQGAGRGG QGAGAAAAAA VGAGQEGIRG QGAGQGGYGG LGSQGSGRGG LGGQGAGAAA

550        560        570        580        590        600
AAAGGAGQGG LGGQGAGQGA GAAAAAAGGV RQGGYGGLGS QGAGRGGQGA GAAAAAAGGA

610        620        630        640        650        660
GQGGYGGLGG QGVGRGGLGG QGAGAAAAGG AGQGGYGGVG SGASAASAAA SRLSSPQASS

670        680        690        700        710        720
RVSSAVSNLV ASGPTNSAAL SSTISNVVSQ IGASNPGLSG CDVLIQALLE VVSALIQILG

730        740
SSSIGQVNYG SAGQATQIVG QSVYQALG
**SEQ NO 6: Nucleotide Sequence of Spidroin-I, Codon Optimized for Nicotiana Benthamiana Chloroplast Expression** 
1 CAAGGAGCTG GAGCTGCAGC TGCAGCTGCT GGAGGAGCTG GACAAGGTGG
ATATGGTGGT
61 TTGGGAGGTC AAGGTGCTGG ACAAGGAGGT TACGGAGGTC TTGGAGGACA
GGGAGCTGGA
121 CAAGGTGCTG GTGCTGCAGC TGCTGCTGCT GCTGGAGGAG CTGGTCAAGG
AGGTTACGGT
181 GGTTTAGGTT CACAAGGAGC TGGTAGAGGA GGACAGGGTG CAGGTGCTGC
TGCTGCAGCA
241 GCAGGAGGTG CTGGACAGGG CGGTTATGGT GGTTTGGGTT CCCAAGGAGC
TGGAAGGGGA
301 GGATTAGGTG GACAGGGAGC TGGTGCTGCT GCTGCTGCTG CTGCAGGAGG
TGCTGGTCAG
361 GGTGGCTATG GTGGTTTGGG GAATCAAGGA GCTGGAAGGG GTGGTCAAGG
AGCTGCAGCA
421 GCTGCTGCTG GAGGTGCTGG ACAAGGCGGT TACGGAGGTT TGGGATCTCA
GGGTGCAGGA
481 AGAGGAGGTC TTGGCGGACA GGGAGCTGGA GCTGCTGCCG CTGCTGCTGG
AGGTGCAGGA
541 CAAGGCGGTT ATGGTGGTCT TGGTGGTCAA GGAGCAGGAC AAGGTGGGTA
CGGGGGACTT
601 GGAAGTCAGG GTGCTGGTAG GGGAGGACTT GGAGGACAAG GTGCTGGGGC
AGCTGCTGCA
661 GCTGCTGCTG GAGGTGCTGG ACAAGGAGGT CTTGGTGGAC AGGGAGCAGG
ACAAGGTGCT
721 GGAGCATCTG CAGCTGCAGC AGGTGGAGCA GGGCAGGGAG GATACGGAGG
ACTTGGTTCT
781 CAAGGTGCTG GTAGAGGAGG TGAAGGAGCA GGTGCTGCTG CAGCTGCAGC
TGGAGGCGCT
841 GGACAAGGAG GCTATGGAGG ACTTGGTGGA CAGGGGGCTG GACAGGGTGG
TTATGGAGGT
901 CTTGGATCCC AGGGAGCTGG GAGGGGAGGA CTCGGAGGAC AAGGAGCTGG
AGCTGCAGCT
961 GCTGGTGGTG CTGGACAAGG TGGACTTGGT GGACAAGGAG CTGGCCAAGG
TGCTGGAGCT
1021 GCAGCTGCTG CTGCTGGTGG TGCAGGTCAA GGAGGATATG GAGGACTTGG
AAGTCAAGGA
1081 GCTGGAAGAG GAGGTCTTGG TGGTCAAGGT GCCGGAGCAG TTGCTGCCGC
TGCTGCTGGA
1141 GGTGCTGGTC AGGGTGGATA TGGAGGTCTT GGTTCTCAGG GTGCAGGAAG
AGGAGGTCAA
1201 GGAGCAGGTG CTGCAGCTGC AGCTGCAGGT GGAGCTGGTC AAAGAGGATA
CGGAGGACTT
1261 GGAAACCAGG GTGCTGGTAG AGGAGGCCTT GGAGGTCAGG GAGCTGGGGC
TGCTGCTGCT
1321 GCTGCAGCTG GTGGTGCTGG ACAAGGTGGA TATGGTGGAC TCGGAAATCA
GGGTGCTGGA
1381 AGAGGTGGAC AAGGTGCTGC AGCTGCAGCT GGCGGAGCAG GACAAGGTGG
ATATGGTGGT
1441 TTGGGTTCAC AGGGAGCAGG AAGAGGAGGA CAGGGAGCTG GTGCTGCTGC
TGCAGCAGCT
1501 GTTGGAGCAG GACAAGAAGG AATTAGAGGA CAAGGAGCTG GTCAGGGAGG
ATATGGAGGT
 
1561 TTGGGATCTC AAGGTTCAGG TAGGGGTGGA CTTGGTGGAC AAGGAGCAGG
AGCAGCTGCT
1621 GCTGCTGCTG GTGGAGCTGG ACAAGGAGGA TTGGGAGGAC AAGGAGCTGG
TCAAGGAGCT
1681 GGAGCTGCTG CCGCAGCTGC TGGAGGAGTG AGACAAGGTG GGTATGGTGG
TTTGGGTTCA
1741 CAAGGAGCAG GTAGAGGTGG ACAAGGAGCA GGTGCAGCAG CAGCAGCTGC
TGGTGGTGCT
1801 GGTCAAGGAG GTTACGGTGG ACTTGGAGGT CAAGGAGTTG GTAGGGGAGG
TCTTGGTGGT
1861 CAAGGGGCTG GTGCTGCTGC CGCTGGTGGA GCTGGACAAG GTGGTTATGG
TGGTGTTGGT
1921 TCTGGAGCTT CTGCTGCTAG TGCTGCTGCA TCAAGACTTT CTTCTCCTCA
AGCTTCTTCT
1981 AGAGTTAGCA GCGCTGTTAG TAACCTTGTA GCTTCAGGTC CAACTAATTC
TGCTGCTCTT
2041 TCTAGTACTA TTTCTAATGT TGTGAGCCAA ATCGGAGCTT CAAATCCAGG
ATTGTCTGGT
2101 TGTGATGTTT TAATTCAAGC TTTGTTGGAG GTGGTTTCTG CTCTTATTCA
GATTTTGGGT
2161 TCTTCTTCTA TCGGTCAAGT TAACTATGGA TCTGCTGGTC AGGCTACACA
AATTGTTGGT
2221 CAGTCTGTTT ACCAGGCTCT TGGGTGA
**SEQ NO 7: Amino Acid Sequence of Fibroin-III** 
10         20         30         40         50         60
ARAGSGQQGP GQQGPGQQGP GQQGPYGPGA SAAAAAAGGY GPGSGQQGPS QQGPGQQGPG

70         80         90         100        110        120
GQGPYGPGAS AAAAAAGGYG PGSGQQGPGG QGPYGPGSSA AAAAAGGNGP GSGQQGAGQQ

130        140        150        160        170        180
GPGQQGPGAS AAAAAAGGYG PGSGQQGPGQ QGPGGQGPYG PGASAAAAAA GGYGPGSGQG

190        200        210        220        230        240
PGQQGPGGQG PYGPGASAAA AAAGGYGPGS GQQGPGQQGP GQQGPGGQGP YGPGASAAAA

250        260        270        280        290        300
AAGGYGPGYG QQGPGQQGPG GQGPYGPGAS AASAASGGYG PGSGQQGPGQ QGPGGQGPYG

310        320        330        340        350        360
PGASAAAAAA GGYGPGSGQQ GPGQQGPGQQ GPGQQGPGGQ GPYGPGASAA AAAGGYGPG

370        380        390        400        410        420
SGQQGPGQQG PGQQGPGQQG PGQQGPGQQG PGQQGPGQQG PGQQGPGGQG AYGPGASAAA

430        440        450        460        470        480
GAAGGYGPGS GQQGPGQQGP GQQGPGQQGP GQQGPGQQGP GQQGPGQQGP YGPGASAAAA

490        500        510        520        530        540
AAGGYGPGSG QQGPGQQGPG QQGPGGQGPY GPGAASAAVS VGGYGPQSSS VPVASAVASR

550        560        570        580        590        600
LSSPAASSRV SSAVSSLVSS GPTKHAALSN TISSVVSQVS ASNPGLSGCD VLVQALLEVV

610        620        630
SALVSILGSS SIGQINYGAS AQYTQMVGQS VAQALA
**SEQ NO 8: Nucleotide Sequence of Fibroin-IIII, Codon Optimized for Nicotiana Benthamiana Chloroplast Expression** 
1 GCAAGAGCAG GATCTGGTCA GCAAGGACCT GGTCAACAAG GTCCAGGACA
GCAGGGACCA
61 GGACAACAAG GTCCTTATGG ACCTGGTGCT TCAGCAGCTG CTGCTGCTGC
TGGTGGTTAT
121 GGACCAGGAT CTGGACAACA GGGCCCTTCT CAGCAAGGAC CTGGACAGCA
GGGTCCTGGT
181 GGTCAAGGAC CTTACGGTCC TGGAGCTTCT GCTGCTGCTG CTGCTGCAGG
TGGATATGGT
241 CCTGGTAGTG GTCAACAAGG TCCAGGAGGA CAAGGACCAT ACGGCCCTGG
TAGCTCAGCT
301 GCTGCAGCAG CTGCTGGTGG TAATGGCCCA GGAAGTGGAC AACAAGGTGC
TGGACAACAG
361 GGTCCAGGAC AACAAGGACC TGGTGCATCA GCCGCTGCTG CTGCTGCTGG
AGGTTATGGA
421 CCAGGTTCTG GACAACAAGG TCCAGGACAA CAAGGACCAG GAGGTCAAGG
ACCTTATGGA
481 CCAGGAGCTA GTGCTGCAGC AGCTGCTGCT GGAGGTTATG GTCCTGGAAG
TGGTCAAGGA
541 CCTGGACAAC AAGGACCTGG GGGTCAAGGT CCTTATGGAC CTGGAGCTTC
CGCTGCTGCT
601 GCAGCAGCCG GAGGTTATGG TCCAGGAAGT GGTCAGCAAG GACCAGGACA
GCAGGGACCA
661 GGACAGCAAG GTCCTGGAGG TCAGGGACCA TATGGTCCAG GAGCTTCTGC
TGCTGCTGCA
721 GCTGCTGGTG GATATGGACC AGGTTATGGA CAACAAGGAC CTGGACAACA
AGGACCTGGA
781 GGGCAAGGTC CATATGGACC AGGAGCTTCT GCTGCTAGTG CTGCTTCAGG
AGGGTATGGA
841 CCAGGTTCTG GACAACAAGG ACCAGGACAA CAAGGTCCAG GTGGTCAAGG
ACCTTATGGA
901 CCGGGTGCTT CTGCAGCTGC CGCAGCTGCT GGAGGATATG GTCCTGGTTC
AGGTCAACAA
961 GGACCCGGTC AACAAGGACC AGGTCAGCAA GGTCCAGGAC AACAGGGTCC
TGGAGGTCAG
1021 GGTCCTTATG GGCCAGGAGC ATCTGCTGCA GCTGCTGCTG CTGGTGGTTA
TGGACCAGGC
1081 TCTGGACAAC AAGGACCTGG TCAACAAGGA CCTGGACAGC AGGGTCCTGG
ACAGCAAGGT
1141 CCAGGACAAC AAGGACCAGG ACAGCAAGGT CCAGGTCAGC AAGGACCTGG
ACAACAAGGA
1201 CCAGGTCAAC AAGGACCTGG TGGTCAGGGT GCTTATGGTC CAGGTGCTAG
TGCTGCTGCC
1261 GGAGCAGCTG GAGGCTATGG ACCTGGATCT GGTCAGCAGG GACCTGGTCA
ACAAGGACCT
1321 GGTCAACAGG GACCAGGTCA ACAAGGACCA GGGCAACAGG GACCTGGACA
ACAAGGACCT
1381 GGACAACAAG GTCCAGGTCA GCAGGGACCT TATGGACCTG GTGCTTCAGC
TGCTGCTGCA
1441 GCTGCTGGAG GATATGGCCC AGGTTCTGGA CAACAGGGAC CTGGACAACA
AGGTCCAGGT
1501 CAACAAGGTC CAGGTGGCCA AGGACCATAC GGACCAGGAG CTGCATCTGC
AGCTGTTTCT
 
1561 GTTGGAGGAT ATGGACCTCA ATCATCATCT GTTCCTGTGG CATCAGCTGT
TGCTTCAAGA
1621 TTGTCTTCAC CAGCTGCTTC ATCTAGAGTT AGCAGTGCAG TTTCTTCCTT
GGTTTCTTCT
1681 GGACCTACTA AACATGCTGC TCTCTCTAAT ACTATTAGTA GTGTTGTTTC
TCAGGTTTCT
1741 GCTTCTAATC CAGGTTTATC AGGATGCGAT GTTCTTGTTC AAGCACTTTT
GGAAGTTGTT
1801 TCCGCTTTGG TTTCAATTTT AGGATCATCT TCTATTGGTC AAATTAATTA
TGGCGCTTCT
1861 GCCCAATATA CTCAGATGGT CGGACAATCA GTTGCTCAAG CTCTTGCTTA A
**SEQ NO 9: Amino Acid Sequence of P4H Alpha Subunit** 
10 20 30 40 50 60
MDISNLPPHI RQQILGLISK PQQNNDESSS SNNKNNLINN EKVSNVLIDL TSNLKIENFK

70 80 90 100 110 120
IFNKESLNQL EKKGYLIIDN FLNDLNKINL IYDESYNQFK ENKLIEAGMN KGTDKWKDKS

130 140 150 160 170 180
IRGDYIQWIH RDSNSRIQDK DLSSTIRNIN YLLDKLDLIK NEFDNVIPNF NSIKTQTQLA

190 200 210 220 230 240
VYLNGGRYIK HRDSFYSSES LTISRRITMI YYVNKDWKKG DGGELRLYTN NPNNTNQKEL

250 260 270 280
KQTEEFIDIE PIADRLLIFL SPFLEHEVLQ CNFEPRIAIT TWIY
**SEQ NO 10: Amino Acid Sequence of P4H Beta Subunit** 
10 20 30 40 50 60
APDEEDHVLV LHKGNFDEAL AAHKYLLVEF YAPWCGHCKA LAPEYAKAAG KLKAEGSEIR

70 80 90 100 110 120
LAKVDATEES DLAQQYGVRG YPTIKFFKNG DTASPKEYTA GREADDIVNW LKKRTGPAAS

130 140 150 160 170 180
TLSDGAAAEA LVESSEVAVI GFFKDMESDS AKQFFLAAEV IDDIPFGITS NSDVFSKYQL

190 200 210 220 230 240
DKDGVVLFKK FDEGRNNFEG EVTKEKLLDF IKHNQLPLVI EFTEQTAPKI FGGEIKTHIL

250 260 270 280 290 300
LFLPKSVSDY EGKLSNFKKA AESFKGKILF IFIDSDHTDN QRILEFFGLK KEECPAVRLI

310 320 330 340 350 360
TLEEEMTKYK PESDELTAEK ITEFCHRFLE GKIKPHLMSQ ELPDDWDKQP VKVLVGKNFE

370 380 390 400 410 420
EVAFDEKKNV FVEFYAPWCG HCKQLAPIWD KLGETYKDHE NIVIAKMDST ANEVEAVKVH

430 440 450 460 470 480
SFPTLKFFPA SADRTVIDYN GERTLDGFKK FLESGGQDGA GDDDDLEDLE EAEEPDLEED

490
DDQKAVKDEL
**SEQ NO 11: Nucleotide Sequence of P4H Alpha Subunit, Codon Optimized for Nicotiana Benthamiana Chloroplast Expression** 
1 ATGGATATTT CTAATTTGCC ACCACATATC AGGCAGCAAA TTCTTGGCTT
GATTTCTAAG
61 CCACAACAAA ATAACGATGA GAGTTCTTCA TCAAACAATA AAAATAACCT
TATTAACAAC
121 GAGAAGGTGT CAAATGTTTT GATTGATCTT ACTTCCAATC TTAAGATTGA
GAACTTCAAA
181 ATCTTTAACA AGGAGTCTCT CAATCAGCTT GAAAAGAAGG GATACCTTAT
TATTGATAAT
241 TTCTTGAATG ATTTGAATAA AATTAATTTA ATTTATGATG AATCCTATAA
TCAATTTAAA
301 GAAAATAAAC TTATAGAAGC TGGAATGAAC AAGGGAACTG ATAAGTGGAA
AGATAAGTCA
361 ATCAGAGGAG ATTACATACA GTGGATTCAT CGGGATTCTA ATAGTAGAAT
TCAGGATAAG
421 GATCTTTCAT CTACGATTAG AAATATTAAT TATCTTCTCG ATAAACTTGA
CTTGATTAAA
481 AATGAGTTTG ATAATGTCAT TCCAAATTTT AATTCAATCA AAACACAAAC
TCAATTAGCA
541 GTGTACTTGA ATGGCGGTAG GTACATTAAG CATAGAGATT CTTTCTATTC
TTCTGAAAGT
601 CTTACTATTT CTAGGAGGAT TACTATGATA TACTATGTGA ATAAGGATTG
GAAGAAGGGA
661 GATGGAGGTG AGTTGAGATT GTATACAAAC AATCCAAATA ATACAAATCA
AAAAGAGTTG
721 AAGCAGACTG AAGAATTCAT TGATATTGAA CCTATTGCTG ATCGTTTACT
TATTTTTCTT
781 TCCCCCTTCC TTGAACATGA GGTGCTTCAA TGCAATTTCG AGCCAAGGAT
AGCTATTACT
841 ACTTGGATAT ACTAA
**SEQ NO 12: Nucleotide Sequence of P4H Beta Subunit, Codon Optimized for Nicotiana Benthamiana Chloroplast Expression** 
1 GCTCCTGATG AAGAGGACCA TGTACTTGTT TTGCATAAGG GTAATTTTGA
TGAAGCTCTT
61 GCAGCACATA AATATCTATT GGTGGAGTTC TATGCACCTT GGTGTGGACA
CTGCAAGGCT
121 TTGGCTCCAG AGTACGCTAA GGCTGCAGGA AAACTTAAGG CTGAGGGTTC
TGAGATTAGA
181 CTCGCTAAGG TTGATGCTAC TGAGGAATCA GATTTGGCTC AGCAATATGG
AGTTAGGGGA
241 TACCCAACTA TTAAATTTTT CAAAAATGGG GATACTGCAT CACCAAAAGA
ATACACTGCC
301 GGGAGAGAAG CTGATGATAT TGTTAATTGG TTGAAGAAGA GGACTGGCCC
TGCTGCTTCT
361 ACTTTGTCTG ATGGAGCAGC TGCAGAGGCA CTTGTTGAAT CTTCAGAAGT
TGCTGTTATT
 
421 GGATTTTTCA AAGATATGGA GTCCGATTCT GCTAAGCAAT TCTTTCTGGC
AGCAGAAGTC
481 ATTGATGATA TTCCATTTGG AATCACTTCA AATTCTGATG TTTTTTCTAA
GTATCAGCTT
541 GATAAGGATG GGGTTGTTCT TTTTAAAAAA TTCGACGAGG GAAGGAATAA
CTTCGAGGGA
601 GAGGTGACAA AAGAGAAGCT TCTTGATTTC ATTAAGCATA ATCAGCTCCC
TCTTGTTATT
661 GAATTTACCG AACAGACTGC ACCTAAGATC TTCGGTGGAG AAATTAAAAC
TCATATTTTG
721 CTTTTTTTGC CAAAATCTGT TTCAGATTAT GAAGGAAAAC TTTCTAATTT
TAAGAAAGCT
781 GCTGAATCAT TTAAGGGTAA GATTTTGTTT ATTTTTATTG ACTCAGATCA
TACTGATAAT
841 CAAAGGATCT TGGAATTTTT TGGGTTAAAG AAGGAGGAAT GTCCTGCTGT
TAGACTTATT
901 ACTTTGGAGG AAGAAATGAC AAAGTACAAG CCTGAAAGTG ATGAATTGAC
AGCAGAAAAG
961 ATTACTGAAT TCTGTCATCG TTTCCTGGAA GGTAAGATTA AGCCACATTT
GATGTCCCAA
1021 GAACTTCCTG ATGATTGGGA TAAGCAACCT GTTAAGGTTC TCGTTGGTAA
GAATTTTGAA
1081 GAGGTGGCTT TTGATGAGAA AAAGAATGTC TTTGTTGAGT TCTATGCACC
TTGGTGTGGA
1141 CACTGTAAAC AATTGGCTCC AATTTGGGAC AAGCTCGGTG AAACCTATAA
GGATCACGAA
1201 AACATTGTTA TTGCTAAGAT GGATTCTACA GCTAATGAAG TGGAGGCTGT
TAAGGTGCAT
1261 TCTTTTCCAA CACTTAAATT TTTCCCAGCT TCAGCTGATA GGACTGTAAT
AGATTATAAC
1321 GGAGAGCGTA CTCTAGATGG TTTCAAAAAG TTTCTGGAGT CAGGTGGTCA
AGATGGAGCT
1381 GGAGATGATG ATGATCTTGA AGATCTCGAG GAAGCTGAGG AACCAGATCT
TGAGGAGGAT
1441 GATGATCAAA AAGCTGTTAA GGATGAGTTG TGA
**SEQ NO 13: Amino Acid Sequence of LH3** 
10 20 30 40 50 60
SDRPRGRDPV NPEKLLVITV ATAETEGYLR FLRSAEFFNY TVRTLGLGEE WRGGDVARTV

70 80 90 100 110 120
GGGQKVRWLK KEMEKYADRE DMIIMFVDSY DVILAGSPTE LLKKFVQSGS RLLFSAESFC

130 140 150 160 170 180
WPEWGLAEQY PEVGTGKRFL NSGGFIGFAT TIHQIVRQWK YKDDDDDQLF YTRLYLDPGL

190 200 210 220 230 240
REKLSLNLDH KSRIFQNLNG ALDEVVLKFD RNRVRIRNVA YDTLPIVVHG NGPTKLQLNY
 
250 260 270 280 290 300
LGNYVPNGWT PEGGCGFCNQ DRRTLPGGQP PPRVFLAVFV EQPTPFLPRF LQRLLLLDYP

310 320 330 340 350 360
PDRVTLFLHN NEVFHEPHIA DSWPQLQDHF SAVKLVGPEE ALSPGEARDM AMDLCRQDPE

370 380 390 400 410 420
CEFYFSLDAD AVLTNLQTLR ILIEENRKVI APMLSRHGKL WSNFWGALSP DEYYARSEDY

430 440 450 460 470 480
VELVQRKRVG VWNVPYISQA YVIRGDTLRM ELPQRDVFSG SDTDPDMAFC KSFRDKGIFL

490 500 510 520 530 540
HLSNQHEFGR LLATSRYDTE HLHPDLWQIF DNPVDWKEQY IHENYSRALE GEGIVEQPCP

550 560 570 580 590 600
DVYWFPLLSE QMCDELVAEM EHYGQWSGGR HEDSRLAGGY ENVPTVDIHM KQVGYEDQWL

610 620 630 640 650 660
QLLRTYVGPM TESLFPGYHT KARAVMNFVV RYRPDEQPSL RPHHDSSTFT LNVALNHKGL

670 680 690 700 710
DYEGGGCRFL RYDCVISSPR KGWALLHPGR LTHYHEGLPT TWGTRYIMVS FVDP
**SEQ NO 14: Nucleotide Sequence of LH3, Codon Optimized for Nicotiana Benthamiana Chloroplast Expression** 
1 TCTGATAGGC CACGTGGAAG AGATCCTGTG AACCCAGAGA AGCTTTTGGT
TATTACTGTT
61 GCTACTGCAG AGACTGAAGG ATACCTTAGA TTTCTTCGTT CAGCAGAGTT
CTTTAATTAT
121 ACTGTTAGAA CTTTGGGATT GGGTGAAGAA TGGAGAGGAG GAGATGTTGC
TAGGACTGTT
181 GGTGGTGGAC AAAAAGTTAG GTGGTTGAAG AAGGAAATGG AAAAGTATGC
TGATAGGGAG
241 GATATGATTA TTATGTTTGT GGATTCCTAT GACGTTATTC TTGCTGGATC
TCCTACCGAG
301 CTTCTTAAAA AGTTTGTTCA ATCAGGATCA AGACTTCTCT TTAGTGCAGA
GAGTTTCTGT
361 TGGCCAGAAT GGGGTCTCGC TGAACAATAC CCAGAAGTTG GAACTGGAAA
GAGATTCTTG
421 AATTCTGGAG GATTTATTGG ATTTGCTACA ACTATTCATC AAATTGTTAG
ACAGTGGAAG
481 TATAAGGATG ATGATGATGA TCAGCTTTTT TATACTAGGC TTTATTTAGA
TCCTGGTCTC
541 AGAGAAAAAC TTTCTTTGAA TCTTGATCAT AAGAGCAGAA TCTTCCAAAA
TCTTAACGGA
601 GCTTTGGATG AGGTTGTTCT TAAATTTGAT AGAAACAGAG TTAGGATTAG
AAATGTTGCT
661 TATGATACTT TGCCTATTGT TGTTCATGGA AATGGACCAA CTAAGCTTCA
ACTTAACTAT
721 CTTGGTAACT ACGTTCCTAA TGGATGGACT CCAGAAGGTG GTTGTGGATT
TTGCAACCAG
 
781 GATAGAAGGA CACTTCCTGG GGGACAACCC CCTCCAAGGG TGTTCTTGGC
TGTTTTTGTT
841 GAACAACCAA CTCCATTTTT GCCTCGTTTT TTGCAAAGAC TGCTACTTCT
TGATTATCCA
901 CCTGATAGAG TTACTTTGTT CTTGCATAAT AATGAAGTTT TTCATGAACC
ACATATTGCA
961 GATTCTTGGC CACAACTCCA AGATCACTTT AGCGCTGTTA AACTTGTTGG
TCCAGAAGAA
1021 GCTTTGTCTC CTGGTGAAGC AAGAGACATG GCCATGGATC TCTGCAGACA
AGACCCAGAA
1081 TGTGAATTTT ATTTTAGTTT GGATGCTGAT GCTGTTTTGA CTAATTTGCA
GACATTGAGA
1141 ATTCTTATCG AAGAAAACAG GAAAGTTATT GCTCCCATGC TTTCAAGGCA
TGGAAAGCTC
1201 TGGTCTAATT TCTGGGGTGC ACTTTCTCCA GACGAATACT ATGCAAGATC
TGAAGATTAT
1261 GTTGAGTTGG TTCAGAGAAA GAGAGTTGGA GTTTGGAACG TTCCCTATAT
TTCACAGGCT
1321 TACGTTATTA GAGGAGATAC CTTGCGTATG GAATTGCCAC AGAGGGATGT
TTTCTCTGGC
1381 TCTGATACAG ATCCAGATAT GGCTTTCTGC AAATCTTTTA GAGACAAAGG
TATTTTTTTG
1441 CATTTGTCAA ACCAACATGA ATTCGGTAGA CTTCTTGCTA CCTCTAGGTA
TGATACTGAA
1501 CACCTTCATC CAGATCTCTG GCAAATTTTT GACAATCCTG TTGACTGGAA
GGAGCAATAT
1561 ATTCACGAGA ACTACTCTAG AGCTCTTGAA GGAGAGGGTA TTGTTGAGCA
ACCTTGCCCT
1621 GATGTGTACT GGTTTCCTTT GCTCTCTGAG CAAATGTGTG ATGAACTTGT
TGCTGAAATG
1681 GAACATTACG GCCAATGGTC AGGTGGTAGG CATGAGGATT CAAGACTTGC
TGGTGGTTAC
1741 GAAAACGTTC CAACAGTGGA TATCCATATG AAGCAAGTTG GTTATGAAGA
TCAGTGGCTT
1801 CAATTGCTTA GGACTTATGT TGGACCTATG ACTGAATCAC TTTTTCCAGG
ATATCACACA
1861 AAGGCTAGAG CTGTGATGAA TTTTGTTGTT AGATATAGAC CAGATGAACA
GCCATCATTG
1921 AGACCACATC ATGATTCTTC TACTTTTACT TTAAATGTGG CTCTTAACCA
TAAGGGACTG
1981 GATTACGAAG GAGGAGGTTG CAGATTTCTT AGATATGATT GTGTTATTTC
CTCTCCAAGA
2041 AAAGGGTGGG CATTGTTACA TCCAGGTAGA CTTACACATT ACCATGAAGG
ACTTCCTACT
2101 ACTTGGGGAA CTCGTTATAT TATGGTTTCT TTCGTTGATC CTTAA
 
SEQ NO 15: Amino Acid Sequence SPID1COL1 comprising a chloroplast transit signal, and two
A2 cleavage sequences

10 20 30 40 50 60
MATTLISKLT LSSAFLGQQF SSRGNSMRSA PAGLFLRGPR QGAGAAAAAA GGAGQGGYGG

70 80 90 100 110 120
LGGQGAGQGG YGGLGGQGAG QGAGAAAAAA AGGAGQGGYG GLGSQGAGRG GQGAGAAAAA

130 140 150 160 170 180
AGGAGQGGYG GLGSQGAGRG GLGGQGAGAA AAAAAGGAGQ GGYGGLGNQG AGRGGQGAAA

190 200 210 220 230 240
AAAGGAGQGG YGGLGSQGAG RGGLGGQGAG AAAAAAGGAG QGGYGGLGGQ GAGQGGYGGL

250 260 270 280 290 300
GSQGAGRGGL GGQGAGAAAA AAAGGAGQGG LGGQGAGQGA GASAAAAGGA GQGGYGGLGS

310 320 330 340 350 360
QGAGRGGEGA GAAAAAAGGA GQGGYGGLGG QGAGQGGYGG LGSQGAGRGG LGGQGAGAAA

370 380 390 400 410 420
AGGAGQGGLG GQGAGQGAGA AAAAAGGAGQ GGYGGLGSQG AGRGGLGGQG AGAVAAAAAG

430 440 450 460 470 480
GAGQGGYGGL GSQGAGRGGQ GAGAAAAAAG GAGQRGYGGL GNQGAGRGGL GGQGAGAAAA

490 500 510 520 530 540
AAAGGAGQGG YGGLGNQGAG RGGQGAAAAA GGAGQGGYGG LGSQGAGRGG QGAGAAAAAA

550 560 570 580 590 600
VGAGQEGIRG QGAGQGGYGG LGSQGSGRGG LGGQGAGAAA AAAGGAGQGG LGGQGAGQGA

610 620 630 640 650 660
GAAAAAAGGV RQGGYGGLGS QGAGRGGQGA GAAAAAAGGA GQGGYGGLGG QGVGRGGLGG

670 680 690 700 710 720
QGAGAAAAGG AGQGGYGGVG SGASAASAAA SRLSSPQASS RVSSAVSNLV ASGPTNSAAL

730 740 750 760 770 780
SSTISNVVSQ IGASNPGLSG CDVLIQALLE VVSALIQILG SSSIGQVNYG SAGQATQIVG

790 800 810 820 830 840
QSVYQALGGS GEGRGSLLTC EDVEENPGPQ LSYGYDEKST GGISVPGPMG PSGPRGLPGP

850 860 870 880 890 900
PGAPGPQGFQ GPPGEPGEPG ASGPMGPRGP PGPPGKNGDD GEAGKPGRPG ERGPPGPQGA

910 920 930 940 950 960
RGLPGTAGLP GMKGHRGFSG LDGAKGDAGP AGPKGEPGSP GENGAPGQMG PRGLPGERGR
 
970 980 990 1000 1010 1020
PGAPGPAGAR GNDGATGAAG PPGPTGPAGP PGFPGAVGAK GEAGPQGPRG SEGPQGVRGE

1030 1040 1050 1060 1070 1080
PGPPGPAGAA GPAGNPGADG QPGAKGANGA PGIAGAPGFP GARGPSGPQG PGGPPGPKGN

1090 1100 1110 1120 1130 1140
SGEPGAPGSK GDTGAKGEPG PVGVQGPPGP AGEEGKRGAR GEPGPTGLPG PPGERGGPGS

1150 1160 1170 1180 1190 1200
RGFPGADGVA GPKGPAGERG SPGPAGPKGS PGEAGRPGEA GLPGAKGLTG SPGSPGPDGK

1210 1220 1230 1240 1250 1260
TGPPGPAGQD GRPGPPGPPG ARGQAGVMGF PGPKGAAGEP GKAGERGVPG PPGAVGPAGK

1270 1280 1290 1300 1310 1320
DGEAGAQGPP GPAGPAGERG EQGPAGSPGF QGLPGPAGPP GEAGKPGEQG VPGDLGAPGP

1330 1340 1350 1360 1370 1380
SGARGERGFP GERGVQGPPG PAGPRGANGA PGNDGAKGDA GAPGAPGSQG APGLQGMPGE

1390 1400 1410 1420 1430 1440
RGAAGLPGPK GDRGDAGPKG ADGSPGKDGV RGLTGPIGPP GPAGAPGDKG ESGPSGPAGP

1450 1460 1470 1480 1490 1500
TGARGAPGDR GEPGPPGPAG FAGPPGADGQ PGAKGEPGDA GAKGDAGPPG PAGPAGPPGP

1510 1520 1530 1540 1550 1560
IGNVGAPGAK GARGSAGPPG ATGFPGAAGR VGPPGPSGNA GPPGPPGPAG KEGGKGPRGE

1570 1580 1590 1600 1610 1620
TGPAGRPGEV GPPGPPGPAG EKGSPGADGP AGAPGTPGPQ GIAGQRGVVG LPGQRGERGF

1630 1640 1650 1660 1670 1680
PGLPGPSGEP GKQGPSGASG ERGPPGPMGP PGLAGPPGES GREGAPGAEG SPGRDGSPGA

1690 1700 1710 1720 1730 1740
KGDRGETGPA GPPGAPGAPG APGPVGPAGK SGDRGETGPA GPAGPVGPVG ARGPAGPQGP

1750 1760 1770 1780 1790 1800
RGDKGETGEQ GDRGIKGHRG FSGLQGPPGP PGSPGEQGPS GASGPAGPRG PPGSAGAPGK

1810 1820 1830 1840 1850 1860
DGLNGLPGPI GPPGPRGRTG DAGPVGPPGP PGPPGPPGPP SAGFDFSFLP QPPQEKAHDG

1870 1880 1890 1900 1910 1920
GRYYRAGSGE GRGSLLTCED VEENPGPQYD GKGVGLGPGP MGLMGPRGPP GAAGAPGPQG

1930 1940 1950 1960 1970 1980
FQGPAGEPGE PGQTGPAGAR GPAGPPGKAG EDGHPGKPGR PGERGVVGPQ GARGFPGTPG

1990 2000 2010 2020 2030 2040
LPGFKGIRGH NGLDGLKGQP GAPGVKGEPG APGENGTPGQ TGARGLPGER GRVGAPGPAG

2050 2060 2070 2080 2090 2100
ARGSDGSVGP VGPAGPIGSA GPPGFPGAPG PKGEIGAVGN AGPAGPAGPR GEVGLPGLSG

2110 2120 2130 2140 2150 2160
PVGPPGNPGA NGLTGAKGAA GLPGVAGAPG LPGPRGIPGP VGAAGATGAR GLVGEPGPAG
 
2170 2180 2190 2200 2210 2220
SKGESGNKGE PGSAGPQGPP GPSGEEGKRG PNGEAGSAGP PGPPGLRGSP GSRGLPGADG

2230 2240 2250 2260 2270 2280
RAGVMGPPGS RGASGPAGVR GPNGDAGRPG EPGLMGPRGL PGSPGNIGPA GKEGPVGLPG

2290 2300 2310 2320 2330 2340
IDGRPGPIGP AGARGEPGNI GFPGPKGPTG DPGKNGDKGH AGLAGARGAP GPDGNNGAQG

2350 2360 2370 2380 2390 2400
PPGPQGVQGG KGEQGPPGPP GFQGLPGPSG PAGEVGKPGE RGLHGEFGLP GPAGPRGERG

2410 2420 2430 2440 2450 2460
PPGESGAAGP TGPIGSRGPS GPPGPDGNKG EPGVVGAVGT AGPSGPSGLP GERGAAGIPG

2470 2480 2490 2500 2510 2520
GKGEKGEPGL RGEIGNPGRD GARGAPGAVG APGPAGATGD RGEAGAAGPA GPAGPRGSPG

2530 2540 2550 2560 2570 2580
ERGEVGPAGP NGFAGPAGAA GQPGAKGERG AKGPKGENGV VGPTGPVGAA GPAGPNGPPG

2590 2600 2610 2620 2630 2640
PAGSRGDGGP PGMTGFPGAA GRTGPPGPSG ISGPPGPPGP AGKEGLRGPR GDQGPVGRTG

2650 2660 2670 2680 2690 2700
EVGAVGPPGF AGEKGPSGEA GTAGPPGTPG PQGLLGAPGI LGLPGSRGER GLPGVAGAVG

2710 2720 2730 2740 2750 2760
EPGPLGIAGP PGARGPPGAV GSPGVNGAPG EAGRDGNPGN DGPPGRDGQP GHKGERGYPG

2770 2780 2790 2800 2810 2820
NIGPVGAAGA PGPHGPVGPA GKHGNRGETG PSGPVGPAGA VGPRGPSGPQ GIRGDKGEPG

2830 2840 2850 2860 2870 2880
EKGPRGLPGL KGHNGLQGLP GIAGHHGDQG APGSVGPAGP RGPAGPSGPA GKDGRTGHPG

2890 2900 2910 2920
TVGPAGIRGP QGHQGPAGPP GPPGPPGPPG VSGGGYDFGY DGDFYRA
**SEQ NO 16: Nucleotide Sequence of SPIDCOL1, Codon Optimized for Nicotiana Benthamiana Chloroplast Expression** 
1 ATGGCTACCA CTCTTATATC TAAGTTGACT CTTTCATCAG CTTTCCTTGG
ACAACAGTTT
61 TCATCTAGAG GTAATTCAAT GAGATCAGCA CCAGCTGGAC TTTTTCTTAG
GGGACCAAGA
121 CAAGGAGCAG GTGCTGCTGC AGCAGCAGCT GGAGGTGCTG GACAAGGTGG
TTATGGAGGA
181 CTTGGAGGAC AAGGAGCAGG ACAGGGTGGA TATGGTGGAT TGGGAGGACA
AGGTGCTGGA
241 CAAGGAGCTG GAGCTGCTGC TGCTGCAGCA GCTGGAGGTG CTGGTCAGGG
TGGATATGGA
301 GGATTGGGAA GCCAAGGAGC TGGTAGGGGT GGTCAAGGAG CTGGTGCTGC
TGCTGCAGCA
361 GCTGGTGGAG CAGGTCAGGG CGGTTATGGA GGCTTGGGTT CTCAAGGAGC
TGGAAGGGGT
421 GGCTTGGGTG GCCAAGGTGC CGGTGCTGCT GCTGCTGCTG CTGCTGGTGG
TGCTGGTCAA
481 GGCGGATATG GAGGACTTGG AAACCAAGGT GCTGGCCGTG GTGGACAGGG
AGCTGCTGCT
541 GCTGCTGCAG GAGGAGCTGG TCAGGGTGGG TATGGTGGTT TGGGTTCACA
GGGAGCTGGA
601 AGGGGTGGAC TTGGAGGACA GGGTGCAGGA GCAGCTGCTG CTGCAGCTGG
TGGTGCAGGT
661 CAAGGTGGAT ACGGTGGTCT TGGTGGACAA GGAGCTGGTC AGGGTGGCTA
CGGTGGACTT
721 GGAAGTCAAG GAGCTGGAAG AGGTGGTCTT GGAGGTCAAG GAGCCGGTGC
TGCTGCTGCA
781 GCTGCAGCTG GTGGAGCTGG ACAAGGCGGT CTGGGTGGCC AAGGTGCTGG
ACAGGGAGCA
841 GGTGCATCTG CAGCTGCAGC TGGTGGAGCT GGTCAAGGTG GCTATGGTGG
ATTGGGTTCT
901 CAGGGAGCTG GTAGAGGTGG AGAAGGAGCT GGAGCTGCTG CAGCTGCTGC
TGGAGGAGCA
961 GGTCAGGGTG GTTACGGAGG TTTAGGAGGT CAAGGAGCCG GACAAGGAGG
ATATGGAGGT
1021 CTTGGTTCTC AAGGGGCAGG GAGAGGAGGT TTAGGTGGAC AGGGAGCTGG
TGCTGCAGCT
1081 GCTGGAGGAG CTGGTCAGGG AGGACTTGGA GGACAAGGTG CAGGTCAAGG
TGCTGGTGCA
1141 GCTGCTGCTG CCGCTGGAGG TGCTGGACAG GGAGGGTATG GAGGCCTTGG
TAGCCAGGGT
1201 GCAGGCAGGG GAGGTTTGGG AGGACAGGGT GCTGGTGCTG TGGCAGCAGC
TGCCGCAGGA
1261 GGTGCTGGAC AAGGAGGATA TGGAGGACTT GGATCTCAAG GTGCTGGTAG
AGGTGGTCAA
1321 GGAGCTGGAG CTGCTGCTGC TGCAGCTGGA GGAGCCGGTC AAAGAGGATA
CGGTGGACTA
1381 GGTAATCAAG GAGCTGGAAG GGGAGGATTG GGTGGTCAGG GAGCTGGAGC
AGCAGCTGCA
1441 GCAGCTGCTG GAGGAGCAGG TCAGGGGGGT TATGGAGGAT TGGGGAATCA
AGGTGCAGGA
1501 AGAGGTGGAC AAGGGGCTGC TGCAGCTGCT GGTGGAGCTG GCCAAGGAGG
TTACGGTGGA
1561 CTTGGTTCTC AGGGAGCAGG AAGAGGAGGG CAGGGAGCTG GAGCTGCAGC
TGCTGCTGCT
1621 GTTGGTGCTG GTCAGGAAGG TATTAGAGGA CAGGGAGCTG GTCAAGGAGG
TTACGGAGGT
 
1681 TTAGGGTCCC AGGGTTCTGG AAGAGGAGGA CTGGGAGGAC AAGGAGCAGG
TGCTGCTGCT
1741 GCTGCAGCTG GTGGTGCTGG ACAAGGAGGT CTTGGAGGAC AAGGAGCTGG
ACAGGGAGCT
1801 GGTGCAGCTG CTGCTGCTGC TGGAGGAGTT AGACAGGGAG GATATGGAGG
TTTGGGATCA
1861 CAAGGTGCAG GAAGAGGTGG ACAGGGAGCT GGAGCTGCAG CTGCTGCGGC
TGGTGGGGCT
1921 GGACAAGGTG GATATGGAGG GCTTGGAGGC CAAGGAGTTG GAAGGGGTGG
GCTTGGTGGA
1981 CAAGGTGCAG GTGCTGCTGC TGCTGGAGGT GCTGGTCAAG GCGGTTACGG
AGGTGTTGGT
2041 TCTGGAGCTT CAGCTGCAAG TGCTGCAGCT AGTAGGCTTT CTAGTCCACA
AGCATCATCT
2101 AGAGTTTCTT CTGCTGTTTC TAATTTGGTG GCATCTGGTC CAACAAACTC
GGCAGCACTT
2161 TCTTCTACTA TTTCTAATGT TGTTTCTCAG ATAGGTGCAT CTAACCCAGG
TCTTTCAGGA
2221 TGTGATGTTT TGATACAGGC TTTGCTTGAA GTGGTTAGTG CTCTTATACA
AATTCTCGGA
2281 TCCTCATCAA TTGGTCAAGT GAACTACGGT TCTGCTGGAC AAGCTACACA
GATTGTTGGT
2341 CAATCAGTTT ATCAAGCACT TGGGGGTTCT GGTGAAGGAA GGGGTAGTCT
TCTTACTTGT
2401 GAAGATGTGG AAGAAAATCC TGGACCACAG CTTTCTTATG GATACGATGA
AAAGTCTACT
2461 GGAGGTATAT CTGTTCCTGG TCCTATGGGA CCTAGTGGTC CTAGAGGTTT
GCCAGGACCT
2521 CCTGGTGCTC CAGGACCACA AGGATTTCAG GGACCACCAG GGGAACCAGG
TGAACCTGGA
2581 GCTTCTGGAC CAATGGGTCC TAGAGGTCCA CCTGGACCTC CTGGTAAAAA
TGGAGATGAT
2641 GGTGAGGCTG GAAAGCCAGG AAGGCCAGGA GAAAGAGGTC CACCAGGACC
ACAGGGTGCT
2701 CGTGGTCTTC CAGGAACAGC CGGTTTACCT GGCATGAAGG GACATAGAGG
ATTTTCAGGT
2761 TTGGATGGAG CTAAAGGAGA TGCTGGACCA GCTGGACCTA AAGGAGAGCC
AGGATCTCCT
2821 GGAGAGAATG GTGCACCTGG CCAGATGGGT CCAAGGGGTC TTCCAGGTGA
GAGAGGTAGA
2881 CCTGGAGCCC CAGGTCCAGC AGGCGCTAGA GGGAATGACG GAGCCACAGG
TGCAGCTGGT
2941 CCACCTGGAC CTACTGGTCC TGCTGGGCCT CCTGGCTTTC CTGGAGCTGT
AGGTGCTAAG
3001 GGTGAGGCTG GACCTCAAGG TCCTCGAGGA TCAGAAGGTC CACAAGGAGT
TAGGGGAGAG
3061 CCTGGCCCAC CAGGTCCAGC TGGAGCTGCA GGTCCTGCTG GTAATCCAGG
AGCTGATGGA
3121 CAACCTGGAG CTAAAGGTGC TAACGGAGCT CCTGGAATTG CTGGAGCACC
AGGTTTTCCT
3181 GGTGCTAGAG GACCATCAGG ACCACAAGGA CCAGGTGGTC CTCCAGGACC
TAAAGGTAAT
3241 AGTGGAGAAC CAGGTGCTCC TGGTTCTAAA GGAGATACTG GTGCTAAGGG
AGAACCAGGC
3301 CCTGTTGGAG TCCAAGGTCC ACCTGGACCA GCTGGAGAAG AAGGAAAGAG
GGGAGCTAGA
3361 GGCGAACCAG GACCTACTGG ATTGCCAGGT CCTCCAGGTG AAAGAGGAGG
TCCAGGTTCT
 
3421 AGGGGTTTCC CAGGAGCAGA TGGAGTAGCT GGACCTAAGG GACCCGCTGG
TGAAAGAGGT
3481 TCACCTGGAC CTGCAGGTCC TAAGGGTTCA CCAGGTGAAG CAGGTAGACC
TGGTGAAGCA
3541 GGTTTGCCTG GAGCTAAGGG TTTGACAGGA AGTCCAGGGT CACCTGGACC
AGATGGAAAG
3601 ACAGGACCTC CTGGTCCAGC TGGTCAAGAT GGAAGACCTG GTCCTCCAGG
ACCACCAGGT
3661 GCAAGAGGAC AAGCTGGAGT TATGGGTTTT CCTGGTCCAA AGGGAGCTGC
TGGAGAGCCA
3721 GGTAAAGCTG GTGAAAGAGG TGTTCCAGGT CCTCCTGGTG CTGTTGGACC
AGCTGGTAAA
3781 GATGGAGAAG CTGGAGCTCA AGGTCCACCT GGTCCTGCAG GACCAGCTGG
AGAAAGAGGC
3841 GAACAAGGTC CTGCTGGTTC GCCAGGATTT CAGGGTTTAC CAGGTCCCGC
TGGTCCTCCA
3901 GGTGAAGCTG GAAAACCTGG AGAACAAGGT GTGCCTGGAG ATTTGGGAGC
TCCAGGACCT
3961 TCTGGTGCAA GAGGTGAGCG TGGTTTCCCT GGAGAAAGGG GTGTTCAAGG
TCCACCTGGA
4021 CCTGCTGGTC CTAGAGGAGC TAACGGAGCT CCAGGAAATG ATGGTGCAAA
GGGTGATGCT
4081 GGTGCACCTG GTGCTCCTGG ATCTCAAGGT GCTCCAGGTC TTCAGGGTAT
GCCAGGAGAG
4141 AGGGGAGCTG CTGGATTACC TGGGCCTAAA GGTGATAGAG GAGATGCTGG
TCCAAAGGGT
4201 GCTGATGGTA GTCCAGGTAA AGATGGTGTT AGAGGACTTA CAGGCCCTAT
TGGTCCACCT
4261 GGGCCAGCTG GTGCACCAGG TGATAAGGGA GAAAGTGGAC CAAGTGGACC
AGCAGGACCA
4321 ACCGGTGCTA GAGGAGCACC AGGTGATAGA GGAGAACCAG GTCCACCAGG
ACCAGCTGGT
4381 TTTGCTGGTC CTCCAGGAGC TGATGGACAA CCAGGAGCTA AAGGTGAGCC
TGGAGATGCT
4441 GGAGCTAAAG GAGATGCTGG TCCACCGGGA CCAGCAGGTC CAGCAGGCCC
ACCAGGTCCA
4501 ATTGGAAACG TTGGTGCACC TGGCGCTAAG GGTGCCAGAG GAAGCGCAGG
TCCACCAGGA
4561 GCAACTGGCT TTCCAGGTGC TGCAGGTAGA GTTGGACCAC CAGGACCTTC
TGGAAACGCT
4621 GGACCTCCTG GGCCTCCAGG ACCTGCTGGA AAGGAAGGAG GGAAGGGTCC
TAGGGGAGAG
4681 ACTGGACCAG CTGGTAGACC AGGTGAGGTT GGACCACCAG GTCCTCCAGG
CCCAGCTGGT
4741 GAAAAGGGTA GTCCCGGTGC TGATGGACCA GCAGGAGCTC CAGGAACACC
AGGACCTCAA
4801 GGTATTGCTG GTCAAAGAGG TGTTGTTGGT TTGCCTGGTC AGAGAGGAGA
AAGAGGATTT
4861 CCAGGATTGC CAGGACCTTC TGGTGAGCCT GGTAAACAGG GTCCATCAGG
TGCTTCTGGG
4921 GAAAGAGGAC CACCTGGTCC TATGGGACCA CCAGGTTTGG CTGGTCCTCC
TGGTGAATCA
4981 GGTAGGGAAG GAGCTCCCGG AGCTGAAGGA TCACCAGGAA GAGATGGATC
TCCTGGAGCT
5041 AAAGGAGATA GAGGAGAAAC AGGTCCAGCT GGACCACCAG GAGCACCTGG
TGCTCCTGGT
5101 GCTCCTGGAC CTGTTGGTCC AGCTGGTAAA TCAGGAGATA GAGGTGAAAC
TGGACCTGCT
 
5161 GGACCAGCTG GTCCAGTTGG ACCTGTTGGT GCTAGAGGGC CAGCAGGACC
ACAGGGTCCT
5221 AGAGGAGATA AGGGAGAGAC TGGTGAACAA GGAGATAGAG GAATCAAAGG
TCATAGAGGA
5281 TTTAGTGGAC TTCAAGGACC ACCTGGCCCT CCTGGTTCTC CTGGAGAACA
AGGCCCATCT
5341 GGTGCTTCTG GACCTGCTGG CCCAAGGGGA CCACCTGGAT CTGCTGGTGC
CCCTGGTAAA
5401 GATGGACTTA ATGGATTGCC AGGTCCAATT GGTCCTCCTG GTCCAAGAGG
AAGGACAGGA
5461 GATGCTGGAC CTGTTGGTCC TCCTGGCCCA CCAGGACCAC CTGGACCTCC
TGGACCTCCA
5521 AGTGCTGGTT TTGATTTCTC TTTTTTACCA CAGCCACCAC AGGAAAAAGC
TCATGATGGT
5581 GGAAGATACT ATAGAGCTGG TTCAGGTGAG GGTAGAGGAT CCTTACTTAC
ATGTGAAGAT
5641 GTTGAGGAAA ATCCTGGACC ACAGTACGAT GGAAAAGGTG TTGGACTTGG
TCCAGGTCCA
5701 ATGGGATTGA TGGGACCAAG AGGACCTCCA GGAGCTGCAG GAGCTCCAGG
ACCACAGGGA
5761 TTTCAAGGTC CTGCAGGAGA GCCTGGAGAG CCAGGTCAAA CTGGACCTGC
AGGAGCTAGA
5821 GGTCCAGCTG GACCTCCAGG TAAAGCTGGA GAAGATGGTC ATCCAGGAAA
GCCAGGGAGG
5881 CCAGGTGAAA GGGGTGTTGT TGGTCCACAG GGGGCTAGAG GCTTCCCTGG
TACACCTGGT
5941 CTTCCAGGAT TTAAAGGTAT TAGAGGTCAT AATGGTTTAG ATGGATTGAA
GGGTCAACCA
6001 GGAGCTCCAG GTGTTAAGGG GGAACCAGGA GCACCAGGTG AAAATGGAAC
TCCTGGTCAG
6061 ACTGGAGCTA GAGGACTTCC AGGAGAAAGA GGTAGAGTGG GTGCACCTGG
TCCAGCAGGG
6121 GCTCGTGGTA GTGATGGTTC CGTTGGACCC GTCGGACCTG CAGGTCCAAT
TGGATCAGCA
6181 GGACCACCTG GATTCCCAGG AGCTCCAGGT CCAAAAGGCG AGATTGGTGC
TGTTGGAAAT
6241 GCTGGGCCTG CTGGACCTGC TGGTCCTAGA GGAGAGGTTG GACTTCCAGG
TTTGTCCGGA
6301 CCAGTGGGAC CACCTGGAAA TCCAGGAGCT AATGGTCTTA CTGGAGCTAA
AGGAGCTGCA
6361 GGGTTGCCTG GTGTTGCTGG AGCTCCAGGA CTTCCTGGAC CTAGAGGAAT
TCCTGGTCCA
6421 GTTGGAGCTG CTGGTGCTAC TGGTGCTAGA GGACTTGTTG GAGAACCAGG
TCCAGCAGGA
6481 TCTAAGGGAG AGTCAGGTAA TAAAGGTGAG CCAGGAAGTG CTGGTCCACA
AGGTCCACCA
6541 GGACCTTCTG GTGAGGAGGG TAAGAGGGGT CCAAATGGTG AAGCTGGATC
AGCTGGACCT
6601 CCAGGACCAC CTGGACTTAG GGGTAGCCCT GGTTCAAGAG GACTGCCTGG
GGCTGATGGA
6661 AGAGCTGGAG TTATGGGACC TCCCGGTAGT AGGGGAGCAT CCGGACCAGC
TGGAGTAAGG
6721 GGACCTAATG GTGATGCTGG AAGACCAGGA GAACCTGGAT TAATGGGTCC
TAGGGGTCTC
6781 CCAGGATCTC CAGGTAACAT TGGTCCTGCT GGTAAAGAAG GACCAGTTGG
TCTTCCAGGC
6841 ATTGATGGTA GACCAGGACC AATTGGGCCA GCTGGTGCTC GTGGCGAACC
TGGTAATATA
 
6901 GGATTCCCAG GTCCTAAGGG ACCAACCGGT GATCCAGGTA AAAATGGTGA
TAAAGGTCAT
6961 GCTGGATTGG CCGGAGCTAG GGGAGCTCCA GGTCCAGATG GAAATAATGG
TGCTCAGGGA
7021 CCACCAGGAC CACAGGGTGT TCAAGGTGGA AAAGGAGAAC AGGGTCCTCC
TGGTCCTCCA
7081 GGTTTCCAAG GACTTCCTGG ACCTTCTGGT CCAGCAGGTG AGGTTGGTAA
ACCAGGAGAG
7141 AGAGGATTGC ACGGAGAATT TGGTTTGCCA GGACCGGCTG GTCCTAGGGG
TGAAAGAGGA
7201 CCACCTGGTG AATCTGGAGC TGCTGGACCA ACTGGTCCTA TTGGTTCAAG
GGGACCTTCT
7261 GGACCTCCAG GTCCAGATGG AAATAAAGGA GAGCCAGGAG TGGTTGGAGC
TGTTGGAACA
7321 GCTGGACCAA GTGGACCTTC AGGACTCCCA GGAGAGAGGG GCGCTGCTGG
TATTCCTGGT
7381 GGAAAAGGTG AGAAGGGTGA GCCTGGACTT AGAGGAGAAA TAGGAAATCC
AGGCAGGGAT
7441 GGTGCACGGG GAGCTCCTGG AGCTGTTGGT GCCCCAGGAC CAGCCGGAGC
AACAGGAGAT
7501 AGGGGAGAGG CTGGTGCTGC TGGTCCAGCT GGACCTGCAG GACCTAGGGG
TTCACCAGGA
7561 GAAAGAGGTG AGGTTGGTCC AGCTGGTCCT AATGGATTTG CTGGTCCTGC
TGGTGCTGCT
7621 GGTCAACCTG GAGCTAAGGG TGAGAGGGGT GCAAAAGGAC CTAAAGGTGA
AAATGGTGTT
7681 GTTGGTCCTA CTGGACCAGT TGGAGCTGCT GGACCTGCTG GACCAAATGG
TCCACCTGGT
7741 CCAGCTGGTT CTAGAGGAGA TGGTGGCCCA CCTGGAATGA CTGGATTCCC
AGGTGCTGCT
7801 GGAAGGACTG GACCACCAGG CCCTAGTGGA ATTTCTGGAC CACCTGGTCC
TCCTGGACCA
7861 GCAGGTAAGG AAGGTTTGAG GGGACCAAGA GGGGATCAGG GACCTGTAGG
TAGAACTGGT
7921 GAGGTTGGTG CTGTTGGCCC ACCAGGTTTC GCTGGCGAAA AGGGACCTTC
AGGTGAAGCT
7981 GGTACAGCTG GTCCTCCTGG TACTCCTGGT CCACAAGGTT TGCTTGGTGC
TCCTGGTATT
8041 CTTGGTCTTC CAGGTTCAAG AGGTGAGAGA GGTCTTCCTG GAGTGGCTGG
AGCTGTTGGA
8101 GAACCAGGTC CATTGGGTAT AGCTGGACCT CCAGGCGCTA GAGGCCCACC
TGGTGCAGTC
8161 GGATCACCAG GTGTTAACGG AGCTCCAGGT GAGGCAGGTA GAGATGGAAA
TCCTGGAAAT
8221 GATGGGCCTC CTGGTAGGGA TGGACAGCCA GGTCATAAAG GTGAAAGAGG
ATACCCTGGA
8281 AATATCGGTC CTGTTGGTGC TGCTGGTGCA CCAGGACCAC ATGGTCCTGT
TGGTCCTGCT
8341 GGAAAGCATG GTAATCGAGG AGAAACTGGA CCATCTGGAC CAGTTGGTCC
AGCAGGTGCT
8401 GTTGGACCAC GAGGACCTTC AGGACCACAG GGAATTAGGG GTGATAAGGG
CGAGCCTGGT
8461 GAAAAGGGAC CTAGGGGTCT TCCAGGTTTG AAAGGTCATA ACGGACTGCA
AGGACTTCCA
8521 GGAATTGCTG GTCACCACGG TGATCAAGGA GCCCCAGGTT CTGTTGGTCC
AGCTGGACCA
8581 AGAGGACCAG CAGGTCCATC AGGTCCAGCT GGAAAAGATG GTAGAACTGG
ACATCCAGGC
 
8641 ACAGTTGGTC CAGCTGGTAT TAGGGGACCT CAAGGTCATC AAGGACCAGC
TGGACCTCCA
8701 GGACCACCTG GTCCACCAGG ACCACCAGGT GTTTCTGGAG GAGGCTACGA
TTTTGGTTAT
8761 GATGGTGATT TTTATAGGGC TTAA
 
SEQ NO 17: Amino Acid Sequence of chimeric P4H/LH3 comprising a chloroplast transit signal, and two A2 cleavage sequences

10 20 30 40 50 60
MATTLISKLT LSSAFLGQQF SSRGNSMRSA PAGLFLRGPR MDISNLPPHI RQQILGLISK

70 80 90 100 110 120
PQQNNDESSS SNNKNNLINN EKVSNVLIDL TSNLKIENFK IFNKESLNQL EKKGYLIIDN

130 140 150 160 170 180
FLNDLNKINL IYDESYNQFK ENKLIEAGMN KGTDKWKDKS IRGDYIQWIH RDSNSRIQDK

190 200 210 220 230 240
DLSSTIRNIN YLLDKLDLIK NEFDNVIPNF NSIKTQTQLA VYLNGGRYIK HRDSFYSSES

250 260 270 280 290 300
LTISRRITMI YYVNKDWKKG DGGELRLYTN NPNNTNQKEL KQTEEFIDIE PIADRLLIFL

310 320 330 340 350 360
SPFLEHEVLQ CNFEPRIAIT TWIYGSGEGR GSLLTCEDVE ENPGPAPDEE DHVLVLHKGN

370 380 390 400 410 420
FDEALAAHKY LLVEFYAPWC GHCKALAPEY AKAAGKLKAE GSEIRLAKVD ATEESDLAQQ

430 440 450 460 470 480
YGVRGYPTIK FFKNGDTASP KEYTAGREAD DIVNWLKKRT GPAASTLSDG AAAEALVESS

490 500 510 520 530 540
EVAVIGFFKD MESDSAKQFF LAAEVIDDIP FGITSNSDVF SKYQLDKDGV VLFKKFDEGR

550 560 570 580 590 600
NNFEGEVTKE KLLDFIKHNQ LPLVIEFTEQ TAPKIFGGEI KTHILLFLPK SVSDYEGKLS

610 620 630 640 650 660
NFKKAAESFK GKILFIFIDS DHTDNQRILE FFGLKKEECP AVRLITLEEE MTKYKPESDE

670 680 690 700 710 720
LTAEKITEFC HRFLEGKIKP HLMSQELPDD WDKQPVKVLV GKNFEEVAFD EKKNVFVEFY

730 740 750 760 770 780
APWCGHCKQL APIWDKLGET YKDHENIVIA KMDSTANEVE AVKVHSFPTL KFFPASADRT

790 800 810 820 830 840
VIDYNGERTL DGFKKFLESG GQDGAGDDDD LEDLEEAEEP DLEEDDDQKA VKDELGSGEG

850 860 870 880 890 900
RGSLLTCEDV EENPGPSDRP RGRDPVNPEK LLVITVATAE TEGYLRFLRS AEFFNYTVRT
 
910 920 930 940 950 960
LGLGEEWRGG DVARTVGGGQ KVRWLKKEME KYADREDMII MFVDSYDVIL AGSPTELLKK

970 980 990 1000 1010 1020
FVQSGSRLLF SAESFCWPEW GLAEQYPEVG TGKRFLNSGG FIGFATTIHQ IVRQWKYKDD

1030 1040 1050 1060 1070 1080
DDDQLFYTRL YLDPGLREKL SLNLDHKSRI FQNLNGALDE VVLKFDRNRV RIRNVAYDTL

1090 1100 1110 1120 1130 1140
PIVVHGNGPT KLQLNYLGNY VPNGWTPEGG CGFCNQDRRT LPGGQPPPRV FLAVFVEQPT

1150 1160 1170 1180 1190 1200
PFLPRFLQRL LLLDYPPDRV TLFLHNNEVF HEPHIADSWP QLQDHFSAVK LVGPEEALSP

1210 1220 1230 1240 1250 1260
GEARDMAMDL CRQDPECEFY FSLDADAVLT NLQTLRILIE ENRKVIAPML SRHGKLWSNF

1270 1280 1290 1300 1310 1320
WGALSPDEYY ARSEDYVELV QRKRVGVWNV PYISQAYVIR GDTLRMELPQ RDVFSGSDTD

1330 1340 1350 1360 1370 1380
PDMAFCKSFR DKGIFLHLSN QHEFGRLLAT SRYDTEHLHP DLWQIFDNPV DWKEQYIHEN

1390 1400 1410 1420 1430 1440
YSRALEGEGI VEQPCPDVYW FPLLSEQMCD ELVAEMEHYG QWSGGRHEDS RLAGGYENVP

1450 1460 1470 1480 1490 1500
TVDIHMKQVG YEDQWLQLLR TYVGPMTESL FPGYHTKARA VMNFVVRYRP DEQPSLRPHH

1510 1520 1530 1540 1550 1560
DSSTFTLNVA LNHKGLDYEG GGCRFLRYDC VISSPRKGWA LLHPGRLTHY HEGLPTTWGT

1570
RYIMVSFVDP
**SEQ NO 18: Nucleotide Sequence of chimeric P4H/LH3, Codon Optimized for Nicotiana Benthamiana Chloroplast Expression** 
1 ATGGCAACAA CACTTATTAG TAAACTCACT CTTTCTAGTG CTTTTCTTGG
ACAGCAATTT
61 TCTAGCAGGG GAAATTCTAT GAGAAGTGCT CCAGCCGGTT TATTTTTGCG
CGGTCCTAGA
121 ATGGATATAA GTAACTTGCC ACCACATATT AGACAGCAAA TTCTTGGTCT
TATCTCAAAG
181 CCTCAACAGA ACAATGATGA ATCTTCATCA TCTAATAATA AGAATAATCT
TATCAATAAC
241 GAAAAGGTTT CTAATGTTCT TATTGATCTT ACTTCTAATT TGAAGATTGA
AAATTTTAAA
301 ATTTTTAATA AAGAGTCACT TAATCAACTC GAAAAAAAGG GATACCTCAT
AATTGATAAT
361 TTCTTAAATG ACCTTAATAA GATTAATCTT ATTTATGATG AATCTTATAA
CCAATTTAAG
 
421 GAAAACAAGC TTATTGAAGC TGGTATGAAT AAGGGTACAG ATAAATGGAA
AGATAAGAGT
481 ATTAGAGGGG ATTATATTCA GTGGATTCAT AGAGATTCCA ATTCTAGAAT
TCAAGATAAG
541 GATCTTTCAA GTACAATTAG AAATATTAAT TATTTGTTGG ACAAGTTGGA
TCTTATTAAG
601 AATGAGTTTG ATAACGTTAT CCCTAATTTT AATTCTATCA AGACTCAAAC
CCAATTGGCT
661 GTATATTTGA ACGGAGGAAG ATACATTAAA CATAGGGATA GTTTTTATTC
CTCAGAATCT
721 TTGACTATTA GCAGAAGAAT TACTATGATT TATTATGTCA ATAAAGACTG
GAAAAAGGGA
781 GATGGAGGAG AGCTTAGACT GTACACTAAT AACCCAAACA ATACTAATCA
AAAAGAGTTG
841 AAACAAACTG AAGAATTTAT TGATATAGAA CCAATAGCAG ACAGATTGCT
TATTTTTTTG
901 TCTCCATTTC TTGAACATGA GGTTCTTCAA TGTAATTTTG AACCACGTAT
TGCTATTACT
961 ACATGGATTT ATGGATCTGG CGAGGGTAGG GGTTCACTCC TTACTTGTGA
GGATGTTGAA
1021 GAGAATCCTG GACCAGCACC AGATGAGGAA GATCATGTTT TGGTTCTTCA
TAAAGGAAAT
1081 TTTGATGAAG CTTTGGCTGC TCACAAATAT TTGCTTGTTG AATTCTATGC
TCCTTGGTGT
1141 GGTCATTGCA AGGCATTGGC CCCTGAGTAT GCTAAGGCAG CTGGAAAGTT
GAAGGCCGAG
1201 GGATCTGAAA TTAGACTTGC AAAGGTTGAC GCTACTGAGG AATCTGATTT
GGCACAACAA
1261 TATGGTGTTA GAGGTTACCC AACTATTAAG TTCTTTAAGA ATGGTGACAC
TGCTAGTCCT
1321 AAGGAATATA CCGCTGGTAG AGAGGCCGAT GATATCGTAA ATTGGCTTAA
GAAAAGGACA
1381 GGACCAGCAG CTTCAACATT GTCAGATGGT GCTGCTGCTG AAGCTTTAGT
CGAATCTTCA
1441 GAAGTTGCTG TGATTGGATT TTTTAAAGAC ATGGAATCTG ATAGTGCCAA
ACAGTTTTTT
1501 TTGGCTGCAG AGGTGATTGA TGATATTCCA TTTGGAATTA CTTCAAATTC
AGATGTGTTT
1561 TCAAAATATC AGCTTGATAA GGACGGAGTT GTTTTGTTCA AAAAGTTCGA
TGAAGGAAGA
1621 AATAATTTTG AAGGTGAAGT GACTAAAGAA AAGCTTCTTG ATTTTATTAA
GCACAATCAA
1681 TTGCCATTGG TTATTGAATT TACTGAGCAA ACTGCTCCAA AGATTTTTGG
TGGTGAAATT
1741 AAGACTCATA TTCTTTTGTT CTTGCCTAAG TCTGTTAGTG ATTATGAAGG
TAAGTTGAGC
1801 AACTTTAAAA AGGCTGCTGA ATCTTTTAAG GGAAAAATTC TTTTTATCTT
CATTGATAGC
1861 GATCACACAG ATAATCAGAG AATATTGGAA TTCTTCGGTT TGAAGAAGGA
AGAATGCCCT
1921 GCTGTTAGGT TGATTACACT GGAGGAGGAG ATGACTAAGT ACAAGCCTGA
ATCTGATGAG
1981 CTTACTGCTG AAAAGATCAC TGAATTCTGC CACAGATTTC TTGAGGGGAA
GATTAAGCCA
2041 CACCTTATGT CTCAGGAGTT ACCTGATGAT TGGGATAAAC AACCTGTTAA
GGTTCTCGTG
2101 GGTAAGAACT TTGAAGAGGT TGCTTTCGAT GAAAAAAAAA ATGTTTTCGT
TGAATTCTAT
 
2161 GCACCTTGGT GCGGTCATTG TAAACAGCTA GCACCAATTT GGGATAAACT
TGGGGAAACT
2221 TATAAGGATC ATGAGAATAT TGTTATAGCT AAAATGGATT CAACGGCTAA
TGAAGTTGAA
2281 GCTGTTAAAG TCCATTCATT TCCTACTCTT AAGTTCTTTC CTGCTTCAGC
AGACCGTACC
2341 GTTATTGATT ACAATGGTGA GAGAACATTG GATGGCTTTA AAAAATTTTT
GGAATCTGGC
2401 GGACAGGATG GAGCTGGAGA TGATGATGAC TTGGAGGATT TAGAGGAGGC
CGAAGAGCCT
2461 GATTTGGAAG AAGATGATGA TCAAAAAGCA GTGAAGGATG AACTAGGTTC
AGGAGAGGGT
2521 AGGGGGAGTT TGTTGACTTG CGAGGATGTA GAAGAAAACC CTGGTCCATC
TGATAGACCT
2581 AGAGGTAGAG ATCCTGTTAA CCCAGAGAAG CTTTTGGTTA TTACAGTGGC
TACAGCTGAG
2641 ACAGAAGGAT ATCTTAGGTT TCTAAGGTCT GCTGAATTTT TTAATTATAC
AGTTAGAACA
2701 TTGGGACTTG GGGAGGAATG GAGAGGTGGG GATGTTGCTC GAACTGTGGG
AGGAGGTCAA
2761 AAGGTTCGTT GGTTGAAGAA AGAAATGGAA AAATATGCAG ATAGAGAAGA
TATGATTATT
2821 ATGTTTGTTG ATAGTTACGA TGTTATTTTG GCTGGAAGCC CTACAGAATT
GTTAAAGAAG
2881 TTTGTTCAAT CTGGCTCAAG GCTTTTGTTC TCCGCAGAGA GTTTCTGCTG
GCCAGAGTGG
2941 GGACTAGCTG AACAGTATCC TGAGGTGGGT ACTGGTAAGA GGTTTCTCAA
TTCCGGTGGT
3001 TTTATTGGCT TCGCAACTAC TATTCACCAA ATTGTTAGAC AATGGAAGTA
TAAAGATGAT
3061 GATGATGATC AACTTTTTTA TACAAGACTT TACCTTGACC CAGGTTTGAG
AGAAAAGTTG
3121 TCTCTGAACT TGGATCACAA GTCTAGAATT TTCCAAAATC TCAATGGAGC
TTTGGATGAA
3181 GTTGTTTTGA AATTTGATAG AAATAGGGTT AGGATTCGTA ATGTTGCCTA
TGACACACTT
3241 CCTATTGTAG TGCATGGAAA TGGACCTACT AAGCTTCAGT TGAACTATTT
AGGTAACTAT
3301 GTGCCTAACG GATGGACTCC AGAAGGTGGT TGTGGATTTT GTAATCAAGA
TCGAAGAACT
3361 TTGCCAGGAG GACAACCTCC ACCAAGGGTT TTTCTTGCTG TTTTCGTTGA
GCAACCTACC
3421 CCATTCCTTC CAAGATTCTT ACAAAGACTT TTGTTGCTTG ATTATCCACC
AGATAGAGTT
3481 ACTTTGTTCC TTCACAATAA TGAGGTGTTT CATGAGCCTC ATATTGCTGA
TAGTTGGCCA
3541 CAACTCCAAG ATCATTTCTC CGCAGTTAAG CTCGTTGGTC CAGAGGAAGC
TTTGTCTCCT
3601 GGTGAAGCTA GGGATATGGC AATGGATCTT TGCAGACAAG ATCCTGAATG
CGAATTTTAC
3661 TTTTCTTTGG ATGCTGATGC TGTGCTTACA AATCTTCAGA CTCTAAGAAT
TTTGATAGAG
3721 GAGAACAGGA AAGTTATTGC TCCAATGCTT AGTAGGCATG GTAAATTGTG
GAGTAATTTC
3781 TGGGGAGCTC TTTCTCCGGA TGAATATTAT GCTAGATCGG AAGATTACGT
GGAGCTTGTT
3841 CAACGTAAGA GAGTTGGTGT ATGGAATGTT CCATATATCT CACAAGCTTA
CGTTATCAGA
 
3901 GGAGATACAT TGAGAATGGA ACTTCCTCAG AGAGATGTTT TTAGCGGATC
AGATACCGAT
3961 CCTGATATGG CATTTTGTAA ATCATTCAGA GATAAGGGAA TTTTCCTTCA
TCTATCTAAT
4021 CAGCACGAAT TCGGAAGGTT GCTTGCTACA TCAAGATATG ATACTGAGCA
CCTGCATCCA
4081 GATTTGTGGC AAATTTTCGA TAATCCAGTG GATTGGAAAG AACAATACAT
ACATGAAAAT
4141 TATTCTAGAG CTCTTGAAGG TGAGGGAATT GTCGAACAAC CTTGCCCAGA
CGTCTATTGG
4201 TTTCCACTTC TTTCTGAGCA AATGTGCGAT GAACTAGTGG CAGAGATGGA
ACATTACGGA
4261 CAATGGTCTG GAGGACGGCA TGAGGATTCA AGATTGGCTG GAGGGTACGA
GAATGTGCCA
4321 ACTGTCGATA TTCATATGAA GCAAGTTGGA TATGAAGATC AGTGGTTGCA
ACTTTTAAGA
4381 ACATACGTTG GTCCTATGAC TGAATCATTG TTTCCAGGAT ACCACACAAA
AGCAAGAGCA
4441 GTTATGAATT TCGTTGTTAG ATACAGACCA GATGAGCAAC CTTCTTTAAG
ACCACATCAT
4501 GATTCTTCTA CATTTACTCT CAATGTTGCT TTGAATCACA AAGGTCTTGA
TTATGAGGGA
4561 GGAGGATGCA GGTTTCTGAG ATATGATTGT GTAATTTCAT CGCCTCGTAA
AGGATGGGCT
4621 TTGCTCCATC CAGGAAGACT TACTCACTAT CATGAAGGAC TCCCTACTAC
ATGGGGTACT
4681 AGATATATTA TGGTTTCATT TGTTGATCCT TGA
 
SEQ NO 19: Amino Acid Sequence FIB3COL1 comprising a chloroplast transit signal, and two
A2 cleavage sequences

10 20 30 40 50 60
MATTLISKLT LSSAFLGQQF SSRGNSMRSA PAGLFLRGPR ARAGSGQQGP GQQGPGQQGP

70 80 90 100 110 120
GQQGPYGPGA SAAAAAAGGY GPGSGQQGPS QQGPGQQGPG GQGPYGPGAS AAAAAAGGYG

130 140 150 160 170 180
PGSGQQGPGG QGPYGPGSSA AAAAAGGNGP GSGQQGAGQQ GPGQQGPGAS AAAAAAGGYG

190 200 210 220 230 240
PGSGQQGPGQ QGPGGQGPYG PGASAAAAAA GGYGPGSGQG PGQQGPGGQG PYGPGASAAA

250 260 270 280 290 300
AAAGGYGPGS GQQGPGQQGP GQQGPGGQGP YGPGASAAAA AAGGYGPGYG QQGPGQQGPG

310 320 330 340 350 360
GQGPYGPGAS AASAASGGYG PGSGQQGPGQ QGPGGQGPYG PGASAAAAAA GGYGPGSGQQ

370 380 390 400 410 420
GPGQQGPGQQ GPGQQGPGGQ GPYGPGASAA AAAAGGYGPG SGQQGPGQQG PGQQGPGQQG

430 440 450 460 470 480
 
PGQQGPGQQG PGQQGPGQQG PGQQGPGGQG AYGPGASAAA GAAGGYGPGS GQQGPGQQGP

490 500 510 520 530 540
GQQGPGQQGP GQQGPGQQGP GQQGPGQQGP YGPGASAAAA AAGGYGPGSG QQGPGQQGPG

550 560 570 580 590 600
QQGPGGQGPY GPGAASAAVS VGGYGPQSSS VPVASAVASR LSSPAASSRV SSAVSSLVSS

610 620 630 640 650 660
GPTKHAALSN TISSVVSQVS ASNPGLSGCD VLVQALLEVV SALVSILGSS SIGQINYGAS

670 680 690 700 710 720
AQYTQMVGQS VAQALAGSGE GRGSLLTCED VEENPGPQLS YGYDEKSTGG ISVPGPMGPS

730 740 750 760 770 780
GPRGLPGPPG APGPQGFQGP PGEPGEPGAS GPMGPRGPPG PPGKNGDDGE AGKPGRPGER

790 800 810 820 830 840
GPPGPQGARG LPGTAGLPGM KGHRGFSGLD GAKGDAGPAG PKGEPGSPGE NGAPGQMGPR

850 860 870 880 890 900
GLPGERGRPG APGPAGARGN DGATGAAGPP GPTGPAGPPG FPGAVGAKGE AGPQGPRGSE

910 920 930 940 950 960
GPQGVRGEPG PPGPAGAAGP AGNPGADGQP GAKGANGAPG IAGAPGFPGA RGPSGPQGPG

970 980 990 1000 1010 1020
GPPGPKGNSG EPGAPGSKGD TGAKGEPGPV GVQGPPGPAG EEGKRGARGE PGPTGLPGPP

1030 1040 1050 1060 1070 1080
GERGGPGSRG FPGADGVAGP KGPAGERGSP GPAGPKGSPG EAGRPGEAGL PGAKGLTGSP

1090 1100 1110 1120 1130 1140
GSPGPDGKTG PPGPAGQDGR PGPPGPPGAR GQAGVMGFPG PKGAAGEPGK AGERGVPGPP
1150 1160 1170 1180 1190 1200
GAVGPAGKDG EAGAQGPPGP AGPAGERGEQ GPAGSPGFQG LPGPAGPPGE AGKPGEQGVP

1210 1220 1230 1240 1250 1260
GDLGAPGPSG ARGERGFPGE RGVQGPPGPA GPRGANGAPG NDGAKGDAGA PGAPGSQGAP

1270 1280 1290 1300 1310 1320
GLQGMPGERG AAGLPGPKGD RGDAGPKGAD GSPGKDGVRG LTGPIGPPGP AGAPGDKGES

1330 1340 1350 1360 1370 1380
GPSGPAGPTG ARGAPGDRGE PGPPGPAGFA GPPGADGQPG AKGEPGDAGA KGDAGPPGPA

1390 1400 1410 1420 1430 1440
GPAGPPGPIG NVGAPGAKGA RGSAGPPGAT GFPGAAGRVG PPGPSGNAGP PGPPGPAGKE

1450 1460 1470 1480 1490 1500
GGKGPRGETG PAGRPGEVGP PGPPGPAGEK GSPGADGPAG APGTPGPQGI AGQRGVVGLP

1510 1520 1530 1540 1550 1560
GQRGERGFPG LPGPSGEPGK QGPSGASGER GPPGPMGPPG LAGPPGESGR EGAPGAEGSP

1570 1580 1590 1600 1610 1620
GRDGSPGAKG DRGETGPAGP PGAPGAPGAP GPVGPAGKSG DRGETGPAGP AGPVGPVGAR

1630 1640 1650 1660 1670 1680
 
GPAGPQGPRG DKGETGEQGD RGIKGHRGFS GLQGPPGPPG SPGEQGPSGA SGPAGPRGPP

1690 1700 1710 1720 1730 1740
GSAGAPGKDG LNGLPGPIGP PGPRGRTGDA GPVGPPGPPG PPGPPGPPSA GFDFSFLPQP

1750 1760 1770 1780 1790 1800
PQEKAHDGGR YYRAGSGEGR GSLLTCEDVE ENPGPQYDGK GVGLGPGPMG LMGPRGPPGA

1810 1820 1830 1840 1850 1860
AGAPGPQGFQ GPAGEPGEPG QTGPAGARGP AGPPGKAGED GHPGKPGRPG ERGVVGPQGA

1870 1880 1890 1900 1910 1920
RGFPGTPGLP GFKGIRGHNG LDGLKGQPGA PGVKGEPGAP GENGTPGQTG ARGLPGERGR

1930 1940 1950 1960 1970 1980
VGAPGPAGAR GSDGSVGPVG PAGPIGSAGP PGFPGAPGPK GEIGAVGNAG PAGPAGPRGE

1990 2000 2010 2020 2030 2040
VGLPGLSGPV GPPGNPGANG LTGAKGAAGL PGVAGAPGLP GPRGIPGPVG AAGATGARGL

2050 2060 2070 2080 2090 2100
VGEPGPAGSK GESGNKGEPG SAGPQGPPGP SGEEGKRGPN GEAGSAGPPG PPGLRGSPGS

2110 2120 2130 2140 2150 2160
RGLPGADGRA GVMGPPGSRG ASGPAGVRGP NGDAGRPGEP GLMGPRGLPG SPGNIGPAGK

2170 2180 2190 2200 2210 2220
EGPVGLPGID GRPGPIGPAG ARGEPGNIGF PGPKGPTGDP GKNGDKGHAG LAGARGAPGP

2230 2240 2250 2260 2270 2280
DGNNGAQGPP GPQGVQGGKG EQGPPGPPGF QGLPGPSGPA GEVGKPGERG LHGEFGLPGP

2290 2300 2310 2320 2330 2340
AGPRGERGPP GESGAAGPTG PIGSRGPSGP PGPDGNKGEP GVVGAVGTAG PSGPSGLPGE

2350 2360 2370 2380 2390 2400
RGAAGIPGGK GEKGEPGLRG EIGNPGRDGA RGAPGAVGAP GPAGATGDRG EAGAAGPAGP

2410 2420 2430 2440 2450 2460
AGPRGSPGER GEVGPAGPNG FAGPAGAAGQ PGAKGERGAK GPKGENGVVG PTGPVGAAGP

2470 2480 2490 2500 2510 2520
AGPNGPPGPA GSRGDGGPPG MTGFPGAAGR TGPPGPSGIS GPPGPPGPAG KEGLRGPRGD

2530 2540 2550 2560 2570 2580
QGPVGRTGEV GAVGPPGFAG EKGPSGEAGT AGPPGTPGPQ GLLGAPGILG LPGSRGERGL

2590 2600 2610 2620 2630 2640
PGVAGAVGEP GPLGIAGPPG ARGPPGAVGS PGVNGAPGEA GRDGNPGNDG PPGRDGQPGH

2650 2660 2670 2680 2690 2700
KGERGYPGNI GPVGAAGAPG PHGPVGPAGK HGNRGETGPS GPVGPAGAVG PRGPSGPQGI

2710 2720 2730 2740 2750 2760
RGDKGEPGEK GPRGLPGLKG HNGLQGLPGI AGHHGDQGAP GSVGPAGPRG PAGPSGPAGK

2770 2780 2790 2800 2810
DGRTGHPGTV GPAGIRGPQG HQGPAGPPGP PGPPGPPGVS GGGYDFGYDG DFYRA
**SEQ NO 20: Nucleotide Sequence of FIB3COL1, Codon Optimized for Nicotiana Benthamiana Chloroplast Expression**

1 ATGGCTACTA CTTTGATTTC AAAGTTAACC CTTTCTAGTG CTTTCCTCGG
CCAACAGTTT
61 TCTTCTAGGG GTAATTCTAT GAGATCTGCA CCTGCAGGAT TGTTTCTTCG
TGGACCAAGA
121 GCTAGAGCAG GATCGGGTCA ACAAGGACCA GGACAACAGG GACCAGGACA
ACAGGGTCCA
181 GGTCAACAAG GACCATATGG TCCTGGAGCA TCAGCAGCTG CTGCTGCAGC
TGGTGGATAC
241 GGTCCAGGAA GCGGTCAACA AGGTCCATCC CAACAAGGTC CTGGTCAACA
AGGACCAGGA
301 GGGCAAGGTC CTTACGGACC TGGTGCTAGT GCAGCTGCTG CAGCTGCTGG
AGGTTACGGA
361 CCAGGTTCTG GTCAACAAGG ACCAGGAGGA CAAGGTCCAT ACGGACCAGG
ATCTTCTGCT
421 GCAGCTGCTG CTGCAGGAGG AAATGGTCCT GGATCTGGAC AACAAGGAGC
AGGTCAACAA
481 GGTCCTGGCC AACAAGGTCC AGGTGCTTCT GCTGCTGCTG CTGCAGCAGG
TGGTTATGGT
541 CCCGGATCAG GACAACAAGG TCCTGGACAA CAAGGTCCTG GAGGACAAGG
ACCTTATGGT
601 CCTGGTGCTA GTGCTGCTGC TGCTGCTGCT GGAGGATATG GTCCAGGAAG
CGGACAAGGA
661 CCAGGACAGC AAGGGCCTGG AGGTCAGGGT CCATATGGTC CTGGAGCTTC
TGCAGCTGCT
721 GCTGCTGCTG GTGGATATGG ACCAGGTTCT GGACAACAGG GTCCTGGTCA
ACAAGGACCA
781 GGACAGCAGG GACCAGGAGG TCAAGGTCCA TATGGACCTG GAGCATCAGC
AGCTGCAGCA
841 GCTGCAGGTG GCTATGGTCC TGGATATGGT CAACAGGGAC CTGGACAGCA
GGGTCCTGGA
901 GGTCAAGGTC CTTATGGTCC TGGAGCTTCA GCTGCTTCTG CAGCTTCCGG
TGGATATGGA
961 CCTGGATCTG GTCAGCAAGG CCCTGGTCAA CAAGGTCCAG GAGGTCAAGG
ACCTTATGGG
1021 CCTGGAGCTT CTGCTGCTGC AGCTGCAGCT GGAGGATACG GACCTGGATC
TGGTCAGCAA
1081 GGACCAGGTC AACAGGGTCC AGGTCAACAA GGACCAGGTC AACAAGGTCC
AGGAGGGCAG
1141 GGACCATATG GACCTGGAGC TTCAGCAGCA GCTGCTGCTG CTGGTGGATA
CGGTCCAGGT
1201 TCAGGACAAC AGGGCCCTGG ACAACAAGGA CCTGGCCAAC AAGGACCTGG
TCAACAAGGT
1261 CCTGGTCAAC AAGGACCTGG TCAACAAGGA CCAGGACAAC AAGGACCAGG
TCAACAGGGA
1321 CCAGGTCAAC AAGGTCCTGG AGGTCAGGGT GCTTATGGTC CAGGTGCTTC
CGCTGCTGCT
1381 GGTGCTGCAG GTGGTTACGG ACCTGGATCT GGACAGCAAG GACCAGGTCA
ACAAGGACCT
1441 GGACAACAAG GTCCAGGACA ACAAGGACCT GGACAACAAG GTCCAGGTCA
ACAAGGTCCT
1501 GGTCAGCAGG GTCCAGGACA ACAAGGTCCT TATGGACCAG GGGCTAGCGC
TGCTGCAGCA

1561 GCAGCAGGTG GATATGGACC AGGTAGTGGT CAACAAGGTC CTGGACAGCA
AGGTCCTGGT
1621 CAACAAGGTC CTGGAGGTCA AGGACCCTAC GGTCCAGGTG CTGCTTCAGC
AGCTGTGTCT
1681 GTTGGTGGAT ATGGACCACA GTCTTCTTCA GTCCCAGTTG CATCTGCAGT
TGCATCTAGA
1741 CTTTCATCTC CAGCTGCTTC ATCTAGAGTT TCTTCTGCTG TTTCTTCTCT
TGTGTCATCT
1801 GGTCCAACTA AACATGCTGC ACTTTCTAAC ACAATTAGTT CAGTTGTTTC
TCAAGTTTCT
1861 GCATCTAACC CAGGACTTTC TGGTTGCGAT GTTCTTGTGC AAGCTCTTCT
GGAAGTTGTT
1921 AGTGCTTTGG TTTCCATTTT GGGTTCTAGC TCTATTGGAC AGATCAATTA
TGGTGCTTCA
1981 GCACAATACA CTCAAATGGT TGGACAAAGC GTTGCTCAGG CTCTTGCTGG
AAGCGGAGAA
2041 GGAAGAGGTA GTCTGCTTAC ATGTGAAGAT GTTGAAGAAA ATCCTGGTCC
ACAACTTTCA
2101 TATGGTTATG ATGAGAAATC AACAGGTGGT ATTTCTGTGC CAGGACCTAT
GGGTCCTTCA
2161 GGCCCTAGAG GATTGCCAGG TCCACCTGGT GCTCCTGGTC CTCAAGGATT
CCAAGGACCA
2221 CCAGGTGAGC CAGGTGAACC TGGAGCTAGT GGACCAATGG GTCCTAGAGG
TCCACCTGGT
2281 CCTCCTGGTA AAAATGGTGA TGATGGAGAG GCAGGAAAGC CTGGAAGACC
TGGAGAAAGA
2341 GGACCACCTG GACCTCAAGG AGCTCGGGGA CTTCCAGGTA CAGCTGGATT
GCCAGGTATG
2401 AAGGGACACA GAGGATTCAG TGGCTTGGAT GGAGCTAAGG GAGATGCTGG
TCCAGCTGGA
2461 CCTAAAGGAG AGCCAGGTTC TCCAGGAGAA AACGGAGCTC CAGGACAAAT
GGGACCTAGA
2521 GGTCTTCCTG GTGAAAGGGG TAGGCCAGGA GCCCCTGGAC CTGCTGGTGC
TAGAGGTAAC
2581 GATGGAGCTA CTGGTGCTGC TGGACCACCA GGACCTACTG GTCCTGCAGG
TCCACCAGGT
2641 TTTCCAGGTG CAGTTGGAGC AAAGGGTGAG GCTGGTCCAC AAGGACCTAG
AGGTTCAGAA
2701 GGACCACAAG GTGTTAGAGG TGAACCAGGT CCACCGGGAC CAGCAGGAGC
CGCTGGCCCC
2761 GCTGGTAATC CTGGTGCTGA TGGTCAACCA GGTGCTAAGG GAGCTAACGG
TGCTCCAGGG
2821 ATTGCTGGTG CTCCAGGATT CCCTGGAGCT AGAGGACCTT CAGGTCCACA
AGGTCCTGGT
2881 GGACCACCTG GACCAAAAGG AAATAGCGGA GAGCCAGGTG CACCTGGCTC
AAAGGGAGAT
2941 ACTGGAGCAA AGGGAGAGCC TGGACCTGTT GGTGTTCAAG GTCCTCCTGG
ACCTGCTGGT
3001 GAGGAGGGAA AGAGAGGTGC AAGAGGTGAG CCTGGTCCTA CAGGACTCCC
TGGTCCTCCT
3061 GGTGAAAGGG GAGGACCTGG ATCTAGGGGT TTTCCAGGTG CTGATGGAGT
TGCTGGACCT
3121 AAAGGACCAG CTGGAGAAAG GGGATCTCCA GGTCCAGCTG GGCCAAAGGG
TTCTCCTGGA
3181 GAGGCAGGAA GACCAGGTGA AGCTGGATTG CCAGGTGCCA AGGGACTTAC
AGGATCTCCT
3241 GGGTCACCAG GACCAGATGG AAAGACTGGT CCTCCTGGAC CAGCTGGACA
AGATGGAAGA

3301 CCTGGACCAC CTGGACCACC TGGAGCAAGG GGTCAAGCTG GTGTTATGGG
TTTTCCAGGT
3361 CCAAAAGGTG CAGCAGGCGA GCCAGGAAAG GCTGGTGAAA GGGGTGTTCC
AGGTCCACCT
3421 GGAGCAGTTG GTCCAGCTGG AAAGGATGGA GAGGCTGGCG CTCAAGGTCC
TCCTGGTCCT
3481 GCTGGGCCAG CAGGTGAAAG AGGAGAACAA GGACCTGCTG GGTCTCCTGG
TTTTCAAGGA
3541 CTTCCTGGAC CAGCTGGTCC TCCAGGTGAA GCAGGCAAGC CAGGAGAGCA
AGGTGTTCCT
3601 GGAGATCTTG GTGCCCCAGG TCCTTCTGGT GCAAGAGGAG AGCGTGGATT
CCCTGGAGAA
3661 AGAGGTGTGC AAGGTCCTCC AGGTCCAGCT GGTCCACGTG GAGCTAACGG
AGCTCCTGGT
3721 AACGATGGAG CTAAAGGAGA TGCTGGTGCC CCAGGCGCAC CTGGTTCACA
AGGTGCTCCT
3781 GGATTGCAAG GTATGCCTGG CGAAAGAGGT GCTGCTGGAC TTCCTGGACC
TAAGGGTGAC
3841 AGAGGTGATG CTGGACCAAA AGGAGCTGAT GGATCACCTG GTAAAGATGG
AGTGAGAGGT
3901 TTAACCGGTC CAATTGGACC ACCAGGTCCC GCTGGAGCTC CAGGAGATAA
AGGAGAAAGT
3961 GGACCATCAG GTCCTGCCGG TCCCACTGGT GCTAGAGGTG CACCTGGTGA
TAGAGGTGAA
4021 CCTGGTCCAC CAGGGCCTGC TGGATTTGCT GGTCCACCAG GAGCAGATGG
ACAACCAGGA
4081 GCAAAAGGTG AGCCTGGAGA TGCTGGAGCT AAAGGAGATG CAGGTCCTCC
TGGACCAGCT
4141 GGACCTGCTG GACCACCTGG ACCAATTGGA AATGTTGGTG CTCCAGGAGC
TAAAGGGGCA
4201 AGAGGATCTG CTGGTCCTCC TGGAGCAACT GGGTTCCCTG GAGCAGCAGG
AAGAGTTGGT
4261 CCTCCTGGAC CTTCTGGAAA CGCTGGACCT CCTGGTCCAC CAGGACCTGC
TGGAAAGGAA
4321 GGAGGAAAGG GTCCAAGAGG CGAAACTGGA CCAGCAGGTA GACCAGGAGA
GGTTGGACCA
4381 CCTGGACCAC CTGGTCCCGC TGGAGAGAAA GGATCTCCTG GAGCTGATGG
ACCAGCAGGT
4441 GCTCCAGGCA CTCCAGGCCC ACAAGGAATT GCTGGTCAAA GGGGAGTTGT
TGGATTGCCT
4501 GGGCAAAGAG GAGAGAGGGG ATTTCCTGGT CTTCCTGGTC CATCAGGTGA
ACCTGGAAAA
4561 CAAGGTCCAT CTGGAGCTAG TGGTGAGAGG GGCCCTCCAG GACCAATGGG
CCCACCTGGA
4621 CTTGCTGGAC CTCCTGGAGA GTCCGGTAGA GAAGGGGCTC CAGGTGCTGA
AGGATCACCA
4681 GGAAGGGATG GATCTCCTGG AGCCAAGGGG GATAGAGGAG AAACAGGTCC
AGCAGGGCCT
4741 CCTGGTGCAC CAGGTGCACC TGGTGCTCCT GGTCCAGTTG GACCTGCAGG
TAAATCTGGT
4801 GATCGTGGAG AAACTGGTCC AGCTGGACCT GCTGGACCTG TTGGACCAGT
GGGTGCTCGT
4861 GGACCTGCTG GTCCACAGGG ACCAAGAGGA GATAAAGGTG AGACTGGTGA
GCAAGGTGAT
4921 AGAGGAATTA AAGGACATAG GGGTTTTTCT GGCTTACAGG GTCCTCCAGG
TCCACCAGGA
4981 TCTCCAGGAG AACAAGGTCC ATCAGGAGCT AGTGGACCAG CAGGGCCAAG
GGGACCTCCT

5041 GGTTCTGCTG GTGCACCAGG TAAAGATGGG CTTAACGGAT TGCCTGGACC
TATAGGTCCT
5101 CCAGGTCCAA GAGGAAGAAC TGGTGATGCT GGTCCTGTTG GACCACCAGG
TCCACCTGGA
5161 CCACCAGGGC CACCTGGACC TCCATCTGCA GGATTTGATT TTTCTTTCCT
TCCACAACCA
5221 CCACAAGAAA AGGCTCACGA TGGTGGAAGG TATTATAGGG CAGGCTCTGG
TGAAGGGCGT
5281 GGAAGTCTTC TTACATGTGA GGATGTTGAA GAAAATCCAG GACCACAATA
TGATGGAAAG
5341 GGTGTTGGAT TGGGTCCAGG TCCAATGGGA TTGATGGGCC CTAGAGGTCC
TCCTGGAGCT
5401 GCTGGAGCTC CTGGACCACA AGGATTCCAG GGCCCAGCTG GTGAACCTGG
AGAACCGGGA
5461 CAAACAGGAC CAGCTGGTGC TAGAGGTCCA GCTGGTCCTC CAGGAAAAGC
TGGAGAAGAT
5521 GGCCATCCTG GTAAACCAGG TAGGCCAGGA GAAAGAGGTG TTGTGGGTCC
ACAGGGAGCT
5581 AGGGGATTTC CTGGTACTCC TGGGTTGCCT GGATTCAAGG GAATTAGGGG
TCATAATGGT
5641 CTTGATGGTC TTAAAGGACA ACCAGGAGCT CCTGGTGTTA AAGGAGAACC
TGGAGCACCT
5701 GGTGAAAATG GTACTCCAGG TCAAACAGGT GCAAGAGGAT TGCCAGGAGA
AAGGGGTAGA
5761 GTGGGAGCAC CAGGTCCTGC TGGAGCTAGA GGTTCAGATG GAAGTGTGGG
ACCTGTGGGA
5821 CCTGCAGGAC CAATTGGATC AGCTGGTCCA CCTGGATTTC CAGGTGCCCC
AGGTCCAAAG
5881 GGAGAAATTG GAGCTGTTGG AAATGCGGGC CCAGCAGGCC CAGCTGGACC
TAGAGGTGAG
5941 GTTGGTCTAC CAGGTCTGTC AGGACCAGTG GGCCCTCCAG GAAATCCTGG
TGCAAATGGG
6001 CTTACAGGAG CTAAGGGAGC AGCTGGATTG CCTGGTGTTG CTGGGGCACC
AGGTCTTCCT
6061 GGTCCAAGAG GTATTCCAGG ACCAGTAGGT GCTGCAGGAG CAACTGGAGC
TAGAGGTTTG
6121 GTTGGTGAAC CAGGACCAGC AGGCTCCAAG GGTGAATCTG GTAATAAGGG
AGAACCTGGT
6181 TCTGCTGGAC CACAAGGACC ACCAGGACCA TCAGGAGAAG AAGGTAAGAG
GGGTCCTAAC
6241 GGAGAGGCCG GTTCTGCAGG TCCACCTGGA CCACCTGGAC TTAGAGGATC
TCCAGGGTCT
6301 AGAGGTTTAC CTGGTGCTGA TGGTAGAGCT GGAGTGATGG GTCCTCCAGG
TTCAAGAGGA
6361 GCATCTGGCC CAGCAGGAGT TAGGGGACCA AATGGTGATG CTGGGAGACC
AGGTGAACCA
6421 GGTCTTATGG GTCCTAGAGG ATTGCCAGGT TCACCAGGAA ATATTGGTCC
AGCTGGAAAA
6481 GAAGGACCAG TTGGACTTCC TGGAATTGAT GGTAGACCAG GTCCTATTGG
TCCTGCTGGT
6541 GCTAGAGGTG AGCCAGGTAA TATCGGTTTT CCAGGACCAA AGGGACCAAC
TGGTGATCCA
6601 GGCAAAAATG GTGATAAGGG ACATGCTGGA CTCGCAGGAG CTAGAGGCGC
TCCAGGACCT
6661 GATGGAAATA ATGGTGCCCA GGGACCTCCA GGACCACAAG GTGTTCAAGG
AGGAAAGGGT
6721 GAGCAAGGAC CTCCAGGACC TCCAGGTTTT CAGGGACTTC CAGGACCATC
TGGACCAGCA

6781 GGTGAGGTTG GTAAGCCAGG AGAAAGGGGT TTACATGGTG AATTCGGTCT
GCCAGGACCA
6841 GCTGGACCAA GGGGTGAAAG GGGTCCACCA GGAGAGTCAG GTGCTGCTGG
ACCAACAGGA
6901 CCAATTGGTT CAAGAGGTCC ATCTGGACCT CCAGGTCCTG ATGGAAACAA
AGGTGAACCA
6961 GGAGTTGTAG GTGCTGTTGG AACTGCTGGT CCTTCAGGCC CAAGCGGACT
TCCAGGTGAA
7021 AGGGGTGCTG CTGGTATTCC TGGAGGTAAG GGTGAAAAAG GGGAGCCTGG
TCTTAGAGGT
7081 GAGATTGGTA ATCCAGGAAG AGATGGGGCT AGAGGTGCAC CAGGAGCCGT
TGGTGCTCCT
7141 GGTCCTGCTG GAGCTACAGG AGATAGAGGA GAGGCAGGAG CTGCTGGTCC
TGCTGGACCA
7201 GCTGGCCCAA GAGGTAGCCC AGGAGAAAGA GGTGAAGTTG GTCCAGCTGG
TCCTAATGGA
7261 TTTGCTGGAC CTGCTGGTGC TGCTGGTCAG CCTGGAGCTA AAGGGGAAAG
AGGAGCCAAA
7321 GGACCTAAAG GAGAAAATGG AGTTGTTGGG CCTACAGGAC CAGTAGGAGC
AGCAGGACCT
7381 GCTGGTCCAA ATGGACCACC AGGACCAGCA GGATCCAGAG GAGATGGTGG
TCCACCAGGA
7441 ATGACAGGTT TTCCTGGTGC TGCTGGAAGA ACAGGACCAC CAGGTCCTTC
AGGTATTTCT
7501 GGTCCTCCAG GTCCTCCAGG ACCAGCTGGA AAGGAGGGTT TGAGAGGACC
TAGAGGTGAT
7561 CAAGGACCTG TGGGAAGAAC AGGAGAAGTT GGAGCAGTTG GACCACCAGG
TTTCGCTGGA
7621 GAAAAGGGAC CATCTGGCGA AGCTGGAACT GCTGGACCAC CAGGTACCCC
TGGACCTCAG
7681 GGACTTCTTG GAGCTCCTGG AATTTTGGGA CTTCCCGGAT CTAGAGGAGA
GAGGGGATTG
7741 CCAGGCGTTG CTGGAGCTGT GGGTGAGCCA GGTCCTCTCG GAATTGCTGG
ACCACCTGGT
7801 GCAAGGGGTC CACCAGGTGC CGTTGGGTCC CCAGGTGTTA ATGGTGCTCC
AGGTGAGGCT
7861 GGTAGGGATG GAAATCCAGG TAATGATGGA CCACCTGGTA GGGATGGACA
GCCTGGCCAT
7921 AAGGGTGAGC GTGGATATCC AGGTAATATT GGACCAGTTG GTGCAGCAGG
GGCACCAGGA
7981 CCACACGGAC CTGTTGGTCC AGCTGGTAAG CACGGCAATA GGGGAGAGAC
TGGTCCTTCA
8041 GGACCTGTGG GTCCGGCTGG AGCAGTTGGA CCTAGAGGTC CATCAGGACC
ACAAGGAATT
8101 AGAGGTGATA AGGGAGAACC CGGGGAGAAA GGACCAAGAG GATTACCTGG
ATTAAAGGGT
8161 CACAATGGAT TACAAGGATT GCCTGGAATT GCTGGACATC ACGGAGATCA
AGGAGCACCA
8221 GGATCAGTTG GACCGGCTGG ACCAAGAGGA CCTGCAGGAC CTTCTGGACC
TGCTGGTAAA
8281 GATGGAAGAA CTGGACATCC TGGTACAGTT GGACCTGCTG GAATTAGAGG
TCCACAAGGT
8341 CATCAAGGGC CTGCCGGTCC TCCAGGACCA CCAGGACCAC CAGGGCCTCC
AGGAGTTTCT
8401 GGCGGTGGAT ATGATTTTGG TTATGATGGA GATTTTTACC GTGCTTGA

## Claims

1. A method of producing fusion proteins of a non-human scleroprotein selected from Spidroin-I or Fibroin-III with a human collagen type I , wherein said fusion proteins are capable of forming hydroxylated triple helix fibers, hereinafter also referred to as a self-fibrillating heterotrimeric collagen comprising fusion protein, in a plant or an isolated plant cell comprising:
(a) Targeting to and accumulating in a chloroplast of the plant or the isolated plant cell a nucleotide sequence encoding a Spid1Col1 construct as set forth by SEQ ID 16 including a signal peptide sequence for targeting to a chloroplast; or a Fib3Col1 construct as set forth by SEQ ID 20 including a signal peptide sequence for targeting to a chloroplast , all of which said sequences are devoid of an ER retention sequence,
(b) Targeting to and accumulating in a chloroplast of the plant or the isolated plant cell a nucleotide sequence encoding an exogenous non-human chimeric Prolyl 4 Hydroxylase (P4H) as set forth by SEQ ID 18, capable of specifically hydroxylating the Y position of Gly-X-Y triplets of said Collagen Type-I Alpha-I chain and said Collagen Type-I Alpha-II chain,
(c) Co-expressing the genes of (a) and (b) in said chloroplast of the plant or the isolated plant cell, thereby obtaining fusion proteins of the non-human scleroprotein with a human collagen.

2. The method of claim 1, wherein co-expressing the genes of (a) and (b) is done in two separate vectors and by means of an A2-enabled tricistronic expression vector that enables the induction of ribosomal skipping during translation of a protein in a cell, thereby making it possible to express the genes of (a) in parallel with the genes of (b) in one single plant; said A2-enabled tricistronic expression vector having an A2 sequence encoding for the A2 sequence GSGEGR GSLLTCEDVE ENPGP or GRGSLLTCED VEENPGP.

3. The method according to any one of the previous claims, wherein the method comprises avoiding the co-expression of a C-terminus and an N-terminus Collagen propeptide which are necessary for the assembly of collagen molecules into fibrils and thus enabling the formation of a triple-helical fibril structure.

4. The method of claim 1, wherein said plant is a Nicotiana benthamiana or Nicotiana tabacum plant.

5. The method of claim 1 further comprises filtrating and/or purifying the extracted fusion proteins of the non-human scleroprotein with a human collagen.

6. The method of claim 5, wherein said filtrating and/or purifying comprises a chromatography process.

7. The method of claim 1, wherein said plant is transiently transformed.

8. The method of claim 7 comprising introducing the nucleotide sequences, into at least one Agrobacterium tumefaciens strain.

9. The fusion proteins of a non-human scleroprotein with a human collagen obtained using a method according to any one of the previous claims.

10. Use of the fusion proteins according to claim 9, for producing nano fibers.

## Patentansprüche

1. Verfahren zum Herstellen von Fusionsproteinen eines nicht humanen Skleroproteins, ausgewählt aus Spidroin-I oder Fibroin-III mit einem humanen Kollagen Typ I, wobei die Fusionsproteine zum Ausbilden von hydroxylierten Dreifachhelix-Fasern in der Lage sind, nachfolgend ebenso als ein selbstfibrillierendes heterotrimeres Kollagen, umfassend Fusionsprotein, bezeichnet, in einer Pflanze oder einer isolierten Pflanzenzelle, umfassend:
(a) Targeting an und Akkumulieren in einem Chloroplasten der Pflanze oder der isolierten Pflanzenzelle einer Nukleotidsequenz, die ein Spid1Col1-Konstrukt codiert, wie durch SEQ ID 16 dargelegt, einschließlich einer Signalpeptidsequenz zum Targeting an einem Chloroplasten; oder ein Fib3Col1-Konstrukt, wie durch SEQ ID 20 dargelegt, einschließlich einer Signalpeptidsequenz zum Targeting an einem Chloroplasten, wobei alle dieser Sequenzen frei von einer ER-Retentionssequenz sind,
(b) Targeting an und Akkumulieren in einem Chloroplasten der Pflanze oder der isolierten Pflanzenzelle einer Nukleotidsequenz, die eine exogene nicht humane chimäre Prolyl-4-Hydroxylase (P4H) codiert, wie durch SEQ ID 18 dargelegt, die zum spezifischen Hydroxylieren der Y-Position von Gly-X-Y-Tripletts der Kollagen-Typ-I-Alpha-I-Kette und der Kollagen-Typ-I-Alpha-II-Kette in der Lage ist,
(c) Coexprimieren der Gene von (a) und (b) in dem Chloroplasten der Pflanze oder der isolierten Pflanzenzelle, wodurch Fusionsproteine des nicht humanen Skleroproteins mit einem humanen Kollagen erhalten werden.

2. Verfahren nach Anspruch 1, wobei das Coexprimieren der Gene von (a) und (b) in zwei separaten Vektoren und mittels eines A2-fähigen tricistronischen Expressionsvektors erfolgt, der die Induktion eines ribosomalen Überspringens während der Translation eines Proteins in einer Zelle ermöglicht, wodurch es möglich wird, die Gene von (a) parallel zu den Genen von (b) in einer einzigen Pflanze zu exprimieren; wobei der A2-fähige tricistronische Expressionsvektor eine A2-Sequenz aufweist, die für die A2-Sequenz GSGFGR GSUTCFDVF FNPGP oder GRGSUTCFD VFFNPGP codiert.

3. Verfahren nach einem der vorstehenden Ansprüche, wobei das Verfahren ein Vermeiden der Coexpression eines C-Terminus und eines N-Terminus Kollagen-Propeptids umfasst, die für die Anordnung von Kollagenmolekülen in Fibrillen notwendig sind und somit die Ausbildung einer dreifach helikalen Fibrillenstruktur ermöglichen.

4. Verfahren nach Anspruch 1, wobei die Pflanze eine Nicotiana benthamiana- oder Nicotiana tabacum-Pflanze ist.

5. Verfahren nach Anspruch 1, das ferner ein Filtrieren und/oder ein Reinigen der extrahierten Fusionsproteine des nicht humanen Skleroproteins mit einem humanen Kollagen umfasst.

6. Verfahren nach Anspruch 5, wobei das Filtrieren und/oder das Reinigen einen Chromatographieprozess umfasst.

7. Verfahren nach Anspruch 1, wobei die Pflanze vorübergehend transformiert ist.

8. Verfahren nach Anspruch 7, umfassend ein Einführen der Nukleotidsequenzen in mindestens einen Agrobacterium tumefaciens-Stamm.

9. Fusionsproteine eines nicht humanen Skleroproteins mit einem humanen Kollagen, das unter Verwendung eines Verfahrens nach einem der vorstehenden Ansprüche erhalten wird.

10. Verwendung der Fusionsproteine nach Anspruch 9 zum Herstellen von Nanofasern.

## Revendications

1. Procédé pour la production de protéines de fusion d'une scléroprotéine non humaine choisie parmi Spidroïne-I ou Fibroïne-III avec un collagène humain de type I, dans lequel lesdites protéines de fusion sont capables de former des fibres en triple hélice hydroxylées, ci-après également appelées collagène hétérotrimérique à autofibrillation comprenant une protéine de fusion, dans une plante ou une cellule végétale isolée, comprenant :
(a) le ciblage et l'accumulation, dans un chloroplaste de la plante ou de la cellule végétale isolée, d'une séquence nucléotidique codant pour une construction Spid1Col1 telle que définie dans SEQ ID n° : 16 comportant une séquence de peptide signal pour le ciblage d'un chloroplaste ; ou une construction Fib3Col1 telle que définie dans SEQ ID n° : 20 comportant une séquence de peptide signal pour le ciblage d'un chloroplaste, toutes lesdites séquences sont dépourvues d'une séquence de rétention dans le RE,
(b) le ciblage et l'accumulation, dans un chloroplaste de la plante ou de la cellule végétale isolée, d'une séquence nucléotidique codant pour une prolyl 4-hydroxylase (P4H) chimérique non humaine exogène, telle que définie dans SEQ ID n° : 18, capable de l'hydroxylation spécifique de la position Y des triplets Gly-X-Y de ladite chaîne Alpha-I de collagène de type I et de ladite chaîne Alpha-Il de collagène de type I,
(c) la co-expression des gènes de (a) et de (b) dans ledit chloroplaste de la plante ou de la cellule végétale isolée, obtenant ainsi des protéines de fusion de la scléroprotéine non humaine avec un collagène humain.

2. Procédé selon la revendication 1, dans lequel la co-expression des gènes de (a) et de (b) est effectuée dans deux vecteurs distincts et au moyen d'un vecteur d'expression tricistronique activé par A2 qui active l'induction du saut de ribosome pendant la traduction d'une protéine dans une cellule, rendant ainsi possible l'expression des gènes sz (a) en parallèle avec les gènes sz (b) dans une seule plante ; ledit vecteur d'expression tricistronique activé par A2 ayant une séquence A2 codant pour la séquence A2 GSGFGR GSUTCFDVF FNPGP ou GRGSUTCFD VFFNPGP.

3. Procédé selon l'une quelconque des revendications précédentes, dans lequel le procédé comprend le fait d'éviter la co-expression d'un propeptide de collagène C-terminal et d'un propeptide de collagène N-terminal qui sont nécessaires à l'assemblage de molécules de collagène en fibrilles et activant ainsi la formation d'une structure de fibrilles en triple hélice.

4. Procédé selon la revendication 1, dans lequel ladite plante est une *Nicotiana benthamiana* ou une *Nicotiana tabacum.*

5. Procédé selon la revendication 1 comprenant en outre la filtration et/ou la purification des protéines de fusion extraites de la scléroprotéine non humaine avec un collagène humain.

6. Procédé selon la revendication 5, dans lequel ladite filtration et/ou purification comprend un processus de chromatographie.

7. Procédé selon la revendication 1, dans lequel la plante est transformée de manière transitoire.

8. Procédé selon la revendication 7 comprenant l'introduction des séquences nucléotidiques dans au moins une souche *d'Agrobacterium tumefaciens.*

9. Protéines de fusion d'une scléroprotéine non humaine avec un collagène humain obtenues à l'aide d'un procédé selon l'une quelconque des revendications précédentes.

10. Utilisation des protéines de fusion selon la revendication 9, pour la production de nanofibres.
